# EUROPÄISCHE PATENTSCHRIFT

(11) **EP 2 566 963 B1**
(45) Veröffentlichungstag und Bekanntmachung des Hinweises auf die Patenterteilung: **21.10.2015**
(21) Anmeldenummer: 11724947.4
(22) Anmeldetag: 03.05.2011
(51) Int. Cl.: C12N 15/09, C12N 15/63, C12P 13/08

(54) **SENSOREN ZUR INTRAZELLULÄREN METABOLIT-DETEKTION**
SENSORS FOR THE DETECTION OF INTRACELLULAR METABOLITES
CAPTEURS POUR LA DÉTECTION DE MÉTABOLITES INTRACELLULAIRES

(30) Priorität: 03.05.2010 DE 102010019059
(43) Veröffentlichungstag der Anmeldung: 13.03.2013
(73) Patentinhaber: Forschungszentrum Jülich GmbH, 52428 Jülich (DE)
(72) Erfinder: EGGELING, Lothar, 52428 Jülich (DE); BOTT, Michael, 52428 Jülich (DE); BINDER, Stephan, 52249 Eschweiler (DE); FRUNZKE, Julia, 50259 Pulheim (DE); MUSTAFI, Nurije, 40225 Düsseldorf (DE)
(74) Vertreter: Herzog, Fiesser & Partner Patentanwälte PartG mbB
(86) Internationale Anmeldenummer: PCT/EP2011/002196
(87) Internationale Veröffentlichungsnummer: WO 2011/138006

(56) Entgegenhaltungen:
- SMOLKE C D ET AL: "Effects of transcription induction homogeneity and transcript stability on expression of two genes in a constructed operon", APPLIED MICROBIOLOGY AND BIOTECHNOLOGY, Bd. 57, Nr. 5-6, Dezember 2001 (2001-12), Seiten 689-696, XP000002657273, ISSN: 0175-7598
- VESELA I CHALOVA ET AL: "Development of a whole cell green fluorescent sensor for lysine quantification", WORLD JOURNAL OF MICROBIOLOGY AND BIOTECHNOLOGY, KLUWER ACADEMIC PUBLISHERS, DO, Bd. 24, Nr. 3, 22. Juli 2007 (2007-07-22), Seiten 353-359, XP019582124, ISSN: 1573-0972
- CHALOVA V I ET AL: "Quantification of total and bioavailable lysine in feed protein sources by a whole-cell green fluorescent protein growth-based Escherichia coli biosensor", APPLIED MICROBIOLOGY AND BIOTECHNOLOGY, SPRINGER, BERLIN, DE, Bd. 76, Nr. 1, 4. Mai 2007 (2007-05-04), Seiten 91-99, XP019538806, ISSN: 1432-0614, DOI: 10.1007/S00253-007-0989-6
- LI JINCAI ET AL: "Green fluorescent protein in Saccharomyces cerevisiae: Real-time studies of the GAL1 promoter", BIOTECHNOLOGY AND BIOENGINEERING, Bd. 70, Nr. 2, 20. Oktober 2000 (2000-10-20), Seiten 187-196, XP000002657274, ISSN: 0006-3592
- YE K ET AL: "Construction of an engineered yeast with glucose-inducible emission of green fluorescence from the cell surface", APPLIED MICROBIOLOGY AND BIOTECHNOLOGY, Bd. 54, Nr. 1, Juli 2000 (2000-07), Seiten 90-96, XP000002657275, ISSN: 0175-7598
- TAYLOR C J ET AL: "Construction of a whole-cell gene reporter for the fluorescent bioassay of nitrate", ANALYTICAL BIOCHEMISTRY, ACADEMIC PRESS INC, NEW YORK, Bd. 328, Nr. 1, 1. Mai 2004 (2004-05-01), Seiten 60-66, XP004501902, ISSN: 0003-2697, DOI: 10.1016/J.AB.2004.01.013
- HILMI HANAN T ABBAS ET AL: "Nisin induction without nisin secretion", MICROBIOLOGY (READING), Bd. 152, Nr. Part 5, Mai 2006 (2006-05), Seiten 1489-1496, XP000002657276, ISSN: 1350-0872
- LUERS G H ET AL: "Reporter-linked monitoring of transgene expression in living cells using the ecdysone-inducible promoter system", EUROPEAN JOURNAL OF CELL BIOLOGY, WISSENSCHAFLICHE VERLAGSGESELLSCHAFT, STUTTGART, DE, Bd. 79, Nr. 9, 1. September 2000 (2000-09-01), Seiten 653-657, XP004954699, ISSN: 0171-9335, DOI: 10.1078/0171-9335-00086
- GRIESBECK ET AL: "Fluorescent proteins as sensors for cellular functions", CURRENT OPINION IN NEUROBIOLOGY, LONDON, GB, Bd. 14, Nr. 5, 1. Oktober 2004 (2004-10-01), Seiten 636-641, XP027185140, ISSN: 0959-4388 [gefunden am 2004-09-29]
- ZHANG C ET AL: "Fluorescent Proteins as a Visible Molecular Signal for Rapid Quantification of Bioprocesses: Potential and Challenges", CHINESE JOURNAL OF CHEMICAL ENGINEERING, CHEMICAL INDUSTRY PRESS, BEIJING, CN, Bd. 18, Nr. 5, 1. Oktober 2010 (2010-10-01), Seiten 863-869, XP027457799, ISSN: 1004-9541, DOI: 10.1016/S1004-9541(09)60140-3 [gefunden am 2010-10-01]

### SEQUENCE LISTING

<110> Forschungszentrum Jülich GmbH
<120> Sensoren zur intrazellularen Metabolit-Detektion
<130> FJ10049PC
<150> DE 10 2010 019 059.4
   <151> 2010-05-03
<160> 72
<170> Patentln version 3.3
<210> 1
   <211> 129
   <212> DNA
   <213> Pseudomonas putida
<220>
   <221> misc_feature
   <223> Gensequenz des bkd-Promotors
<400> 1
   agtttgcgca tgagacaaaa tcaccggttt tttgtgttta tgcggaatgt ttatctgccc    60
   cgctcggcaa aggcaatcaa ttgagagaaa aattctcctg ccggaccact aagatgtagg   120
   ggacgctga                                                            129
<210> 2
   <211> 486
   <212> DNA
   <213> Pseudomonas putida
<220>
   <221> misc_feature
   <223> Gensequenz des BkdR-Regulators
<400> 2
   ctattcgcgc aaggtcatgc cattggccgg caacggcaag gctgtcttgt agcgcacctg    60
   tttcaaggca aaactcgagc ggatattcgc cacacccggc aaccgggtca ggtaatcgag   120
   aaaccgctcc agcgcctgga tactcggcag cagtacccgc aacaggtagt ccgggtcgcc 180
   cgtcatcagg tagcactcca tcacctcggg ccgttcggca atttcttcct cgaagcggtg 240
   cagcgactgc tctacctgtt tttccaggct gacatggatg aacacattca catccagccc 300
   caacgcctcg ggcgacaaca aggtcacctg ctggcggatc acccccagtt cttccatggc 360
   ccgcacccgg ttgaaacagg gcgtgggcga caggttgacc gagcgtgcca gctcggcgtt 420
   ggtgatgcgg gcgttttcct gcaggctgtt gagaatgccg atatcggtac gatcgagttt 480
   gcgcat 486
<210> 3
   <211> 105
   <212> DNA
   <213> Bacillus subtilis
<220>
   <221> misc_feature
   <223> Gensequenz des ackA-Promotors
<400> 3
   aacctatagt gaatgtgtct gaaaataacg acttcttatt gtaagcgtta tcaatacgca 60
   agttgacttg aaaagccgac atgacaatgt ttaaatggaa aagtc 105
<210> 4
   <211> 780
   <212> DNA
   <213> Bacillus subtilis
<220>
   <221> misc_feature
   <223> Gensequenz des CodY-Aktivators
<400> 4
   atggctttat tacaaaaaac aagaattatt aactccatgc tgcaagctgc ggcagggaaa 60
   ccggtaaact tcaaggaaat ggcggagacg ctgcgggatg taattgattc caatattttc 120
   gttgtaagcc gcagagggaa actccttggg tattcaatta accagcaaat tgaaaatgat 180
   cgtatgaaaa aaatgcttga ggatcgtcaa ttccctgaag aatatacgaa aaatctgttt 240
   aatgtccctg aaacatcttc taacttggat attaatagtg aatatactgc tttccctgtt 300
   gagaacagag acctgtttca agctggttta acaacaattg tgccgatcat cggaggcggg 360
   gaaagattag gaacacttat tctttcgcgt ttacaagatc aattcaatga cgatgactta 420
   attctagctg aatacggcgc aacagttgtc ggaatggaaa tcctaagaga aaaagcagaa 480
   gaaattgaag aggaagcaag aagcaaagct gtcgtacaaa tggctatcag ctcgctttct 540
   tacagtgagc ttgaagcaat tgagcacatt tttgaggagc ttgacggaaa tgaaggtctt 600
   cttgttgcaa gtaaaattgc tgaccgtgtc ggcattaccc gttctgttat tgtgaacgca 660
   ctcagaaagc tggagagcgc cggtgttatc gagtctagat cattaggaat gaaaggtact 720
   tatatcaagg tactaaacaa caaattccta attgaattag aaaatctaaa atctcattaa 780
<210> 5
   <211> 106
   <212> DNA
   <213> Pseudomonas putida
<220>
   <221> misc_feature
   <223> Gensequenz des mdeA-Promotors
<400> 5
   tgttgttttt atgtcagtga gcggcgcttt tcgtaggcgt atttggaaaa atttaagccg 60
   gtccgtggaa taagcttata acaaaccaca agaggcggtt gccatg 106
<210> 6
   <211> 480
   <212> DNA
   <213> Pseudomonas putida
<220>
   <221> misc_feature
   <223> Gensequenz des MdeR-Regulators
<400> 6
   tcaaatatgc ttctgtgcca ccggaatcac ccgcttctcc ttcaccgcct tgaacgagaa    60
   gctcgaatag atctccttca cccccggcag ccgctgcagt acctcgcggg tgaactcgcc 120
   gaacgactcc agatcccgcg ccagaatctc cagcaggaag tcatagcgcc cggagatgtt 180
   gtggcacgcc acgatttcgg ggatatccat cagccgctgc tcgaatgccc gggccatctc 240
   cttgctgtgc gaatccatca tgatgctgac gaaggcggtc actccgaagc ccagtgcctt 300
   gggtgacagg atggcctgat agccggtgat gtagcccgac tcctccagca gcttgacccg 360
   ccgccagcac ggcgaggtgg tcagggcgac gctgtcggcg agctcggcca cggtcagtcg 420
   ggcattgtct tgcagcgcgg ccagcagtgc gcggtcggta cggtcgatgg cgctaggcat 480
<210> 7
   <211> 186
   <212> DNA
   <213> Corynebacterium glutamicum
<220>
   <221> misc_feature
   <223> Gensequenz des brnF-Promotors
<400> 7
   tttttagacc ttgcgcgatt tcgtagcgcc gataaccttt atcatctggt tccagggctg 60
   ccttggatgg cgacacctcc aggcttgaat gaatctcttg cgttttttgc acactacaat 120
   catcacacaa ttgccgggta gttttgttgc cagtttgcgc acctcaacta ggctattgtg 180
   caatat 186
<210> 8
   <211> 456
   <212> DNA
   <213> Corynebacterium glutamicum
<220>
   <221 > misc_feature
   <223> Gensequenz des Lrp-Regulators
<400> 8
   atgaagctag attccattga tcgcgcaatt attgcggagc ttagcgcgaa tgcgcgcatc 60
   tcaaatctcg cactggctga caaggtgcat ctcactccgg gaccttgctt gaggagggtg 120
   cagcgtttgg aagccgaagg aatcattttg ggctacagcg cggacattca ccctgcggtg 180
   atgaatcgtg gatttgaggt gaccgtggat gtcactctca gcaacttcga ccgctccact 240
   gtagacaatt ttgaaagctc cgttgcgcag catgatgaag tactggagtt gcacaggctt 300
   tttggttcgc cagattattt tgtccgcatc ggcgttgctg atttggaggc gtatgagcaa 360
   tttttatcca gtcacattca aaccgtgcca ggaattgcaa agatctcatc acgttttgct 420
   atgaaagtgg tgaaaccagc tcgcccccag gtgtga 456
<210> 9
   <211> 89
   <212> DNA
   <213> Escherichia coli
<220>
   <221> misc_feature
   <223> Gensequenz des cysP-Promotors
<400> 9
   aacttattcc cttttcaact tccaaatcac caaacggtat ataaaaccgt tactcctttc 60
   acgtccgtta taaatatgat ggctattag 89
<210> 10
   <211> 975
   <212> DNA
   <213> Escherichia coli
<220>
   <221 > misc_feature
   <223> Gensequenz des CysB-Regulators
<400> 10
   atgaaattac aacaacttcg ctatattgtt gaggtggtca atcataacct gaatgtctca 60
   tcaacagcgg aaggacttta cacatcacaa cccgggatca gtaaacaagt cagaatgctg 120
   gaagacgagc taggcattca aattttttcc cgaagcggca agcacctgac gcaggtaacg 180
   ccagcagggc aagaaataat tcgtatcgct cgcgaagtcc tgtcgaaagt cgatgccata 240
   aaatcggttg ccggagagca cacctggccg gataaaggtt cactgtatat cgccaccacg 300
   catacccagg cacgctacgc attaccaaac gtcatcaaag gctttattga gcgttatcct 360
   cgcgtttctt tgcatatgca ccagggctcg ccgacacaaa ttgctgatgc cgtctctaaa 420
   ggcaatgctg atttcgctat cgccacagaa gcgctgcatc tgtatgaaga tttagtgatg 480
   ttaccgtgct accactggaa tcgggctatt gtagtcactc cggatcaccc gctggcaggc 540
   aaaaaagcca ttaccattga agaactggcg caatatccgt tggtgacata taccttcggc 600
   tttaccggac gttcagaact ggatactgcc tttaatcgcg cagggttaac gccgcgtatc 660
   gttttcacgg caacggatgc tgacgtcatt aaaacttacg tccggttagg gctgggggta 720
   ggggtcattg ccagcatggc ggtggatccg gtcgccgatc ccgaccttgt gcgtgttgat 780
   gctcacgata tcttcagcca cagtacaacc aaaattggtt ttcgccgtag tactttcttg 840
   cgcagttata tgtatgattt cattcagcgt tttgcaccgc atttaacgcg tgatgtcgtt 900
   gatgcggctg tcgcattgcg ctctaatgaa gaaattgagg tcatgtttaa agatataaaa 960
   ctgccggaaa aataa 975
<210> 11
   <211> 270
   <212> DNA
   <213> Escherichia coli
<220>
   <221 > misc_feature
   <223> Gensequenz des cadB-Promotors
<400> 11
   tttttattac ataaatttaa ccagagaatg tcacgcaatc cattgtaaac attaaatgtt 60
   tatcttttca tgatatcaac ttgcgatcct gatgtgttaa taaaaaacct caagttctca 120
   cttacagaaa cttttgtgtt atttcaccta atctttagga ttaatccttt tttcgtgagt 180
   aatcttatcg ccagtttggt ctggtcagga aatagttata catcatgacc cggactccaa 240
   attcaaaaat gaaattagga gaagagcatg 270
<210> 12
   <211> 1539
   <212> DNA
   <213> Escherichia coli
<220>
   <221> misc_feature
   <223> Gensequenz des CadC-Regulators
<400> 12
   ttattctgaa gcaagaaatt tgtcgagata aggtacaaca taaggaacag aagtctggaa 60
   tataccattt tcaatccagt aaagggtgtt tgcccctggg cgtaaattaa aggcggtgag 120
   atatgcatca gctgcttccc ggttcatccc cttcatttca taaaccttgc caagcaacac 180
   ataatttagc caggacattt caagatcaat gccagtattt atcgcctggt aagactcatc 240
   tgttttacct tttaccagag cactgaccgc ttttatttga tatataatgg acaggttgtt 300
   caattccggc agtgtaacaa tgttatctat ttctgtgttc agtgctgcta attgtttttc 360
   atctaaagga tgttgagaat ggcgcacgat atcaactaat gctttttctg ctctcgcgta 420
   ggtaaattct ggggatgatt gaacaatctc acctaataat tcactggcac ggttcaatga 480
   tttatcatcg ccatgcagta aataatcatg tgcctgataa aaattagtta ataacgcacc 540
   acgatgcggc aaaattttct ggagcgtctc ctgcattcgt tgtggccacg gttggtttaa 600
   cgcttttgat aaactctcca gtaaatcatt ttgaatcgcc agctgattac cgttagtgat 660
   gacataacgt ttatccagca tggttgaacc atctgcattg tctaccaatt ttatcgacat 720
   aaagcattgt tgagcacggt attggcgctg attaacaaac gcaatagata atgttttacc 780
   ggaactgctc ggttcatcaa tgttgtagtt gattttgtca tgcaccataa aggtggagaa 840
   ggtgttaagt gatgtcgcca ccaaatcacc cacgcctatc gcgtaagaga gctgatacgg 900
   ggaactccag ctgttacaac ttttatttac catattaatg tcaatatcgc gtggattgag 960
   caaaatacgc gatttgctca taggaagacg tgtatcaaga cttgaaaacg ctaccagtgc 1020
   tacacagata cctaacgaca acaggaaaaa aaaccatacc caaaaggtag tgaatcgttt 1080
   gcttttaact ggggattgtt caggtggcgt tgcggtgttt tgaatgttaa gactgtggga 1140
   gggagaatct gtggcaggaa ccgcctctgg tataggggga ggcgaagata gcattatttc 1200
   ctctccctct tcttcgctgt accagataac cggcaccatt aatttatagc cgcgctttgg 1260
   tacagtagcg atatagacag gactatcttc atcattatct tttaatgact tacgtagttc 1320
   tgagatactc tgcgtcacaa cgtgattggt gacaatactt ctcttccaga cattatcgat 1380
   aagttcatcc ctgctaagta cttcgccact gtgttgagca aagaaaacca gaagatcgat 1440
   taatctcggc tcaagggtaa gttgacgccc attgcggcta atttggttta tggacggagt 1500
   aacaagccat tcgccaacgc gaactacagg ttgttgcat 1539
<210> 13
   <211> 16
   <212> DNA
   <213> Corynebacterium glutamicum
<220>
   <221> misc_feature
   <223> Gensequenz des metY-Promotors
<400> 13
   tagaccaaga tgttca 16
<210> 14
   <211> 642
   <212> DNA
   <213> Corynebacterium glutamicum
<220>
   <221> misc_feature
   <223> Gensequenz des McbR-Regulators
<400> 14
   ctaaattgag tagtccgcag gtggagccga caacaactgc cgagccaaat cgcgagccgt 60
   ctcaagagga ctgatgttgt ggaccaatcg agatccagca agtccaccat caaggaacac 120
   caacagctga ttcgcctggg tggtgcctgg gtagccgttc ttctcagtga gcaaatcagt 180
   cagagtctta tgacaccact cgcggtgctc taacactgct gcaacaatgc ccttttcgct 240
   atcagtttcg gggcgagggt actcactagc cgcattctga aagtgcgagc cgcggaaatc 300
   tttttctggt tcttcctcaa tgcactgatc aaagaacgcg atgattttat cttccggatc 360
   cttcataccg acggtgcgct cacgccacgc ttcacgccac agctgatcga ggttctccag 420
   gtatgcaata accaaggcgt ccttcgatcc gaaaagggaa tagaggctcg ccttcgccac 480
   gtcagcttca cggaggatac gatcaatacc gatgacgcga ataccttctg tggtgaaaag 540
   gttggttgcg ctatcgagga gacgctgtcg ggggcttggt cgattgcgac gacggtttgc 600
   cccggcactt gttttactct tgcctgaagc gctagcagcc ac 642
<210> 15
   <211> 101
   <212> DNA
   <213> Escherichia coli
<220>
   <221> misc_feature
   <223> Gensequenz des argO-Promotors
<400> 15
   cttattagtt tttctgattg ccaattaata ttatcaattt ccgctaataa caatcccgcg 60
   atatagtctc tgcatcagat acttaattcg gaatatccaa c 101
<210> 16
   <211> 894
   <212> DNA
   <213> Escherichia coli
<220>
   <221> misc_feature
   <223> Gensequenz des ArgP-Regulators
<400> 16
   atgaaacgcc cggactacag aacattacag gcactggatg cggtgatacg tgaacgagga 60
   tttgagcgcg cggcacaaaa gctgtgcatt acacaatcag ccgtctcaca gcgcattaag 120
   caactggaaa atatgttcgg gcagccgctg ttggtgcgta ccgtaccgcc gcgcccgacg 180
   gaacaagggc aaaaactgct ggcactgctg cgccaggtgg agttgctgga agaagagtgg 240
   ctgggcgatg aacaaaccgg ttcgactccg ctgctgcttt cactggcggt caacgccgac 300
   agtctggcga cgtggttgct tcctgcactg gctcctgtgt tggctgattc gcctatccgc 360
   ctcaacttgc aggtagaaga tgaaacccgc actcaggaac gtctgcgccg cggcgaagtg 420
   gtcggcgcgg tgagtattca acatcaggcg ctgccgagtt gtcttgtcga taaacttggt 480
   gcgctcgact atctgttcgt cagctcaaaa ccctttgccg aaaaatattt ccctaacggc 540
   gtaacgcgtt cggcattact gaaagcgcca gtggtcgcgt ttgaccatct tgacgatatg 600
   caccaggcct ttttgcagca aaacttcgat ctgcctccag gcagcgtgcc ctgccatatc 660
   gttaattctt cagaagcgtt cgtacaactt gctcgccagg gcaccacctg ctgtatgatc 720
   ccgcacctgc aaatcgagaa agagctggcc agcggtgaac tgattgactt aacgcctggg 780
   ctatttcaac gacggatgct ctactggcac cgctttgctc ctgaaagccg catgatgcgt 840
   aaagtcactg atgcgttact cgattatggt cacaaagtcc ttcgtcagga ttaa 894
<210> 17
   <211> 110
   <212> DNA
   <213> Corynebacterium glutamicum
<220>
   <221> misc_feature
   <223> Gensequenz des lysE-Promotors
<400> 17
   gcaaagtgtc cagttgaatg gggttcatga agctatatta aaccatgtta agaaccaatc 60
   attttactta agtacttcca taggtcacga tggtgatcat ggaaatcttc 110
<210> 18
   <211> 873
   <212> DNA
   <213> Corynebacterium glutamicum
<220>
   <221> misc_feature
   <223> Gensequenz des LysE-Regulators
<400> 18
   atgaacccca ttcaactgga cactttgctc tcaatcattg atgaaggcag cttcgaaggc 60
   gcctccttag ccctttccat ttccccctcg gcggtgagtc agcgcgttaa agctctcgag 120
   catcacgtgg gtcgagtgtt ggtatcgcgc acccaaccgg ccaaagcaac cgaagcgggt 180
   gaagtccttg tgcaagcagc gcggaaaatg gtgttgctgc aagcagaaac taaagcgcaa 240
   ctatctggac gccttgctga aatcccgtta accatcgcca tcaacgcaga ttcgctatcc 300
   acatggtttc ctcccgtgtt caacgaggta gcttcttggg gtggagcaac gctcacgctg 360
   cgcttggaag atgaagcgca cacattatcc ttgctgcggc gtggagatgt tttaggagcg 420
   gtaacccgtg aagctaatcc cgtggcggga tgtgaagtag tagaacttgg aaccatgcgc 480
   cacttggcca ttgcaacccc ctcattgcgg gatgcctaca tggttgatgg gaaactagat 540
   tgggctgcga tgcccgtctt acgcttcggt cccaaagatg tgcttcaaga ccgtgacctg 600
   gacgggcgcg tcgatggtcc tgtggggcgc aggcgcgtat ccattgtccc gtcggcggaa 660
   ggttttggtg aggcaattcg ccgaggcctt ggttggggac ttcttcccga aacccaagct 720
   gctcccatgc taaaagcagg agaagtgatc ctcctcgatg agatacccat tgacacaccg 780
   atgtattggc aacgatggcg cctggaatct agatctctag ctagactcac agacgccgtc 840
   gttgatgcag caatcgaggg attgcggcct tag 873
<210> 19
   <211> 198
   <212> DNA
   <213> Escherichia coli
<220>
   <221> misc_feature
   <223> Gensequenz des fadE-Promotors
<400> 19
   gtaccggata ccgccaaaag cgagaagtac gggcaggtgc tatgaccagg actttttgac 60
   ctgaagtgcg gataaaaaca gcaacaatgt gagctttgtt gtaattatat tgtaaacata 120
   ttgctaaatg tttttacatc cactacaacc atatcatcac aagtggtcag acctcctaca 180
   agtaaggggc ttttcgtt 198
<210> 20
   <211> 720
   <212> DNA
   <213> Escherichia coli
<220>
   <221> misc_feature
   <223> Gensequenz des FadR-Regulators
<400> 20
   atggtcatta aggcgcaaag cccggcgggt ttcgcggaag agtacattat tgaaagtatc 60
   tggaataacc gcttccctcc cgggactatt ttgcccgcag aacgtgaact ttcagaatta 120
   attggcgtaa cgcgtactac gttacgtgaa gtgttacagc gtctggcacg agatggctgg 180
   ttgaccattc aacatggcaa gccgacgaag gtgaataatt tctgggaaac ttccggttta 240
   aatatccttg aaacactggc gcgactggat cacgaaagtg tgccgcagct tattgataat 300
   ttgctgtcgg tgcgtaccaa tatttccact atttttattc gcaccgcgtt tcgtcagcat 360
   cccgataaag cgcaggaagt gctggctacc gctaatgaag tggccgatca cgccgatgcc 420
   tttgccgagc tggattacaa catattccgc ggcctggcgt ttgcttccgg caacccgatt 480
   tacggtctga ttcttaacgg gatgaaaggg ctgtatacgc gtattggtcg tcactatttc 540
   gccaatccgg aagcgcgcag tctggcgctg ggcttctacc acaaactgtc ggcgttgtgc 600
   agtgaaggcg cgcacgatca ggtgtacgaa acagtgcgtc gctatgggca tgagagtggc 660
   gagatttggc accggatgca gaaaaatctg ccgggtgatt tagccattca ggggcgataa 720
<210> 21
   <211> 169
   <212> DNA
   <213> Bacillus subtilis
<220>
   <221> misc_feature
   <223> Gensequenz des fadM-Promotors
<400> 21
   ttaatttgca tagtggcaat tttttgccag actgaagagg tcataccagt tatgacctct 60
   gtacttataa caacaacgta aggttattgc gctatgcaaa cacaaatcaa agttcgtgga 120
   tatcatctcg acgtttacca gcacgtcaac aacgcccgct accttgaat 169
<210> 22
   <211> 648
   <212> DNA
   <213> Bacillus subtilis
<220>
   <221> misc_feature
   <223> Gensequenz des FabR-Regulators
<400> 22
   atgggcgtaa gagcgcaaca aaaagaaaaa acccgccgtt cgctggtgga agccgcattt 60
   agccaattaa gtgctgaacg cagcttcgcc agcctgagtt tgcgtgaagt ggcgcgtgaa 120
   gcgggcattg ctcccacctc tttttatcgg catttccgcg acgtagacga actgggtctg 180
   accatggttg atgagagcgg tttaatgcta cgccaactca tgcgccaggc gcgtcagcgt 240
   atcgccaaag gcgggagtgt gatccgcacc tcggtctcca catttatgga gttcatcggt 300
   aataatccta acgccttccg gttattattg cgggaacgct ccggcacctc cgctgcgttt 360
   cgtgccgccg ttgcgcgtga aattcagcac ttcattgcgg aacttgcgga ctatctggaa 420
   ctcgaaaacc atatgccgcg tgcgtttact gaagcgcaag ccgaagcaat ggtgacaatt 480
   gtcttcagtg cgggtgccga ggcgttggac gtcggcgtcg aacaacgtcg gcaattagaa 540
   gagcgactgg tactgcaact gcgaatgatt tcgaaagggg cttattactg gtatcgccgt 600
   gaacaagaga aaaccgcaat tattccggga aatgtgaagg acgagtaa 648
<210> 23
   <211> 152
   <212> DNA
   <213> Escherichia coli
<220>
   <221> misc_feature
   <223> Gensequenz des rhaSR-Promotors
<400> 23
   ccgtcatact ggcctcctga tgtcgtcaac acggcgaaat agtaatcacg acgtcaggtt 60
   cttaccttaa attttcgacg gaaaaccacg taaaaaacgt cgatttttca agatacaagc 120
   gtgaattttc aggaaatggc ggtgagcatc ac 152
<210> 24
   <211> 149
   <212> DNA
   <213> Escherichia coli
<220>
   <221> misc_feature
   <223> Gensequenz des rhaBAD-Promotors
<400> 24
   atcaccacaa ttcagcaaat tgtgaacatc atcacgttca tctttccctg gttcccaatg 60
   gcccattttc ctgtagtaac gagaacgtcg cgaattcagg cgctctttag actggtcgta 120
   atgaaattca gcaggatcac attatgacc 149
<210> 25
   <211> 759
   <212> DNA
   <213> Escherichia coli
<220>
   <221> misc_feature
   <223> Gensequenz des RhaR-Regulators
<400> 25
   gtggcgcatc agttaaaact tctcaaagat gatttttttg ccagcgacca gcaggcagtc 60
   gctgtggctg accgttatcc gcaagatgtc tttgctgaac atacacatga tttttgtgag 120
   ctggtgattg tctggcgcgg taatggcctg catctggttt tgcagaatat tatttattgc 180
   ccggagcgtc tgaagctgaa tcttgactgg cagggggcga ttccgggatt taacgccagc 240
   gcagggcaac cacactggcg cttaggtagc atggggatgg cgcaggcgcg gcaggttatc 300
   ggtcagcttg agcatgaaag tagtcagcat gtgccgtttg ctaacgaaat ggctgagttg 360
   ctgttcgggc agttggtgat gttgctgaat cgccatcgtt acaccagtga ttcgttgccg 420
   ccaacatcca gcgaaacgtt gctggataag ctgattaccc ggctggcggc tagcctgaaa 480
   agtccctttg cgctggataa attttgtgat gaggcatcgt gcagtgagcg cgttttgcgt 540
   cagcaatttc gccagcagac tggaatgacc atcaatcaat atctgcgaca ggtcagagtg 600
   tgtcatgcgc aatatcttct ccagcatagc cgcctgttaa tcagtgatat ttcgaccgaa 660
   tgtggctttg aagatagtaa ctatttttcg gtggtgttta cccgggaaac cgggatgacg 720
   cccagccagt ggcgtcatct caattcgcag aaagattaa 759
<210> 26
   <211> 849
   <212> DNA
   <213> Escherichia coli
<220>
   <221> misc_feature
   <223> Gensequenz des RhaS-Regulators
<400> 26
   gtggcgcatc agttaaaact tctcaaagat gatttttttg ccagcgacca gcaggcagtc 60
   gctgtggctg accgttatcc gcaagatgtc tttgctgaac atacacatga tttttgtgag 120
   ctggtgattg tctggcgcgg taatggcctg catgtactca acgatcgccc ttatcgcatt 180
   acccgtggcg atctctttta cattcatgct gacgataaac actcctacgc ttccgttaac 240
   gatctggttt tgcagaatat tatttattgc ccggagcgtc tgaagctgaa tcttgactgg 300
   cagggggcga ttccgggatt taacgccagc gcagggcaac cacactggcg cttaggtagc 360
   atggggatgg cgcaggcgcg gcaggttatc ggtcagcttg agcatgaaag tagtcagcat 420
   gtgccgtttg ctaacgaaat ggctgagttg ctgttcgggc agttggtgat gttgctgaat 480
   cgccatcgtt acaccagtga ttcgttgccg ccaacatcca gcgaaacgtt gctggataag 540
   ctgattaccc ggctggcggc tagcctgaaa agtccctttg cgctggataa attttgtgat 600
   gaggcatcgt gcagtgagcg cgttttgcgt cagcaatttc gccagcagac tggaatgacc 660
   atcaatcaat atctgcgaca ggtcagagtg tgtcatgcgc aatatcttct ccagcatagc 720
   cgcctgttaa tcagtgatat ttcgaccgaa tgtggctttg aagatagtaa ctatttttcg 780
   gtggtgttta cccgggaaac cgggatgacg cccagccagt ggcgtcatct caattcgcag 840
   aaagattaa 849
<210> 27
   <211> 77
   <212> DNA
   <213> Anabaena sp.
<220>
   <221> misc_feature
   <223> Gensequenz des hetC-Promotors
<400> 27
   tatcggaaaa aatctgtaac atgagataca caatagcatt tatatttgct ttagtatctc 60
   tctcttgggt gggattc 77
<210> 28
   <211> 76
   <212> DNA
   <213> Anabaena sp.
<220>
   <221> misc_feature
   <223> Gensequenz des nrrA-Promotors
<400> 28
   gtaattgtgg ctagagtaac aaagactaca aaaccttggg catgggcttg ttactttgaa 60
   attcatcgac gctaag 76
<210> 29
   <211> 77
   <212> DNA
   <213> Anabaena sp.
<220>
   <221> misc_feature
   <223> Gensequenz des devB-Promotors
<400> 29
   cctcgcccct catttgtaca gtctgttacc tttacctgaa acagatgaat gtagaattta 60
   taaaactagc atttgat 77
<210> 30
   <211> 672
   <212> DNA
   <213> Anabaena sp.
<220>
   <221> misc_feature
   <223> Gensequenz des NtcA-Regulators
<400> 30
   atgatcgtga cacaagataa ggccctagca aatgtttttc gtcagatggc aaccggagct 60
   tttcctcctg ttgtcgaaac gtttgaacgc aataaaacga tcttttttcc tggcgatcct 120
   gccgaacgag tctactttct tttgaaaggg gctgtgaaac tttccagggt gtacgaggca 180
   ggagaagaga ttacagtagc actactacgg gaaaatagcg tttttggtgt cctgtctttg 240
   ttgacaggaa acaagtcgga taggttttac catgcggtgg catttactcc agtagaattg 300
   ctttctgcac caattgaaca agtggagcaa gcactgaagg aaaatcctga attatcgatg 360
   ttgatgctgc ggggtctgtc ttcgcggatt ctacaaacag agatgatgat tgaaacctta 420
   gcgcaccgag atatgggttc gagattggtg agttttctgt taattctctg tcgtgatttt 480
   ggtgttcctt gtgcagatgg aatcacaatt gatttaaagt tatctcatca ggcgatcgcc 540
   gaagcaattg gctctactcg cgttactgtt actaggctac taggggattt gcgggagaaa 600
   aagatgattt ccatccacaa aaagaagatt actgtgcata aacctgtgac tctcagcaga 660
   cagttcactt aa 672
<210> 31
   <211> 909
   <212> DNA
   <213> Mycobacterium sp.
<220>
   <221> misc_feature
   <223> Gensequenz des CbbR-Regulators
<400> 31
   atgaccaacg cgcgattgcg agctctggtc gaactggcgg ataccggttc ggtgcgcgcc 60
   gctgctgagc gactcgtggt caccgaatct tcgatctcct cggctttacg cgcattgagc 120
   aacgacatcg gcatcagctt ggtcgaccgg catggccgcg gggtgcggct gactcctgcc 180
   ggcctgcgtt acgtcgaata cgcgcggcgg atcctcggct tgcacgacga ggcgatattg 240
   gctgcccgcg gagaggccga cccggagaat ggctcgatcc ggctggctgc ggtcacctcc 300
   gcgggggaac tgctcatccc cgccgcgttg gcatcgttcc gtgccgcgta ccccggtgtc 360
   gttctgcatc tggaggtggc ggcgcgcagc ttggtgtggc ctatgctggc ccgccacgag 420
   gtcgacctcg ttgtggcggg acggccgccg gacgaattgg tccggaaagt gtgggtgcgc 480
   gccgtcagcc cgaacgcgct tgtcgtcgtg ggaccacccg cggtagcgaa gggattccag 540
   cccgccaccg cgacctggct gctgcgtgag accggatccg gtacccgctc tacgttgacg 600
   gcactgcttg acgacctcga tgtcgcgcca cctcaattgg tgctcggatc gcacggcgcg 660
   gtggttgccg cggcggtggc cgggctgggc gtgacgttgg tgtcgcgtca ggctgtgcag 720
   cgcgaactgg ccgccggcgc actcgtcgaa ctgccggtgc ccggtactcc gataagccgg 780
   ccatggcatg tggtcagcca gatcagtccg acgatgtcga ccgaactgct catcaagcac 840
   ctcttgtccc agcgagacct gggctggcgc gatatcaaca ccacccttcg gggagccgtt 900
   accgcctga 909
<210> 32
   <211> 120
   <212> DNA
   <213> Streptomyces cattleya
<220>
   <221> misc_feature
   <223> Gensequenz des pcbAB-Promotors
<400> 32
   gtgctggtcc cgcaccgggc ggtggacagc ttccggcggc agctgaccgg ccgctacttc 60
   ggcggcccgg acacctcccg cgagggcgtg ctcttcctgg ccaactacgt cttcgacttc 120
<210> 33
   <211> 807
   <212> DNA
   <213> Streptomyces cattleya
<220>
   <221> misc_feature
   <223> Gensequenz des ThnU-Regulators
<400> 33
   atggacgcag acgactgttg ggcgcgggcg ggcaccgtgc ggatccgcct gctcggcccg 60
   gtggagctgg cctgcggcac gcggccggtg ccggtgaccg ggcggcgcca gttgagggtg 120
   gtggccgcgc tcgcgctgga ggccggacgg gtgctctcca ccgcggggct gatcgcctcg 180
   ttgtgggcgg acgagccgcc gcgcaccgcc gcccggcagc tccagaccag cgtgtggatg 240
   atccgccggg cgctcgcctc ggtgggcgcg ccgcagtgcg tcgtccgctc caccccggcc 300
   ggctacctgc tcgacccggc ccactacgaa ctcgacagcg accggttccg gcacgcggtg 360
   ctgaccgccc gggagttgca gcgggacggg cggctggccc aggcccgggc ccgggtcgac 420
   gaggggctgg cgctgtggcg cggccccgcc ctcggcgcgg cggcgggcgc cggactccag 480
   ccccgggccc gccggctgga ggaggaacgg gtcttcgccc tggagcagcg cgccgggctc 540
   gacctcgcgc tcggccgcca cgagacggcc atcggcgaac tcctcgacct catcgcccag 600
   catccgctgc gcgaggcggc ctacgccgac ctgatgctcg ccctgtaccg ttccggccgc 660
   cagtccgacg cgctcgccgt ctaccgcagg gcgcagcggg tgctcgccga cgagctggcc 720
   gtccgccccg gcccccgcct cgccggcctg gagcgggcca tcctgcggca ggacgagtcg 780
   ctgctggccg gcgcggcggt gccctga 807
<210> 34
   <211> 120
   <212> DNA
   <213> Streptomyces viridochromogenes
<220>
   <221> misc_feature
   <223> Genseqzenz des aviRa-Promotors
<400> 34
   tcaggggcct gcctccagca cgtcggctgc ccggaccagt acggccgagc gggtgccgat 60
   cttcagccgc tccagggcct ttacgggagc caccgggatc ttacggctgc ggtcggtgac 120
<210> 35
   <211> 621
   <212> DNA
   <213> Streptomyces viridochromogenes
<220>
   <221> misc_feature
   <223> Gensequenz des AviC1- bzw. AviC2-Regulators
<400> 35
   ctaggaaccc gcggacgtat cgggtggatg gtcggatccc tctgcatcgc cgatgtgtcc 60
   gggaagcccg tgggcgaagg caaccagtcc ggcctgaaga cgggattcga ccccgagctt 120
   cgccagtatc tgggccatat gagccttgac ggtgcgctcg gtgaccccga gcagcgcggc 180
   gatctcacgg ttggagtagc cgtggctcag caggaggaag acctggagct cgcggtcgga 240
   gagtaaatgt acctggctga gcccttccag ccaggggaac tggtcctcgt ggagaaatcg 300
   atcgtcgcca gaatcactgg aatcgcagcc ggaatatggc aaagtctggc ccccgtatga 360
   gcgtgtggtc cttgcatgcc ctaagaggtc atccgacgca tcgagtatca aggcgccgaa 420
   gggcgccacc actgaactat gaagacgtga gggcgatacc acccatgcga cgaatgggtc 480
   ctggacatta ctcatcttga tcatcttatc gcatctacgg ccgggttggg gcgccttggt 540
   gccgcctgct gtcgtgagca gggcccgccg aggcgtgggc aaggcggata aggcggcccg 600
   tgcccggtgt gtgcacggca a 621
<210> 36
   <211> 130
   <212> DNA
   <213> Nocardia uniformis
<220>
   <221> misc_feature
   <223> Gensequenz des nocF-Promotors
<400> 36
   catcacgaac ctccagccgt gggatcgccc tccggcagca tttatagacg gtttgcttat 60
   cgatccgttt tcacattcac ccgcagtgat aaggaattga taaacgattt tcctagcctg 120
   agcggactat 130
<210> 37
   <211> 1748
   <212> DNA
   <213> Nocardia uniformis
<220>
   <221> misc_feature
   <223> Gensequenz des NocR-Regulators
<400> 37
   gtgcgcgcgg gcgggcgccg ggtccaggtc ggcgggccgc gccagcggac ggtgctggcg 60
   acgctgctgc tcaacgccga ccgcgtggtg tcggtggacg cgctggccga gacggtctgg 120
   ggcgcccggc ccccgtcgac cagccggacg caggtggcga tctgcgtgtc cgcgctgcgc 180
   aaggcgttcc gcgcgagcgg cgccgacgag gtgatcgaga ccgtcgcgcc ggggtacgtc 240
   ctgcgctccg gcgggcaccg gctggacacc ctggacttcg acgaactggt ggcgctggcg 300
   agggcggcgg cccggcaggg ccggggcgcg gaggccgtcc ggctgtacgg ctcggcgctc 360
   gcgctgcgcc ggggcccggt gctggcgaac gtgaccggga cggtgcccga gcacctgtcc 420
   tgccagtggg aggagaccct gctcaccgcc tacgaggagc aggtcgagct gcgcctggcg 480
   ctgggcgagc accgcctgct ggtcgccggg ctcgcggcgg cggtcgagcg gcacccgctg 540
   cgcgaccggc tctacggcct gctcatcatc gcccagtacc gctccggcca ccgggccgcg 600
   gcgctggaga cgttcgcccg gttgcgccgc cgctcggtcg acgagctcgg cctggagccg 660
   gggatggagc tgcgccggct gcacgagcgc atcctgcgcg acgaggaccg cccggcggtc 720
   gagcgcccgc cgtcgcagct gcccgccgcg acgcaggtgt tcgtcgggcg cgccgaggag 780
   ctggcggtgc tggaccggct ggccgccgag gacgggcagg cgggcgcgcc gccgctcgga 840
   ctgctggtcg gcggcgtcgg cgtgggcaag accgcgctgg cggtgcggtg ggcgcacgcc 900
   aacgccgacc tgttccccga cggccagctg ttcgtcgacc tgggcgggca cgacccgcac 960
   cacccgccgt cggcccccgg cgccgtgctc gcgcacctgc tgcacgcgct gggcgtgccg 1020
   cccgagcggg tgccggtcgc cgccgaacga cccgcgctgt tccgcaccgc gatggccgcc 1080
   cgccggatgc tgctggtgct ggacgacgcc cgcgacgcgg cccaggtctg gccgctgctg 1140
   ccgaacaccg ccacctgccg ggtgctggtg acctcccgcg acccgctgcg cgagctggtc 1200
   gcccgcagcg gggcggtgcc gctgcggctg ggcggcctcg ggttcgacga gtccgtggcg 1260
   ctggtgcgcg gcatcatcgg cgaggcgcgg gccgggcgcg acccggacgc cctggtcggg 1320
   ctggtcgagc tggtcgagct gtgcggtcgg gtgccgggcg cgctgctggc cgccgccgcg 1380
   cacctggcca gcaaaccgca ctggggcgtg cccaggatgg tccgggagct caaccgcccg 1440
   cgcagcaggc tgtccggcct cggcgggcag cacctgcgcg acgggctcgc ctccagcgcc 1500
   cgctgcctgg acccggtggc ggccgacctg taccgggcgc tgggcggcct gcccacgccg 1560
   gagctgacgt cctggacggc cacggccctg ctgggctgct cgacacccga ggccgacgac 1620
   gtgctggagc gcctggtcga cgcgcacctg ctggagcccg ccggggcggg cgccggcggc 1680
   gagagccact accggctgcc cagcctgtcc cacgcctacg cggcgaactt gccacgaccg 1740
   gcccgtga 1748
<210> 38
   <211> 30
   <212> DNA
   <213> Artificial
<220>
   <223> Primer
<220>
   <221> misc_feature
   <223> plysGE_for
<400> 38
   cgcggatccc taagccgcaa tccctgattg 30
<210> 39
   <211> 35
   <212> DNA
   <213> Artificial
<220>
   <223> Artificial sequence
<220>
   <221> misc_feature
   <223> plysGE_rev
<400> 39
   tccgatggac agtaaaagac tggcccccaa agcag 35
<210> 40
   <211> 46
   <212> DNA
   <213> Artificial
<220>
   <223> Primer
<220>
   <221> misc_feature
   <223> peYFP_rev
<400> 40
   tgaggatcct tattacttgt cagctcgtcc atgccgagag tgatcc 46
<210> 41
   <211> 55
   <212> DNA
   <213> Artificial
<220>
   <223> Primer
<220>
   <221> misc_feature
   <223> peYFP_fw2
<400> 41
   cttttactgt ccatcggaac tagctatggt gagcaagggc gaggagctgt tcacc 55
<210> 42
   <211> 22
   <212> DNA
   <213> Artificial
<220>
   <223> Primer
<220>
   <221> misc_feature
   <223> ARS_for
<400> 42
   tcaactgcta tcccccctgt ta 22
<210> 43
   <211> 33
   <212> DNA
   <213> Artificial
<220>
   <223> Primer
<220>
   <221> misc_feature
   <223> ARS_rev
<400> 43
   aaactccttt acttaaatgt tttgataaat aaa 33
<210> 44
   <211> 22
   <212> DNA
   <213> Artificial
<220>
   <223> Primer
<220>
   <221> misc_feature
   <223> EYFP_for_Ndel
<400> 44
   tacatatggt gagcaagggc ga 22
<210> 45
   <211> 30
   <212> DNA
   <213> Artificial
<220>
   <223> Primer
<220>
   <221> misc_feature
   <223> EYFP_rev_EcoRI
<400> 45
   tagaattctt atctagactt gtacagctcg 30
<210> 46
   <211> 24
   <212> DNA
   <213> Artificial
<220>
   <223> Primer
<220>
   <221> misc_feature
   <223> pEKEx2_for
<400> 46
   cggcgtttca cttctgagtt cggc 24
<210> 47
   <211> 30
   <212> DNA
   <213> Artificial
<220>
   <223> Primer
<220>
   <221> misc_feature
   <223> EYFP_rev
<400> 47
   tagaattctt atctagactt gtacagctcg 30
<210> 48
   <211> 1060
   <212> DNA
   <213> Artificial
<220>
   <223> Genkonstrukt
<400> 48
   tcaactgcta tcccccctgt tattaaaacg cttacattga ttattatagt catttaattt 60
   taaatgtcta tacttttata aaataaatat aatcatattt ttttccggtt caccgtttta 120
   taaatttttc tatggaagat tcattcataa tgtggtacac tcatcaacgg aaacgaatca 180
   attaaatagc tattatcact tgtataacct caataatatg gtttgagggt gtctaccagg 240
   aaccgtaaaa tcctgattac aaaatttgtt tatgacattt tttgtaatca ggattttttt 300
   tatttatcaa aacatttaag taaaggagtt tgttatggtg agcaagggcg aggagctgtt 360
   caccggggtg gtgcccatcc tggtcgagct ggacggcgac gtaaacggcc acaagttcag 420
   cgtgtccggc gagggcgagg gcgatgccac ctacggcaag ctgaccctga agttcatctg 480
   caccaccggc aagctgcccg tgccctggcc caccctcgtg accaccttcg gctacggcct 540
   gcagtgcttc gcccgctacc ccgaccacat gaagcagcac gacttcttca agtccgccat 600
   gcccgaaggc tacgtccagg agcgcaccat cttcttcaag gacgacggca actacaagac 660
   ccgcgccgag gtgaagttcg agggcgacac cctggtgaac cgcatcgagc tgaagggcat 720
   cgacttcaag gaggacggca acatcctggg gcacaagctg gagtacaact acaacagcca 780
   caacgtctat atcatggccg acaagcagaa gaacggcatc aaggtgaact tcaagatccg 840
   ccacaacatc gaggacggca gcgtgcagct cgccgaccac taccagcaga acacccccat 900
   cggcgacggc cccgtgctgc tgcccgacaa ccactacctg agctaccagt ccgccctgag 960
   caaagacccc aacgagaagc gcgatcacat ggtcctgctg gagttcgtga ccgccgccgg 1020
   gatcactctc ggcatggacg agctgtacaa gtctagataa 1060
<210> 49
   <211> 723
   <212> DNA
   <213> enhanced yellow fluorescens protein (eyfp)
<400> 49
   gtgagcaagg gcgaggagct gttcaccggg gtggtgccca tcctggtcga gctggacggc 60
   gacgtaaacg gccacaagtt cagcgtgtcc ggcgagggcg agggcgatgc cacctacggc 120
   aagctgaccc tgaagttcat ctgcaccacc ggcaagctgc ccgtgccctg gcccaccctc 180
   gtgaccacct tcggctacgg cctgcagtgc ttcgcccgct accccgacca catgaagcag 240
   cacgacttct tcaagtccgc catgcccgaa ggctacgtcc aggagcgcac catcttcttc 300
   aaggacgacg gcaactacaa gacccgcgcc gaggtgaagt tcgagggcga caccctggtg 360
   aaccgcatcg agctgaaggg catcaacttc aaggaggacg gcaacatcct ggggcacaag 420
   ctggagtaca actacaacag ccacaacgtc tatatcatgg ccgacaagca gaagaacggc 480
   atcaaggtga acttcaagat ccgccacaac atcgagggcg gcagcgtgca gctcgccgac 540
   cactaccagc agaacacccc catcggcgac ggccccgtgc tgctgcccga caaccactac 600
   ctgagctacc agtccgccct gagcaaagac cccaacgaga agcgcgatca catggtcctg 660
   ctggagttcg tgaccgccgc cgggatcact ctcggcatgg acgagctgta caagtctaga 720
   taa 723
<210> 50
   <211> 30
   <212> DNA
   <213> Artificial
<220>
   <223> Primer
<220>
   <221> misc_feature
   <223> Irp-fw-A-BamHI
<400> 50
   gcgcggatcc tcacacctgg gggcgagctg 30
<210> 51
   <211> 51
   <212> DNA
   <213> Artificial
<220>
   <223> Primer
<220>
   <221> misc_feature
   <223> Irp-brnF-rv-I-Ndel
<400> 51
   gcgccatatg atatctcctt cttaaagttc agcttgaatg aatctcttgc g 51
<210> 52
   <211> 28
   <212> DNA
   <213> Artificial
<220>
   <223> Primer
<220>
   <221> misc_feature
   <223> eyfp-fw-H-NdeI
<400> 52
   gcgccatatg gtgagcaagg gcgaggag 28
<210> 53
   <211> 34
   <212> DNA
   <213> Artificial
<220>
   <223> Primer
<220>
   <221> misc_feature
   <223> eyfp-rv-D-SalI
<400> 53
   gcgcgtcgac ttatctagac ttgtacagct cgtc 34
<210> 54
   <211> 20
   <212> DNA
   <213> Artificial
<220>
   <223> Primer
<220>
   <221> misc_feature
   <223> Seq_pJC1_for1
<400> 54
   cgatcctgac gcagattttt 20
<210> 55
   <211> 20
   <212> DNA
   <213> Artificial
<220>
   <223> Primer
<220>
   <221> misc_feature
   <223> Seq_pJC1_rev1
<400> 55
   ctcaccggct ccagatttat 20
<210> 56
   <211> 1765
   <212> DNA
   <213> Fragment BamHI-<-EYFP-lysE'-lysG->-Sall
<220>
   <221> misc_feature
   <223> BamHI-<-EYFP-IysE'-IysG->-SalI
<400> 56
   ggatccttat tacttgtaca gctcgtccat gccgagagtg atcccggcgg cggtcacgaa 60
   ctccagcagg accatgtgat cgcgcttctc gttggggtct ttgctcaggg cggactggta 120
   gctcaggtag tggttgtcgg gcagcagcac ggggccgtcg ccgatggggg tgttctgctg 180
   gtagtggtcg gcgagctgca cgctgccgcc ctcgatgttg tggcggatct tgaagttcac 240
   cttgatgccg ttcttctgct tgtcggccat gatatagacg ttgtggctgt tgtagttgta 300
   ctccagcttg tgccccagga tgttgccgtc ctccttgaag ttgatgccct tcagctcgat 360
   gcggttcacc agggtgtcgc cctcgaactt cacctcggcg cgggtcttgt agttgccgtc 420
   gtccttgaag aagatggtgc gctcctggac gtagccttcg ggcatggcgg acttgaagaa 480
   gtcgtgctgc ttcatgtggt cggggtagcg ggcgaagcac tgcaggccgt agccgaaggt 540
   ggtcacgagg gtgggccagg gcacgggcag cttgccggtg gtgcagatga acttcagggt 600
   cagcttgccg taggtggcat cgccctcgcc ctcgccggac acgctgaact tgtggccgtt 660
   tacgtcgccg tccagctcga ccaggatggg caccaccccg gtgaacagct cctcgccctt 720
   gctcaccata tgatatctcc ttcttaaagt tcatctaggt ccgatggaca gtaaaagact 780
   ggcccccaaa agcagacctg taatgaagat ttccatgatc accatcgtga cctatggaag 840
   tacttaagta aaatgattgg ttcttaacat ggtttaatat agcttcatga accccattca 900
   actggacact ttgctctcaa tcattgatga aggcagcttc gaaggcgcct ccttagccct 960
   ttccatttcc ccctcggcgg tgagtcagcg cgttaaagct ctcgagcatc acgtgggtcg 1020
   agtgttggta tcgcgcaccc aaccggccaa agcaaccgaa gcgggtgaag tccttgtgca 1080
   agcagcgcgg aaaatggtgt tgctgcaagc agaaactaaa gcgcaactat ctggacgcct 1140
   tgctgaaatc ccgttaacca tcgccatcaa cgcagattcg ctatccacat ggtttcctcc 1200
   cgtgttcaac gaggtagctt cttggggtgg agcaacgctc acgctgcgct tggaagatga 1260
   agcgcacaca ttatccttgc tgcggcgtgg agatgtttta ggagcggtaa cccgtgaagc 1320
   taatcccgtg gcgggatgtg aagtagtaga acttggaacc atgcgccact tggccattgc 1380
   aaccccctca ttgcgggatg cctacatggt tgatgggaaa ctagattggg ctgcgatgcc 1440
   cgtcttacgc ttcggtccca aagatgtgct tcaagaccgt gacctggacg ggcgcgtcga 1500
   tggtcctgtg gggcgcaggc gcgtatccat tgtcccgtcg gcggaaggtt ttggtgaggc 1560
   aattcgccga ggccttggtt ggggacttct tcccgaaacc caagctgctc ccatgctaaa 1620
   agcaggagaa gtgatcctcc tcgatgagat acccattgac acaccgatgt attggcaacg 1680
   atggcgcctg gaatctagat ctctagctag actcacagac gccgtcgttg atgcagcaat 1740
   cgagggattg cggccttagg tcgac 1765
<210> 57
   <211> 2506
   <212> DNA
   <213> Fragment BamHI-T7terminator-<-Crimson----lacIQ->-BamHI
<220>
   <221> misc_feature
   <223> BamHI-T7terminator-<-Crimson--laclQ->-BamHI
<400> 57
   ggatcccgag aaaggaaggg aagaaagcga aaggagcggg cgctagggcg ctggcaagtg 60
   tagcggtcac gctgcgcgta accaccacac ccgccgcgct taatgcgccg ctacagggcg 120
   cgtcccattc gccaatccgg atatagttcc tcctttcagc aaaaaacccc tcaagacccg 180
   tttagaggcc ccaaggggtt atgctagtta ttgctcagcg gtggcagcag ccaactcagc 240
   ttcctttcgg gctttgttag cagccggatc tcagtgggaa ttcctactgg aacaggtggt 300
   ggcgggcctc ggcgcgctcg tactgctcca ccacggtgta gtcctcgttg tgggaggtga 360
   tgtcgagctt gtagtccacg tagtggtagc cgggcagctt cacgggcttc ttggccatgt 420
   agatggactt gaactcacac aggtagtggc cgccgccctt cagcttcagc gccatgtggt 480
   tctcgccctt cagcacgccg tcgcgggggt agttgcgctc agtggagggc tcccagccca 540
   gagtcttctt ctgcattacg gggccgtcgg aggggaagtt cacgccgatg aacttcacgt 600
   ggtagatgag ggtgccgtcc tgcagggagg agtcctgggt cacggtcacc acgccgccgt 660
   cctcgaagtt catcacgcgc tcccacttga agccctcggg gaaggactgc ttgaggtagt 720
   cggggatgtc ggcggggtgc ttgatgtacg ccttggagcc gtagaagaac tggggggaca 780
   ggatgtccca ggcgaagggc agggggccgc ccttggtcac ttgcagcttg gcggtctggg 840
   tgccctcgta gggcttgccc tcgcccacgc cctcgatctc gaactcgtgg ccgttcacgg 900
   agccctccat gtgcaccttg aagcgcatga agggcttgat gacgttctca gtgctatcca 960
   tatgtatatc tccttctgca ggcatgcaag cttggcgtaa tcatggtcat atcttttaat 1020
   tctgtttcct gtgtgaaatt gttatccgct cacaattcca cacattatac gagccgatga 1080
   ttaattgtca acagctcatt tcagaatatt tgccagaacc gttatgatgt cggcgcaaaa 1140
   aacattatcc agaacgggag tgcgccttga gcgacacgaa ttatgcagtg atttacgacc 1200
   tgcacagcca taccacagct tccgatggct gcctgacgcc agaagcattg gtgcaccgtg 1260
   cagtcgataa gcccggatca gcttgcaatt cgcgcgcgaa ggcgaagcgg catgcattta 1320
   cgttgacacc atcgaatggt gcaaaacctt tcgcggtatg gcatgatagc gcccggaaga 1380
   gagtcaattc agggtggtga atgtgaaacc agtaacgtta tacgatgtcg cagagtatgc 1440
   cggtgtctct tatcagaccg tttcccgcgt ggtgaaccag gccagccacg tttctgcgaa 1500
   aacgcgggaa aaagtggaag cggcgatggc ggagctgaat tacattccca accgcgtggc 1560
   acaacaactg gcgggcaaac agtcgttgct gattggcgtt gccacctcca gtctggccct 1620
   gcacgcgccg tcgcaaattg tcgcggcgat taaatctcgc gccgatcaac tgggtgccag 1680
   cgtggtggtg tcgatggtag aacgaagcgg cgtcgaagcc tgtaaagcgg cggtgcacaa 1740
   tcttctcgcg caacgcgtca gtgggctgat cattaactat ccgctggatg accaggatgc 1800
   cattgctgtg gaagctgcct gcactaatgt tccggcgtta tttcttgatg tctctgacca 1860
   gacacccatc aacagtatta ttttctccca tgaagacggt acgcgactgg gcgtggagca 1920
   tctggtcgca ttgggtcacc agcaaatcgc gctgttagcg ggcccattaa gttctgtctc 1980
   ggcgcgtctg cgtctggctg gctggcataa atatctcact cgcaatcaaa ttcagccgat 2040
   agcggaacgg gaaggcgact ggagtgccat gtccggtttt caacaaacca tgcaaatgct 2100
   gaatgagggc atcgttccca ctgcgatgct ggttgccaac gatcagatgg cgctgggcgc 2160
   aatgcgcgcc attaccgagt ccgggctgcg cgttggtgcg gatatctcgg tagtgggata 2220
   cgacgatacc gaagacagct catgttatat cccgccgtta accaccatca aacaggattt 2280
   tcgcctgctg gggcaaacca gcgtggaccg cttgctgcaa ctctctcagg gccaggcggt 2340
   gaagggcaat cagctgttgc ccgtctcact ggtgaaaaga aaaaccaccc tggcgcccaa 2400
   tacgcaaacc gcctctcccc gcgcgtcggc cgccatgccg gcgataatgg cctgcttctc 2460
   gccgaaacgt ttggtggcgg gaccagtgac gaaggcttga ggatcc 2506
<210> 58
   <211> 20
   <212> DNA
   <213> Artificial
<220>
   <223> Primer
<220>
   <221> misc_feature
   <223> lysC-32F
<400> 58
   gaacatcagc gacaggacaa 20
<210> 59
   <211> 20
   <212> DNA
   <213> Artificial
<220>
   <223> Primer
<220>
   <221> misc_feature
   <223> lysC-1938R
<400> 59
   gggaagcaaa gaaacgaaca 20
<210> 60
   <211> 20
   <212> DNA
   <213> Artificial
<220>
   <223> Primer
<220>
   <221> misc_feature
   <223> hom-289F
<400> 60
   cctccccggg ttgatattag 20
<210> 61
   <211> 20
   <212> DNA
   <213> Artificial
<220>
   <223> Primer
<220>
   <221> misc_feature
   <223> thrB-2069R
<400> 61
   ggccagcacg aatagcttta 20
<210> 62
   <211> 20
   <212> DNA
   <213> Artificial
<220>
   <223> Primer
<220>
   <221> misc_feature
   <223> hom-1684F
<400> 62
   aggaatctcc ctgcgtacaa 20
<210> 63
   <211> 20
   <212> DNA
   <213> Artificial
<220>
   <223> Primer
<220>
   <221> misc_feature
   <223> thrB-2951R
<400> 63
   ccggattcatccaagaaagc 20
<210> 64
   <211> 20
   <212> DNA
   <213> Artificial
<220>
   <223> Primer
<220>
   <221> misc_feature
   <223> thrC-22F
<400> 64
   gccttaaaac gccactcaat 20
<210> 65
   <211> 20
   <212> DNA
   <213> Artificial
<220>
   <223> Primer
<220>
   <221> misc_feature
   <223> thrB-2951 R
<220>
   <221> misc_feature
   <223> thrC-2046R
<400> 65
   ggccgttgat cattgttctt 20
<210> 66
   <211> 20
   <212> DNA
   <213> Artificial
<220>
   <223> Primer
<220>
   <221> misc_feature
   <223> murE-34F
<400> 66
   aactccacgc tggagctcac 20
<210> 67
   <211> 17
   <212> DNA
   <213> Artificial
<220>
   <223> Primer
<220>
   <221> misc_feature
   <223> murE-1944R
<400> 67
   agaacgcgga gtccacg 17
<210> 68
   <211> 521
   <212> PRT
   <213> Aminosäuresequenz von murE L121 F
<220>
   <221> MISC_FEATURE
   <223> MurE-Proteinsequenz
<400> 68
   Met Ala Thr Thr Leu Leu Asp Leu Thr Lys Leu Ile Asp Gly Ile Leu
   1 5 10 15
   Lys Gly Ser Ala Gln Gly Val Pro Ala His Ala Val Gly Glu Gln Ala
   20 25 30
   Ile Ala Ala Ile Gly Leu Asp Ser Ser Ser Leu Pro Thr Ser Asp Ala
   35 40 45
   Ile Phe Ala Ala Val Pro Gly Thr Arg Thr His Gly Ala Gln Phe Ala
   50 55 60
   Gly Thr Asp Asn Ala Ala Lys Ala Val Ala Ile Leu Thr Asp Ala Ala
   65 70 75 80
   Gly Leu Glu Val Leu Asn Glu Ala Gly Glu Thr Arg Pro Val Ile Val
   85 90 95
   Val Asp Asp Val Arg Ala Val Leu Gly Ala Ala Ser Ser Ser Ile Tyr
   100 105 110
   Gly Asp Pro Ser Lys Asp Phe Thr Phe Ile Gly Val Thr Gly Thr Ser
   115 120 125
   Gly Lys Thr Thr Thr Ser Tyr Leu Leu Glu Lys Gly Leu Met Glu Ala
   130 135 140
   Gly His Lys Val Gly Leu Ile Gly Thr Thr Gly Thr Arg Ile Asp Gly
   145 150 155 160
   Glu Glu Val Pro Thr Lys Leu Thr Thr Pro Glu Ala Pro Thr Leu Gln
   165 170 175
   Ala Leu Phe Ala Arg Met Arg Asp His Gly Val Thr His Val Val Met
   180 185 190
   Glu Val Ser Ser His Ala Leu Ser Leu Gly Arg Val Ala Gly Ser His
   195 200 205
   Phe Asp Val Ala Ala Phe Thr Asn Leu Ser Gln Asp His Leu Asp Phe
   210 215 220
   His Pro Thr Met Asp Asp Tyr Phe Asp Ala Lys Ala Leu Phe Phe Arg
   225 230 235 240
   Ala Asp Ser Pro Leu Val Ala Asp Lys Gln Val Val Cys Val Asp Asp
   245 250 255
   Ser Trp Gly Gln Arg Met Ala Ser Val Ala Ala Asp Val Gln Thr Val
   260 265 270
   Ser Thr Leu Gly Gln Glu Ala Asp Phe Ser Ala Thr Asp Ile Asn Val
   275 280 285
   Ser Asp Ser Gly Ala Gln Ser Phe Lys Ile Asn Ala Pro Ser Asn Gln
   290 295 300
   Ser Tyr Gln Val Glu Leu Ala Leu Pro Gly Ala Phe Asn Val Ala Asn
   305 310 315 320
   Ala Thr Leu Ala Phe Ala Ala Ala Ala Arg Val Gly Val Asp Gly Glu
   325 330 335
   Ala Phe Ala Arg Gly Met Ser Lys Val Ala Val Pro Gly Arg Met Glu
   340 345 350
   Arg Ile Asp Glu Gly Gln Asp Phe Leu Ala Val Val Asp Tyr Ala His
   355 360 365
   Lys Pro Ala Ala Val Ala Ala Val Leu Asp Thr Leu Arg Thr Gln Ile
   370 375 380
   Asp Gly Arg Leu Gly Val Val Ile Gly Ala Gly Gly Asp Arg Asp Ser
   385 390 395 400
   Thr Lys Arg Gly Pro Met Gly Gln Leu Ser Ala Gln Arg Ala Asp Leu
   405 410 415
   Val Ile Val Thr Asp Asp Asn Pro Arg Ser Glu Val Pro Ala Thr Ile
   420 425 430
   Arg Ala Ala Val Thr Ala Gly Ala Gln Gln Gly Ala Ser Glu Ser Glu
   435 440 445
   Arg Pro Val Glu Val Leu Glu Ile Gly Asp Arg Ala Glu Ala Ile Arg
   450 455 460
   Val Leu Val Glu Trp Ala Gln Pro Gly Asp Gly Ile Val Val Ala Gly
   465 470 475 480
   Lys Gly His Glu Val Gly Gln Leu Val Ala Gly Val Thr His His Phe
   485 490 495
   Asp Asp Arg Glu Glu Val Arg Ala Ala Leu Thr Glu Lys Leu Asn Asn
   500 505 510
   Lys Leu Pro Leu Thr Thr Glu Glu Gly
   515 520
<210> 69
   <211> 1566
   <212> DNA
   <213> Nukleotidsequenz von murE L121F
<220>
   <221> misc_feature
   <223> murE-Gensequenz
<400> 69
   atggcaacca cgttgctgga cctcaccaaa cttatcgatg gcatcctcaa gggctctgcc 60
   cagggcgttc ccgctcacgc agtaggggaa caagcaatcg cggctattgg tcttgactcc 120
   tccagcttac ctacctcgga cgctattttt gctgcagttc caggaacccg cactcacggc 180
   gcacagtttg caggtacgga taacgctgcg aaagctgtgg ccattttgac tgacgcagct 240
   ggacttgagg tgctcaacga agcaggagag acccgcccag tcatcgttgt tgatgatgtc 300
   cgcgcagtac ttggcgcagc atcatcaagc atttatggcg atccttcaaa agatttcacg 360
   ttcattggag tcactggaac ctcaggtaaa accaccacca gctacctctt ggaaaaagga 420
   ctcatggagg caggccacaa agttggtttg atcggcacca caggtacacg tattgacggg 480
   gaagaagtac ccacaaagct caccactcca gaagcgccga ctctgcaggc attgtttgct 540
   cgaatgcgcg atcacggtgt cacccacgtg gtgatggaag tatccagcca tgcattgtca 600
   ttgggcagag ttgcgggttc ccactttgat gtagctgcgt ttaccaacct gtcgcaggat 660
   caccttgatt tccaccccac catggatgat tactttgacg cgaaggcatt gttcttccgc 720
   gcagattctc cacttgtggc tgacaaacag gtcgtgtgcg tggatgattc ttggggtcag 780
   cgcatggcca gcgtggcagc ggatgtgcaa acagtatcca cccttgggca agaagcagac 840
   ttcagcgcta cagacatcaa tgtcagcgac tctggcgccc agagttttaa gatcaacgcc 900
   ccctcaaacc agtcctacca ggtcgagcta gctcttccag gtgcgttcaa cgttgctaac 960
   gccacgttgg catttgccgc tgcggcacgc gtgggtgttg atggcgaagc gtttgctcga 1020
   ggcatgtcca aggtcgcggt tccaggccgt atggaacgca ttgatgaggg acaagacttc 1080
   cttgcagtgg tggattatgc ccacaagcct gctgcagtgg ctgctgtgtt ggatacgttg 1140
   aggacccaga ttgacgggcg cctcggagtg gttatcggtg ctggtggaga ccgcgattcc 1200
   accaagcgtg gccccatggg gcagttgtcc gcacagcgtg ctgatctagt tattgtcact 1260
   gatgacaacc ctcgttcaga ggtgcctgcc acgattcgcg cagcagtcac tgcaggagca 1320
   cagcagggtg cttcagagtc cgaacgaccg gtggaagtcc tagaaattgg tgaccgtgca 1380
   gaagcaattc gcgttttggt cgagtgggca cagcctggag atggcattgt agtagctgga 1440
   aaaggccatg aagttggaca actagttgct ggtgtcaccc accattttga tgaccgcgaa 1500
   gaagttcgcg ctgctttgac agaaaagctc aacaataaac ttccccttac tacggaagaa 1560
   ggatag 1566
<210> 70
   <211> 28
   <212> DNA
   <213> Artificial
<220>
   <223> Primer
<220>
   <221> misc_feature
   <223> 7-39-L-F
<400> 70
   taggatcccg acaacatccc actgtctg 28
<210> 71
   <211> 27
   <212> DNA
   <213> Artificial
<220>
   <223> Primer
<220>
   <221> misc_feature
   <223> 7-39-R-R
<400> 71
   aagtcgacgt ctgcttcttg cccaagg 27
<210> 72
   <211> 240
   <212> PRT
   <213> enhanced yellow fluorescens protein (eyfp)
<400> 72
   Val Ser Lys Gly Glu Glu Leu Phe Thr Gly Val Val Pro Ile Leu Val
   1 5 10 15
   Glu Leu Asp Gly Asp Val Asn Gly His Lys Phe Ser Val Ser Gly Glu
   20 25 30
   Gly Glu Gly Asp Ala Thr Tyr Gly Lys Leu Thr Leu Lys Phe Ile Cys
   35 40 45
   Thr Thr Gly Lys Leu Pro Val Pro Trp Pro Thr Leu Val Thr Thr Phe
   50 55 60
   Gly Tyr Gly Leu Gln Cys Phe Ala Arg Tyr Pro Asp His Met Lys Gln
   65 70 75 80
   His Asp Phe Phe Lys Ser Ala Met Pro Glu Gly Tyr Val Gln Glu Arg
   85 90 95
   Thr Ile Phe Phe Lys Asp Asp Gly Asn Tyr Lys Thr Arg Ala Glu Val
   100 105 110
   Lys Phe Glu Gly Asp Thr Leu Val Asn Arg Ile Glu Leu Lys Gly Ile
   115 120 125
   Asn Phe Lys Glu Asp Gly Asn Ile Leu Gly His Lys Leu Glu Tyr Asn
   130 135 140
   Tyr Asn Ser His Asn Val Tyr Ile Met Ala Asp Lys Gln Lys Asn Gly
   145 150 155 160
   Ile Lys Val Asn Phe Lys Ile Arg His Asn Ile Glu Gly Gly Ser Val
   165 170 175
   Gln Leu Ala Asp His Tyr Gln Gln Asn Thr Pro Ile Gly Asp Gly Pro
   180 185 190
   Val Leu Leu Pro Asp Asn His Tyr Leu Ser Tyr Gln Ser Ala Leu Ser
   195 200 205
   Lys Asp Pro Asn Glu Lys Arg Asp His Met Val Leu Leu Glu Phe Val
   210 215 220
   Thr Ala Ala Gly Ile Thr Leu Gly Met Asp Glu Leu Tyr Lys Ser Arg
   225 230 235 240

## Beschreibung

Die vorliegende Erfindung betrifft eine gegenüber ihrem Wildtyp gentechnisch veränderte Zelle, ein Verfahren zum Identifizieren einer Zelle mit erhöhter intrazellulärer Konzentration eines bestimmten Metaboliten, ein Verfahren zur Herstellung einer gegenüber ihrem Wildtyp gentechnisch veränderten Zelle mit optimierter Produktion eines bestimmten Metaboliten, eine durch dieses Verfahren erhaltene Zelle, ein Verfahren zur Herstellung von Metaboliten sowie ein Verfahren zur Herstellung einer Mischung.

Mikrobiologisch hergestellte Metaboliten sind von großem wirtschaftlichem Interesse. So werden Aminosäuren wie L-Lysin, L-Threonin, L-Methionin und L-Tryptophan als Futtermittelzusatz, L-Glutamat als Gewürzzusatz, L-Isoleucin und L-Tyrosin in der pharmazeutischen Industrie, L-Arginin und L-Isoleucin als Medikament oder L-Glutamat, L-Aspartat und L-Phenylalanin als Ausgangssubstanz zur Synthese von Feinchemikalien verwendet. Ein anderes Beispiel eines aus technischer Sicht relevanten Metaboliten ist Oxoglutarat, welches Anwendung findet als Nahrungsergänzungsmittel oder als Vorstufe von Argenin-Alpha-Ketoglutarat, welches die Freisetzung von Wachstumshormonen und Insulin fördert.

Eine bevorzugte Methode zur Herstellung derartiger Metaboliten ist die biotechnologische Herstellung mittels Mikroorganismen. Insbesondere bei der Herstellung von Aminosäuren können auf diese Weise direkt die biologisch wirksame und optisch aktive Form des jeweiligen Metaboliten erhalten und darüber hinaus auch einfache und preisgünstige Rohstoffe eingesetzt werden. Als Mikroorganismen werden z. B. *Corynebacterium glutamicum*, seine Verwandten *ssp. flavum* und *ssp*. *lactofermentum* (Liebl et al., Int. J System Bacteriol. 1991, 41: 255 bis 260) oder auch *Escherichia coli* und verwandte Bakterien eingesetzt.

Bei der Herstellung der vorstehend beschriebenen Metabolite auf mikrobiologischem Wege wird üblicherweise durch Mutationen die Regulation der Biosynthese des jeweiligen Metaboliten so verändert, dass sie ihn über den Eigenbedarf hinaus produzieren und in das Medium ausscheiden. So offenbart beispielsweise WO-A-2005/059139 die Herstellung von L-Lysin mittels eines gentechnisch veränderten *Corynebacterium glutamicum*-Stammes, bei dem eineerhöhte L-Lysin-Produktion durch eine Verbesserung der Metabolisierung über den Pentosephosphat-Stoffwechselweg erzielt wird. In WO-A-97/23597 wird eine Erhöhung der Produktion von Aminosäuren wie L-Lysin in Mikroorganismen dadurch erzielt, dass Aktivität von Exportcarriern, welche diese Aminosäuren aus der Zelle heraus schleusen, erhöht wird.

Derartige Überproduzenten erhält man üblicherweise durch die Suche nach Mutanten, welche den Metaboliten in besonders großer Menge produzieren. Diese Suche wird als "*Screening*" bezeichnet. Im Screening werden in einem Ausgangsstamm meistens mittels gebräuchlicher chemischer oder physikalischer Mutagene (z. B. MNNG oder UV) zufällige Mutationen (ungerichtete Mutagenese) induziert und mit üblichen mikrobiologischen Methoden Mutanten selektioniert. Eine andere Möglichkeit der Bereitstellung von Metabolit-Überproduzenten besteht darin, durch gezielte Gen-Überexpressionen oder Deletionen bestimmte Synthesewege zu verstärken oder konkurrierende Synthesewege zu vermeiden.

Problematisch hierbei ist allerdings, dass insbesondere bei der ungerichteten Mutagenese in einer Ansammlung von Zellen schwer zu detektieren ist, in welchen der Zellen eine Mutation erfolgt ist, die zu einer verstärkten intrazellulären Synthese des im Focus stehenden Metaboliten geführt hat. Die hierzu erforderlichen Screening-Verfahren sind sehr zeitaufwendig und kostspielig.

Smolke et al. (Appl Microbiol Biotechnol. (2001), Vol. 57 (5-6), Seiten 689-696) beschreiben den Effekt der Homogenität der Transkriptionsinduktion und der Stabilität des Transkripts auf die Expression zweier Gene in einem konstruierten Operon.

Vesala et al. (World Journal of Microbiology and Biotechnology (2008), Vol. 24 (3), Seiten 353-359) beschreiben die Entwicklung eines grünen Ganzzellen-Fluoreszenzsensors zur quantitativen Lysin-Bestimmung.

Chavola et al. (Applied Microbiology and Biotechnology (2007), Vol. 76 (1), Seiten 91-99) beschreiben die Quantifizierung der Lysin-Menge in proteinhaltigen Futtermitteln unter Verwendung eines grünen Ganzzellen-*E*.*coli*-Fluoreszenzsensors

Li et al. (Biotechnol. Bioeng. (2000), Vol. 70 (2), Seiten 187-196) beschreiben die Verwendung eines grünen Fluoreszenzproteins (GFP) zur Untersuchung der Regulation des Galaktose-induzierbaren GAL1-Promotors im Hefestamm *Saccharomyces cerevisiae*.

Ye et al. (Applied Microbiology and Biotechnology (2000), Vol. 54 (1), Seiten 90-96) offenbaren die Konstruktion einer gentechnisch veränderten Hefe mit einer durch Glukose induzierbaren grünen Fluoreszenzemission von der Zelloberfläche.

Taylor et al. (Anal Biochem. (2004), Vol. 328 (1), Seiten 60-66) beschreiben die Konstruktion eines Ganzzellen-Genreporters für einen Nitrat-Fluoreszenz-Bioassay.

Hilmi et al. (Microbiology (2006), Vol. 152 (5), Seiten 1489-1496) befassen sich mit Nisin Z, einem post-translational modifizierten antimikrobiellen Peptid aus *Lactococcus lactis* und der Autoregulation dieses Peptides durch extrazelluläres Nisin.

Luers et al. (Eur. J. Cell Biol. (2000), Vol. 79 (9), Seiten 653-657) beschreiben die Überwachung einer transgenen Expression in lebenden Zellen unter Verwendung eines Ecdyson-induzierbaren Promotorsystems.

Der vorliegenden Erfindung lag die Aufgabe zugrunde, die sich aus dem Stand der Technik ergebenden Nachteile im Zusammenhang mit der Detektion gentechnisch veränderte Zellen, welche einen bestimmten Metaboliten überproduzieren, zu überwinden.

Insbesondere lag der vorliegenden Erfindung die Aufgabe zugrunde, eine gentechnisch veränderte Zelle bereitzustellen, in der nach einer Mutation diejenigen Mutanten, die eine Überproduktion eines bestimmten Metaboliten bewirken, in einfacher Weise identifiziert und gegebenenfalls von den übrigen Zellen abgetrennt werden können.

Eine weitere, der vorliegenden Erfindung zugrunde liegende Aufgabe bestand darin, ein Verfahren zum Identifizieren einer Zelle mit erhöhter intrazellulärer Konzentration eines bestimmten Metaboliten anzugeben, welches in besonders einfacher und kostengünstiger Weise ein Identifizieren und gegebenenfalls gezieltes Abtrennen einer solchen Zelle in bzw. aus einer Vielzahl von Zellen, beispielsweise in bzw. aus einer Zellsuspension, ermöglicht.

Auch lag der vorliegenden Erfindung die Aufgabe zugrunde, eine Zelle mit optimierter Produktion eines bestimmten Metaboliten bereitzustellen, in der gezielt Gene oder Mutationen eingebracht bzw. durch gezielte Mutationen erzeugt werden, welche durch das vorstehend beschriebene Screening-Verfahren als für eine Überproduktion dieses Metaboliten vorteilhaft identifiziert worden sind.

Einen Beitrag zur Lösung der eingangs genannten Aufgaben leistet eine gegenüber ihrem Wildtyp gentechnisch veränderte Zelle wie in Anspruch 1 definiert.

Unter dem Begriff "*Metabolit*", wie er hierin verwendet wird, soll ganz allgemein ein Zwischenprodukt eines biochemischen Stoffwechselweges verstanden werden, wobei erfindungsgemäß Aminosäuren oder Aminosäurederivate, beispielsweise L-Isoleucin, L-Leucin, L-Valin, L-Lysin, L-Arginin, L-Citrullin, L-Histidin, L-Methionin, L-Cystein L-Tryptophan, L-Glyzin oder O-Acetyl-L-Serin, Nukleotide oder Nukleotidderivate, beispielsweise Xanthin, GTP oder zyklisches Diguanosinmonophosphat, Fettsäuren oder Fettsäurederivate, beispielsweise Acyl-Coenzym A-Thioester, Zucker oder Zuckerderivate, beispielsweise Glucose, Rhamnose, Ribulose-bis-phosphat, beta-D-Galactoside oder D-Glucosamin-6-phosphat, Ketosäuren, beispielsweise Oxoglutarat, Antibiotika, beispielsweise Thienamycin, Avilamycin, Nocardicin oder Tetrazycline, Vitamine oder Vitaminderivate, beispielsweise Biotin oder Thiaminpyrophosphat oder Purinalkaloide, beispielsweise Theophyllin. Unter "Derivaten" der vorstehend beschriebenen Metaboliten werden insbesondere Amine, Phosphate oder Ester der entsprechenden Verbindungen verstanden.. Ganz besonders bevorzugte Metaboliten sind Aminosäuren, insbesondere eine Aminosäure ausgewählt aus der Gruppe bestehend aus L-Isoleucin, L-Leucin, L-Valin, L-Lysin, L-Arginin, L-Citrullin, L-Histidin, L-Methionin, L-Cystein, L-Tryptophan, O-Acetyl-L-Serin, besonders bevorzugt aus der Gruppe bestehend aus L-Lysin, L-Arginin, L-Citrullin und L-Histidin. Der erfindungsgemäß am meisten bevorzugte Metabolit ist L-Lysin.

Unter einem "*Wildtyp*" einer Zelle wird vorzugsweise eine Zelle verstanden, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "*Wildtyp*" fallen daher insbesondere nicht solche Zellen bzw. solche Gene, deren Gensequenzen zumindest teilweise durch den Menschen mittels rekombinanter Verfahren verändert worden sind.

Erfindungsgemäß besonders bevorzugte Zellen sind diejenigen der Gattungen *Corynebacterium, Brevibacterium, Bacillus, Lactobacillus, Lactococcus*, *Candida*, *Pichia*, *Kluveromyces*, *Saccharomyces*, *Escherichia, Zymomonas, Yarrowia, Methylobacterium, Ralstonia* und *Clostridium,* wobei *Brevibacterium flavum*, *Brevibacterium lactofermentum*, *Escherichia coli*, *Saccharomyces cerevisiae*, *Kluveromyces lactis*, *Candida blankii*, *Candida rugosa*, *Corynebacterium glutamicum*, *Corynebacterium efficiens, Zymonomas mobilis, Yarrowia lipolytica, Methylobacterium extorquens, Ralstonia eutropha* und *Pichia pastoris* besonders bevorzugt sind. Erfindungsgemäß am meisten bevorzugte Zellen sind solche der Gattung *Corynebacterium* und *Escherichia*, wobei *Corynebacterium glutamicum* und *Escherichia coli* ganz besonders bevorzugte Bakterienstämme sind.

Insbesondere in dem Fall, in dem der Metabolit L-Lysin ist, können sich die gentechnisch veränderten Zellen insbesondere von Zellen ausgewählt aus der Gruppe bestehend aus *Corynebacterium glutamicum* ATCC13032, *Corynebacterium acetoglutamicum* ATCC15806, *Corynebacterium acetoacidophilum* ATCC13870, *Corynebacterium melassecola* ATCC17965, *Corynebacterium thermoaminogenes* FERM BP-1539, Brevibacterium *flavum* ATCC14067, *Brevibacterium lactofermentum* ATCC13869 und *Brevibacterium divaricatum* ATCC14020, und daraus hergestellte L-Aminosäuren produzierende Mutanten bzw. Stämme wie beispielsweise die L-Lysin produzierenden Stämme *Corynebacterium glutamicum* FERM-P 1709, *Brevibacterium flavum* FERM-P 1708, *Brevibacterium lactofermentum* FERM-P 1712, *Corynebacterium glutamicum* FERM-P 6463, *Corynebacterium glutamicum* FERM-P 6464 und *Corynebacterium glutamicum* DSM 5715 oder wie beispielsweise der L-Methionin produzierende Stamm *Corynebacterium glutamicum* ATCC21608 ableiten. Als Beispiele geeigneter *Escherichia coli*-Stämme seien *Escherichia coli* AJ11442 (siehe JP 56-18596 und US 4,346,170), *Escherichia coli*-Stamm VL611 und *Escherichia coli*-Stamm WC 196 (siehe WO-A-96/17930) genannt.

Die erfindungsgemäßen, gegenüber ihrem Wildtyp gentechnisch veränderten Zellen sind nun dadurch gekennzeichnet, dass sie eine für ein Autofluoreszenzprote-. in kodierende Gensequenz beinhalten, wobei die Expression dieses Autofluoreszenzproteins von der intrazellulären Konzentration eines bestimmten Metaboliten abhängig ist.

Als für ein Autofluoreszenzprotein kodierende Gensequenz kommen alle dem Fachmann bekannten Gensequenzen in Betracht, welche für ein Autofluoreszenzprotein kodieren. Besonders bevorzugt sind Gensequenzen, welche für fluoreszierende Proteine der Gattung *Aequora,* wie das Green Fluorescent Protein (GFP), und Varianten davon, die in einem anderen Wellenlängenbereich fluoreszieren (z.B. Yellow Fluorescent Protein, YFP; Blue Fluorescent Protein, BFP; Cyan Fluorescent Protein, CFP) oder deren Fluoreszenz verstärkt ist (enhanced GFP oder EGFP, beziehungsweise EYFP, EBFP oder ECFP), kodieren. Ferner können erfindungsgemäß auch Gensequenzen verwendet werden, welche für andere autofluoreszierende Proteine, z.B. DsRed, HcRed, AsRed, AmCyan, ZsGreen, AcGFP, ZsYellow, wie sie von BD Biosciences, Franclin Lakes, USA, bekannt sind, kodieren.

Das Merkmal, wonach die Expression des Autofluoreszenzproteins von der intrazellulären Konzentration eines bestimmten Metaboliten abhängig ist und damit durch die Zelle in Abhängigkeit von dieser Metaboliten-Konzentration kontrolliert werden kann, lässt sich nun erfindungsgemäß auf verschiedene Art und Weise realisieren.

Gemäß einer ersten besonderen Ausführungsform der erfindungsgemäßen Zelle erfolgt die Kontrolle der Expression der für das Autofluoreszenzprotein kodierenden Gensequenz in Abhängigkeit von der intrazellulären Konzentration des bestimmten Metaboliten auf der Ebene der Transkription. In Abhängigkeit von der intrazellulären Konzentration des jeweiligen Metaboliten wird mithin mehr oder weniger mRNA gebildet, die in den Ribosomen unter Bildung des Autofluoreszenzproteins translatiert werden kann.

Im Zusammenhang mit dieser ersten besonderen Ausführungsform der erfindungsgemäßen Zelle kann die Kontrolle der Expression auf der Ebene der Translation dadurch erfolgen, dass sich die für das Autofluoreszenzprotein kodierende Gensequenz unter der Kontrolle eines heterologen Promotors befindet, der im Wildtyp der Zelle die Expression eines Gens kontrolliert, dessen Expression in der Wildtyp-Zelle von der intrazellulären Konzentration eines bestimmten Metaboliten abhängig ist. Auch kann sich die die für das Autofluoreszenzprotein kodierende Gensequenz unter der Kontrolle eines Promotors befinden, der sich von einem solchen Promotor ableitet.

Die Formulierung "*unter der Kontrolle eines heterologen Promotors*" zeigt an, dass der Promotor in natürlicherweise, insbesondere in der Wildtyp-Zelle, aus der die Promotorsequenz isoliert und gegebenenfalls zur weiteren Steigerung der Promotoreffizienz gentechnisch verändert wurde, nicht die Expression des für das Autofluoreszenzprotein kodierenden Gensequenz reguliert. In diesem Zusammenhang bedeutet die Formulierung "*der sich von einem solchen Promotor ableitet*", dass der in der gentechnisch veränderten Zelle enthaltene Promotor, der die Expression des für das Autofluoreszenzprotein kodierenden Gensequenz reguliert, kein Promotor sein muss, der mit identischer Nukleinsäuresequenz in einer Wildtyp-Zelle enthalten sein muss. Vielmehr kann diese Promotorsequenz zum Zwecke der Steigerung der Promotoreffektivität etwa durch Insertion, Deletion oder Austausch einzelner Basen, beispielsweise durch Palindromisierung einzelner Nukleinsäure-Sequenzen, verändert worden sein. Auch muss der Promotor, der die Expression des für das Autofluoreszenzprotein kodierenden Gensequenz reguliert, nicht zwingend ein Promotor sein oder sich von einem Promotor ableiten, der im Genom der gentechnisch veränderten Zelle selbst enthalten ist. Dennoch kann es sich als durchaus vorteilhaft erweisen, wenn der Promotor ein Promotor ist bzw. sich von einem Promotor ableitet, der im Genom der gentechnisch veränderten Zelle selbst enthalten ist, dort aber die Expression eines Gens kontrolliert, dessen Expression von der intrazellulären Konzentration eines bestimmten Metaboliten abhängig ist.

Die für das Autofluoreszenzprotein kodierende Gensequenz befindet sich bei dieser Ausführungsform der erfindungsgemäßen Zelle unter der Kontrolle eines Promotors. Der Begriff "*unter der Kontrolle eines Promotors*" ist dabei vorzugsweise so zu verstehen, dass die für das Autofluoreszenzprotein kodierende Gensequenz mit dem Promotor funktionell verknüpft ist. Funktionell verknüpft sind der Promotor und die für das Autofluoreszenzprotein kodierende Gensequenz, wenn diese beiden Sequenzen sowie gegebenenfalls weitere regulative Elemente, wie zum Beispiel ein Terminator, derart sequentiell angeordnet sind, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die für das Autofluoreszenzprotein kodierende Gensequenz hinter (d. h. am 3'-Ende) der Promotorsequenz positioniert wird, so dass beide Sequenzen kovalent miteinander verbunden sind. Bevorzugt ist dabei der Abstand zwischen der für das Autofluoreszenzprotein kodierenden Gensequenz und der Promotorsequenz kleiner als 200 Basenpaare, besonders bevorzugt kleiner als 100 Basenpaare, ganz besonders bevorzugt kleiner als 50 Basenpaare. Auch ist es möglich, dass die für das Autofluoreszenzprotein kodierende Gensequenz und der Promotor derart funktionell miteinander verknüpft sind, dass zwischen diesen beiden Gensequenzen noch eine Teilsequenz des homologen Gens (also desjenigen Gens, dessen Expression in der Wildtyp-Zelle durch den Promotor reguliert wird) befinden. Bei der Expression eines solchen DNA-Konstruktes wird ein Fusionsprotein aus dem Autofluoreszenzprotein und der Aminosäuresequenz, welche von der entsprechenden Teilsequenz des homologen Gens kodiert wird, erhalten. Die Länge solcher Teilsequenzen des homologen Gens sind unkritisch, solange die die Funktionsfähigkeit des Autofluoreszenzproteins, also seine Eigenschaft, bei Anregung mit Licht einer bestimmten Wellenlänge zu fluoreszieren, nicht nennenswert beeinträchtigt wird.

Neben dem Promotor und der für das Autofluoreszenzprotein kodierenden Gensequenz kann die erfindungsgemäße Zelle gemäß dieser besonderen Ausgestaltung auch eine für den Regulator kodierende Gensequenz umfassen, wobei der Regulator vorzugsweise ein Protein ist, welches in irgendeiner Weise mit dem Metaboliten und dem Promotor wechselwirkt und auf diese Weise die Bindungsaffinität der Promotorsequenz zur RNA-Polymerase beeinflusst. Die Wechselwirkung zwischen dem Regulator und der Promotorsequenz ist dabei von der Anwesenheit des Metaboliten abhängig. In der Regel wird der Metabolit an bestimmten, funktionellen Bereichen des Regulators gebunden und bewirkt auf diese Weise eine Konformationsänderung des Regulators, die sich auf die Wechselwirkung zwischen dem Regulator und der Promotorsequenz auswirkt. Der Regulator kann dabei grundsätzlich ein Aktivator oder ein Repressor sein.

Als Promotoren kommen erfindungsgemäß grundsätzlich alle Promotoren in Betracht, die normalerweise über eine funktionelle Verknüpfung die Expression eines Gens kontrollieren, dessen Expression von der intrazellulären Konzentration eines bestimmten Metaboliten abhängig ist. Ganz besonders bevorzugt handelt es sich bei dem Promotor um einen Promotor, der normalerweise die Expression eines Gens kontrolliert, dessen Expression von der intrazellulären Konzentration eines bestimmten Metaboliten abhängig ist und das für ein Protein kodiert, welches die Verminderung der intrazellulären Konzentration eines Metaboliten entweder über eine chemische Umsetzung des Metaboliten oder über das Ausschleusen des Metaboliten aus der Zelle ermöglicht. Dieses Protein ist daher entweder ein Enzym, welches die Umsetzung des Metaboliten in ein von dem Metaboliten verschiedenes Stoffwechselprodukt katalysiert, oder aber ein aktiver oder passiver Transporter, der den Efflux des Metaboliten aus der Zelle heraus katalysiert.

Weiterhin kann es sich bei den Promotoren um solche Promotoren handeln, welche in Gegenwart des Metaboliten mit bestimmten Aktivatoren interagieren und so die Expression der für das Autofluoreszenzprotein kodierenden Gensequenz bewirken, oder aber Promotoren, welche durch einen Repressor gehemmt sind, wobei der Repressor durch Interaktion mit einem bestimmten Metaboliten vom Promotor abdiffundiert, wodurch die Hemmung beseitigt und die Expression der für das Autofluoreszenzprotein kodierenden Gensequenz bewirkt wird.

Geeignete Beispiele für erfindungsgemäße Zellen dieser ersten besonderen Ausführungsform werden nun nachfolgend näher beschrieben. Es sei jedoch an dieser Stelle betont, dass die vorliegende Erfindung nicht auf die nachstehenden, unter die erste besondere Ausführungsform der erfindungsgemäßen Zelle fallenden Beispiele beschränkt ist.

So kann es sich bei der gentechnisch veränderten Zelle gemäß der ersten Ausführungsform um eine gentechnisch veränderte Zelle, vorzugsweise um eine gentechnisch veränderte *Pseudomonas putida*-Zelle handeln, die eine für ein Autofluoreszenzprotein kodierende Gensequenz umfasst, die sich unter der Kontrolle des *bkd*-Promotors befindet (zum BkdR-Regulator in *Pseudomonas putida* siehe beispielsweise J. Bact., 181 (1999), Seiten 2.889-2.894, J. Bact., 187 (2005), Seite 664;). Hier führt eine erhöhte intrazelluläre Konzentration an L-Isoleucin, L-Leucin, L-Valin oder D-Leucin zu einer Expression des Autofluoreszenzproteins. Eine solche Zelle weist neben dem *bkd*-Promotor und der unter der Kontrolle dieses Promotors befindlichen Gensequenz für ein Autofluoreszenzprotein vorzugsweise auch eine für den BkdR-Regulator (branched-chain keto acid dehydrogenase regulatory protein) kodierende Gensequenz auf. Die DNA-Sequenz des vom BdkR-Regulator regulierten *bkd*-Promotors ist in SEQ.-ID.-Nr. 01 und die Sequenz des BkdR-Regulators selbst in SEQ.-ID.-Nr. 02 wiedergegeben.

Weiterhin kann es sich bei der gentechnisch veränderten Zelle gemäß der ersten Ausführungsform um eine gentechnisch veränderte Zelle, vorzugsweise um eine gentechnisch veränderte *Bacillus subtilis*-Zelle handeln, die eine für ein Autofluoreszenzprotein kodierende Gensequenz umfasst, die sich unter der Kontrolle des *ackA*-Promotors befindet (zum CodY-Repressor siehe Mol. Mic. 62 (2006), Seite 811). Auch hier führt eine erhöhte intrazelluläre Konzentration an L-Isoleucin, L-Leucin und L-Valin zu einer Expression des Autofluoreszenzproteins. Eine solche Zelle weist neben dem *ackA*-Promotor und der unter der Kontrolle dieses Promotors befindlichen Gensequenz für ein Autofluoreszenzprotein vorzugsweise auch eine für den CodY-Repressor kodierende Gensequenz auf. Die DNA-Sequenz des vom CodY-Aktivator regulierten *ackA*-Promotors ist in SEQ.-ID.-Nr. 03 und die Sequenz des CodY-Aktivators selbst in SEQ.-ID.-Nr. 04 wiedergegeben.

Auch kann es sich bei der gentechnisch veränderten Zelle gemäß der ersten Ausführungsform um eine gentechnisch veränderte Zelle, vorzugsweise um eine gentechnisch veränderte *Pseudomonas putida*-Zelle handeln, die eine für ein Autofluoreszenzprotein kodierende Gensequenz umfasst, die sich unter der Kontrolle des *mdeA*-Promotors befindet (zum MdeR-Regulator siehe J. Bacteriol., 179 (1997), Seite 3.956). Hier führt eine erhöhte intrazelluläre Konzentration an L-Methionin zu einer Expression des Autofluoreszenzproteins. Eine solche Zelle weist neben dem *mdeA*-Promotor und der unter der Kontrolle dieses Promotors befindlichen Gensequenz für ein Autofluoreszenzprotein vorzugsweise auch eine für den MdeR-Regulator kodierende Gensequenz auf. Die DNA-Sequenz des vom MdeR-Regulator regulierten *mdeA*-Promotors ist in SEQ.-ID.-Nr. 05 und die Sequenz des MdeR-Regulators selbst in SEQ.-ID.-Nr. 06 wiedergegeben.

Weiterhin kann es sich bei der gentechnisch veränderten Zelle gemäß der ersten Ausführungsform um eine gentechnisch veränderte Zelle, vorzugsweise um eine gentechnisch veränderte *Corynebacterium glutamicum*-Zelle handeln, die eine für ein Autofluoreszenzprotein kodierende Gensequenz umfasst, die sich unter der Kontrolle des *brnF*-Promotors befindet (zum Lrp-Regulator in *Corynebacterium glutamicum* siehe J. Bact., 184 (14) (2002), Seiten 3.947-3.956). Hier führt eine erhöhte intrazelluläre Konzentration an L-Isoleucin, L-Leucin und L-Valin zu einer Expression des Autofluoreszenzproteins. Eine solche Zelle weist neben dem *brnF*-Promotor und der unter der Kontrolle dieses Promotors befindlichen Gensequenz für ein Autofluoreszenzprotein vorzugsweise auch eine für den Lrp-Regulator kodierende Gensequenz auf. Die DNA-Sequenz des vom Lrp-Regulator regulierten *brnF*-Promotors ist in SEQ.-ID.-Nr. 07 und die Sequenz des Lrp-Regulators selbst in SEQ.-ID.-Nr. 08 wiedergegeben.

Weiterhin kann es sich bei der gentechnisch veränderten Zelle gemäß der ersten Ausführungsform um eine gentechnisch veränderte Zelle, vorzugsweise um eine gentechnisch veränderte *Escherichia coli*-Zelle handeln, die eine für ein Autofluoreszenzprotein kodierende Gensequenz umfasst, die sich unter der Kontrolle des *cysP*-Promotors befindet (zum CysB-Regulator in *Escherichia coli* siehe Mol. Mic., 53 (2004), Seite 791). Hier führt eine erhöhte intrazelluläre Konzentration an O-Acetyl-L-Serin zu einer Expression des Autofluoreszenzproteins. Eine solche Zelle weist neben dem *cysP*-Promotor und der unter der Kontrolle dieses Promotors befindlichen Gensequenz für ein Autofluoreszenzprotein vorzugsweise auch eine für den CysB-Regulator kodierende Gensequenz auf. Die DNA-Sequenz des vom CysB-Regulator regulierten *cysP*-Promotors ist in SEQ.-ID.-Nr. 09 und die Sequenz des Lrp-Regulators selbst in SEQ.-ID.-Nr. 10 wiedergegeben.

Auch kann es sich bei der gentechnisch veränderten Zelle gemäß der ersten Ausführungsform um eine gentechnisch veränderte Zelle, vorzugsweise um eine gentechnisch veränderte *Escherichia coli*-Zelle handeln, die eine für ein Autofluoreszenzprotein kodierende Gensequenz umfasst, die sich unter der Kontrolle des *cadB*-Promotors befindet (zum CadC-Regulator in *Escherichia coli* siehe Mol. Mic. 51 (2004), Seiten 1.401-1.412). Hier führt eine erhöhte intrazelluläre Konzentration an Diaminen wie Cadaverin oder Putrescin zu einer Expression des Autofluoreszenzproteins. Eine solche Zelle weist neben dem *cadB*-Promotor und der unter der Kontrolle dieses Promotors befindlichen Gensequenz für ein Autofluoreszenzprotein vorzugsweise auch eine für den CadC-Regulator kodierende Gensequenz auf. Die DNA-Sequenz des vom CadC-Regulator regulierten *cadB-*Promotors ist in SEQ.-ID.-Nr. 11 und die Sequenz des CadC-Regulators selbst in SEQ.-ID.-Nr. 12 wiedergegeben.

Weiterhin kann es sich bei der gentechnisch veränderten Zelle gemäß der ersten Ausführungsform um eine gentechnisch veränderte Zelle, vorzugsweise um eine gentechnisch veränderte *Corynebacterium glutamicum*-Zelle handeln, die eine für ein Autofluoreszenzprotein kodierende Gensequenz umfasst, die sich unter der Kontrolle des *metY*-, *metK*-, *hom*-, *cysK*-, *cysI*- oder *suuD*-Promotors befindet (zum McbR-Regulator in *Corynebacterium glutamicum* und der Promotorsequenzen, die dadurch reguliert werden, siehe Mol. Mic. 56 (2005), Seiten 871-887). Hier führt eine erhöhte intrazelluläre Konzentration an S-Adenosylhomocystein zu einer Expression des Autofluoreszenzproteins. Eine solche Zelle weist neben dem *metY*-, *metK*-, *hom*-, *cysK*-, *cysI*- oder *suuD*-Promotor und der unter der Kontrolle dieses Promotors befindlichen Gensequenz für ein Autofluoreszenzprotein vorzugsweise auch eine für den McbR-Regulator kodierende Gensequenz auf. Die DNA-Sequenz des vom McB-Regulator regulierten *metY*-Promotors ist in SEQ.-ID.-Nr. 13 und die Sequenz des MecR-Regulators selbst in SEQ.-ID.-Nr. 14 wiedergegeben.

Auch kann es sich bei der gentechnisch veränderten Zelle gemäß der ersten Ausführungsform um eine gentechnisch veränderte Zelle, vorzugsweise um eine gentechnisch veränderte *Escherichia coli*-Zelle handeln, die eine für ein Autofluoreszenzprotein kodierende Gensequenz umfasst, die sich unter der Kontrolle des *argO*-Promotors befindet. Hier führt eine erhöhte intrazelluläre Konzentration an L-Lysin zu einer Expression des Autofluoreszenzproteins. Eine solche Zelle weist neben dem *argO*-Promotor und der unter der Kontrolle dieses Promotors befindlichen Gensequenz für ein Autofluoreszenzprotein vorzugsweise auch eine für den ArgP-Regulator kodierende Gensequenz auf. Die DNA-Sequenz des vom ArgO-Regulator regulierten *argO*-Promotors ist in SEQ.-ID.-Nr. 15 und die Sequenz des ArgP-Regulators selbst in SEQ.-ID.-Nr. 16 wiedergegeben.

Darüberhinaus kann es sich bei der gentechnisch veränderten Zelle gemäß einer besonders bevorzugten Ausgestaltung der ersten Ausführungsform um eine gentechnisch veränderte Zelle, vorzugsweise um eine gentechnisch veränderte *Corynebacterium glutamicum*-Zelle handeln, die eine für ein Autofluoreszenzprotein kodierende Gensequenz umfasst, die sich unter der Kontrolle des *lysE*-Promotors befindet (zum *lysE*-Promotor und seinem Regulator LysG siehe Microbiology, 147 (2001), Seite 1.765). Hier führt eine erhöhte intrazelluläre Konzentration an L-Lysin, L-Arginin, L-Histidin und L- Citrullin zu einer Expression des Autofluoreszenzproteins. Eine solche Zelle weist neben dem *lysE*-Promotor und der unter der Kontrolle dieses Promotors befindlichen Gensequenz für ein Autofluoreszenzprotein vorzugsweise auch eine für den LysG-Regulator kodierende Gensequenz auf. Die DNA-Sequenz des vom LysG-Regulator regulierten *lysE-*Promotors ist in SEQ.-ID.-Nr. 17 und die Sequenz des LysG-Regulators selbst in SEQ.-ID.-Nr. 18 wiedergegeben.

In *Corynebacterium glutamicum* kodiert das *lysE*-Gen für einen sekundären Carrier, der weder auf molekularer noch auf struktureller Ebene Ähnlichkeiten zu einer der 12 bekannten Transportersuperfamilien aufweist, die am Efflux von organischen Molekülen und Kationen beteiligt sind. Aufgrund der neuartigen Funktion und ungewöhnlichen Struktur wurde LysE als erstes Mitglied einer neuen Translokatorfamilie identifiziert. Mittlerweile konnten dieser Familie im Rahmen der Genomsequenzierungen zahlreiche Proteine allerdings bisher noch weitgehend unbekannter Funktion zugeordnet werden. Die LysE-Familie, zu der LysE gehört, bildet mit der RhtB Familie und der CadD Familie die LysE-Superfamilie, der bisher insgesamt 22 Mitglieder zugeordnet sind. Aus der LysE-Familie ist der Lysin-Exporter aus *Corynebacterium glutamicum* das bislang einzige funktionell charakterisierte Mitglied. Auf genetischer Ebene wird *lysE* durch den Regulator LysG (*governing L-lysine export*) reguliert. LysG weist hohe Ähnlichkeiten zu bakteriellen Regulatorproteinen der LTTR-Familie *(LysR type transcriptional regulator*) auf. Dabei wirkt L-Lysin als Induktor der LysG-vermittelten Transkription von *lysE*. Neben L-Lysin sind auch die beiden basischen Aminosäuren L-Arginin und L-Histidin, sowie L-Citrullin Induktoren der LysG-vermittelten *lysE-*Expression.

Weiterhin kann es sich bei der gentechnisch veränderten Zelle gemäß der ersten besonderen Ausführungsform um eine gentechnisch veränderte Zelle, vorzugsweise um eine gentechnisch veränderte *Escherichia coli*-Zelle handeln, die eine für ein Autofluoreszenzprotein kodierende Gensequenz umfasst, die sich unter der Kontrolle des *fadE-* oder *fadBA*-Promotors befindet (zum FadR-Regulator in *Escherichia coli* siehe beispielsweise Mol. Biol., 29 (4) (2002), Seiten 937-943). Hier führt eine erhöhte intrazelluläre Konzentration an Acyl-Coenzym A zu einer Expression des Autofluoreszenzproteins. Eine solche Zelle weist neben dem *fadE-*oder *fadBA*-Promotor und der unter der Kontrolle dieses Promotors befindlichen Gensequenz für ein Autofluoreszenzprotein vorzugsweise auch eine für den FadR-Regulator kodierende Gensequenz auf. Die DNA-Sequenz des vom FadR-Regulator regulierten *fadE*-Promotors ist in SEQ.-ID.-Nr. 19 und die Sequenz des LysG-Regulators selbst in SEQ.-ID.-Nr. 20 wiedergegeben.

Auch kann es sich bei der gentechnisch veränderten Zelle gemäß der ersten besonderen Ausführungsform um eine gentechnisch veränderte Zelle, vorzugsweise um eine gentechnisch veränderte *Bacillus subtilis*-Zelle handeln, die eine für ein Autofluoreszenzprotein kodierende Gensequenz umfasst, die sich unter der Kontrolle des *fadM*-Promotors befindet (zum FabR-Regulator in *Bacillus subtilis* siehe beispielsweise J. Bacteriol., 191 (2009), Seiten 6.320-6.328). Auch hier führt eine erhöhte intrazelluläre Konzentration an Acyl-Coenzym A zu einer Expression des Autofluoreszenzproteins. Eine solche Zelle weist neben dem *fadM*-Promotor und der unter der Kontrolle dieses Promotors befindlichen Gensequenz für ein Autofluoreszenzprotein vorzugsweise auch eine für den FabR-Regulator kodierende Gensequenz auf. Die DNA-Sequenz des vom FabR-Regulator regulierten *fadM*-Promotors ist in SEQ.-ID.-Nr. 21 und die Sequenz des FabR-Regulators selbst in SEQ.-ID.-Nr. 22 wiedergegeben.

Weiterhin kann es sich bei der gentechnisch veränderten Zelle gemäß der ersten besonderen Ausführungsform um eine gentechnisch veränderte Zelle, vorzugsweise um eine gentechnisch veränderte *Escherichia coli*-Zelle handeln, die eine für ein Autofluoreszenzprotein kodierende Gensequenz umfasst, die sich unter der Kontrolle des *rhaSR-, rhaBAD-* oder *rhaT*-Promotors befindet (zum RhaR- und RhaS-Regulator in *Escherichia coli* siehe beispielsweise J. Bacteriol., 189 (1) (2007), 269-271). Hier führt eine erhöhte intrazelluläre Konzentration an Rhamnose zu einer Expression des Autofluoreszenzproteins. Eine solche Zelle weist neben dem *rhaSR-, rhaBAD-* oder *rhaT*-Promotor und der unter der Kontrolle dieses Promotors befindlichen Gensequenz für ein Autofluoreszenzprotein vorzugsweise auch eine für den RhaR- oder RhaS-Regulator kodierende Gensequenz auf. Die DNA-Sequenz des vom RhaR-Regulator regulierten *rhaSR*-Promotors ist in SEQ.-ID.-Nr. 23, die Sequenz des *rhaBAD*-Promotors in SEQ.-ID.-Nr. 24, die Sequenz des RhaR-Regulators in SEQ.-ID.-Nr. 25 und die Sequenz des RhaS-Regulators in SEQ.-ID.-Nr. 26 wiedergegeben.

Auch kann es sich bei der gentechnisch veränderten Zelle gemäß der dritten Ausgestaltung um eine gentechnisch veränderte Zelle, vorzugsweise um eine gentechnisch veränderte *Anabaena sp.*-Zelle handeln, die eine für ein Autofluoreszenzprotein kodierende Gensequenz umfasst, die sich unter der Kontrolle des *hetC*-, *nrrA*- oder *devB*-Promotors befindet (zum NtcA-Regulator in *Anabaena sp*. siehe beispielsweise J. Bacteriol., 190 (18) (2008), Seiten 6.126-6.133). Hier führt eine erhöhte intrazelluläre Konzentration an Oxoglutarat zu einer Expression des Autofluoreszenzproteins. Eine solche Zelle weist neben dem *hetC*-, *nrrA*- oder *devB-*Promotor und der unter der Kontrolle dieses Promotors befindlichen Gensequenz für ein Autofluoreszenzprotein vorzugsweise auch eine für den NtcA-Regulator kodierende Gensequenz auf. Die DNA-Sequenz des vom NtcA-Regulator regulierten *hetC*-Promotors ist in SEQ.-ID.-Nr. 27, die Sequenz des *nrrA*--Promotors in SEQ.-ID.-Nr. 28, die Sequenz des devB-Promotors in SEQ.-ID.-Nr. 29 und die Sequenz des NtcA-Regulators in SEQ.-ID.-Nr. 30 wiedergegeben.

Weiterhin kann es sich bei der gentechnisch veränderten Zelle gemäß der ersten besonderen Ausführungsform um eine gentechnisch veränderte Zelle, vorzugsweise um eine gentechnisch veränderte *Mycobacterium sp.*-Zelle handeln, die eine für ein Autofluoreszenzprotein kodierende Gensequenz umfasst, die sich unter der Kontrolle des *cbbLS-2-* oder *cbbLS-1*-Promotors befindet (zum CbbR-Regulator in *Mycobacterium sp.* siehe beispielsweise Mol. Micr. 47 (2009), Seite 297). Hier führt eine erhöhte intrazelluläre Konzentration an Ribulose-bis-Phosphat zu einer Expression des Autofluoreszenzproteins. Eine solche Zelle weist neben dem *cbbLS-2-* oder *cbbLS-1*-Promotor und der unter der Kontrolle dieses Promotors befindlichen Gensequenz für ein Autofluoreszenzprotein vorzugsweise auch eine für den CbbR-Regulator kodierende Gensequenz auf. Die DNA-Sequenz des CbbR-Regulators in SEQ.-ID.-Nr. 31 wiedergegeben.

Weiterhin kann es sich bei der gentechnisch veränderten Zelle gemäß der ersten besonderen Ausführungsform um eine gentechnisch veränderte Zelle, vorzugsweise um eine gentechnisch veränderte *Streptomyces cattleya*-Zelle handeln, die eine für ein Autofluoreszenzprotein kodierende Gensequenz umfasst, die sich unter der Kontrolle des *pcbAB*-Promotors befindet (zum ThnU-Regulator in *Strep-tomyces cattleya* siehe beispielsweise Mol. Micr., 69 (2008), Seite 633). Hier führt eine erhöhte intrazelluläre Konzentration an Thienamycin zu einer Expression des Autofluoreszenzproteins. Eine solche Zelle weist neben dem *pcbAB*-Promotor und der unter der Kontrolle dieses Promotors befindlichen Gensequenz für ein Autofluoreszenzprotein vorzugsweise auch eine für den ThnU-Regulator kodierende Gensequenz auf. Die DNA-Sequenz des vom ThnU-Regulator regulierten *pcbAB-*Promotors ist in SEQ.-ID.-Nr. 32 und die Sequenz des ThnU-Regulators selbst in SEQ.-ID.-Nr. 33 wiedergegeben.

Auch kann es sich bei der gentechnisch veränderte Zelle gemäß der ersten besonderen Ausführungsform um eine gentechnisch veränderte Zelle, vorzugsweise um eine gentechnisch veränderte *Streptomyces viridochromogenes*-Zelle handeln, die eine für ein Autofluoreszenzprotein kodierende Gensequenz umfasst, die sich unter der Kontrolle des *aviRa*-Promotors befindet (zum AviCl- bzw. AviC2-Regulator in *Streptomyces viridochromogenes* siehe beispielsweise J. Antibiotics, 62 (2009), Seite 461). Hier führt eine erhöhte intrazelluläre Konzentration an Avilamycin zu einer Expression des Autofluoreszenzproteins. Eine solche Zelle weist neben dem *aviRa*-Promotor und der unter der Kontrolle dieses Promotors befindlichen Gensequenz für ein Autofluoreszenzprotein vorzugsweise auch eine für den AviCl- und/oder AviC2-Regulator kodierende Gensequenz auf. Die DNA-Sequenz des vom AviC1- bzw. AviC2-Regulator regulierten *aviRa*-Promotors ist in SEQ.-ID.-Nr. 34 und die Sequenz des AviCl- bzw. AviC2-Regulators selbst in SEQ.-ID.-Nr. 35 wiedergegeben.

Weiterhin kann es sich bei der gentechnisch veränderte Zelle gemäß der ersten besonderen Ausführungsform um eine gentechnisch veränderte Zelle, vorzugsweise um eine gentechnisch veränderte *Nocardia uniformis*-Zelle handeln, die eine für ein Autofluoreszenzprotein kodierende Gensequenz umfasst, die sich unter der Kontrolle des *nocF*-Promotors befindet (zum NocR-Regulator in *Nocardia uniformis* siehe beispielsweise J. Bacteriol. 191 (2009), Seite 1.066). Hier führt eine erhöhte intrazelluläre Konzentration an Nocardicin zu einer Expression des Autofluoreszenzproteins. Eine solche Zelle weist neben dem *nocF*-Promotor und der unter der Kontrolle dieses Promotors befindlichen Gensequenz für ein Autofluoreszenzprotein vorzugsweise auch eine für den NocR-Regulator kodierende Gensequenz auf. Die DNA-Sequenz des vom NocR-Regulator regulierten *nocF-*Promotors ist in SEQ.-ID.-Nr. 36 und die Sequenz des NocR-Regulators selbst in SEQ.-ID.-Nr. 37 wiedergegeben.

Grundsätzlich gibt es nun verschiedene Möglichkeiten, eine erfindungsgemäße Zelle gemäß der ersten besonderen Ausführungsform beinhaltend einen vorstehend beschriebenen Promotor und eine unter der Kontrolle dieses Promotors befindliche, für ein Autofluoreszenzprotein kodierende Nukleinsäure herzustellen.

Eine erste Möglichkeit besteht beispielsweise darin, von einer Zelle auszugehen, deren Genom bereits einen der vorstehend beschriebenen Promotoren und vorzugsweise eine für den entsprechenden Regulator kodierende Gensequenz umfasst, und dann eine für ein Autofluoreszenzprotein kodierende Gensequenz derart in das Genom der Zelle einzubringen, dass sich diese Gensequenz unter der Kontrolle des Promotors befindet. Gegebenenfalls kann die Nukleinsäuresequenz des Promotors selbst vor oder nach der Integration der für das Autofluoreszenzprotein kodierenden Gensequenz in das Genom durch einen oder mehrere Nukleotid-Austausche, Nukleotid-Deletionen oder Nukleotid-Insertionen zum Zwecke der Steigerung der Promotoreffektivität verändert werden.

Eine zweite Möglichkeit besteht beispielsweise darin, ein oder mehrere Nukleinsäurekonstrukte, enthaltend die Promotorsequenz und die unter der Kontrolle des Promotors befindliche, für das Autofluoreszenzprotein kodierende Gensequenz in die Zelle einzuführen, wobei auch hier die Nukleinsäuresequenz des Promotors selbst durch einen oder mehrere Nukleotid-Austausche, Nukleotid-Deletionen oder Nukleotid-Insertionen zum Zwecke der Steigerung der Promotoreffektivität verändert sein kann. Die Insertion des Nukleinsäurekonstruktes kann chromosomal oder extrachromosomal, beispielsweise auf einem extrachromosomal replizierenden Vektor, erfolgen. Als Vektoren eignen sich solche, die in den jeweiligen Bakterienstämmen repliziert werden. Zahlreiche bekannte Plasmidvektoren wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren wie z. B. solche, die auf pCG4 (US 4,489,160), oder pNG2 (Serwold-Davis et al., FEMS Microbiology Letters 66, 119- 124 (1990)), oder pAG1 (US 5,158,891) beruhen, können in gleicher Weise verwendet werden. Diese Aufzählung ist für die vorliegende Erfindung jedoch nicht limitierend.

Anleitungen zur Herstellung von Genkonstrukten umfassend einen Promotor und eine unter der Kontrolle dieses Promotors befindliche Gensequenz und das Einschleusen eines solchen Konstruktes in das Chromosom einer Zelle oder das Einschleusen eines dieses Genkonstrukt umfassenden, extrachromosomal replizierenden Vektors in eine Zelle sind dem Fachmann beispielsweise aus Martin et al. (Bio/Technology 5, 137-146 (1987)), aus Guerrero et al. (Gene 138, 35-41 (1994)), aus Tsuchiya und Morinaga (Bio/Technology 6, 428-430 (1988)), aus Eikmanns et al. (Gene 102, 93-98 (1991)), aus EP-A-0 472 869, aus US 4,601,893, aus Schwarzer und Pühler (Bio/Technology 9, 84-87 (1991), aus Remscheid et al. (Applied and Environmental Microbiology 60, 126-132 (1994)), aus LaBarre et al. (Journal of Bacteriology 175, 1001-1007 (1993)), aus WO-A-96/15246, aus Malumbres et al. (Gene 134, 15-24 (1993), aus JP-A-10-229891, aus Jensen und Hammer (Biotechnology and Bioengineering 58, 191-195 (1998)) und aus bekannten Lehrbüchern der Genetik und Molekularbiologie hinlänglich bekannt.

Gemäß einer zweiten besonderen Ausführungsform der erfindungsgemäßen Zelle erfolgt die Kontrolle der Expression der für das Autofluoreszenzprotein kodierenden Gensequenz in Abhängigkeit von der intrazellulären Konzentration des bestimmten Metaboliten mittels eines sogenannten "*Riboswiches*", wobei mittels eines solchen "*Riboswitches*" die Expression sowohl auf der Ebene der Transkription als auch auf der Ebene der Translation reguliert werden kann. Unter einem "Riboswitch" werden regulatorische Elemente verstanden, die ausschließlich aus mRNA bestehen. Sie wirken zugleich als Sensor und als regulatorisches Element. Eine Übersicht über Riboswitches findet sich beispielsweise in Vitrechak et al., Trends in Genetics, 20 (1) (2004), Seiten 44-50. Weitere Einzelheiten zur Regulation der Genexpression mit Riboswitch können auch der Dissertation von Jonas Noeske (2007) mit dem Titel "Strukturelle Untersuchungen an Metabolit-bindenden Riboswitch-RNAs mittels NMR", vorgelegt beim Fachbereich Biochemie, Chemie und Pharmazie der Johann Wolfgang Goethe-Universität in Frankfurt am Main, entnommen werden.

Verwenden lassen sich Riboswitches in den erfindungsgemäßen Zellen gemäß dieser zweiten besonderen Ausführungsform dadurch, dass die für das Autofluoreszenzprotein kodierende Gensequenz funktionell mit einer DNA-Sequenz verbunden ist, die auf der Ebene der mRNA in der Lage ist, den Metaboliten zu binden, wobei in Abhängigkeit von der Bindung des Metaboliten an die mRNA entweder die weitere Transkription entlang der DNA oder aber die Translation an den Ribosomen beeinflusst wird. Auf diese Weise reguliert der Riboswitch auf der Ebene der Transkription oder der Ebene der Translation die Expression der für das Autofluoreszenzprotein kodierenden Gensequenz. In den erfindungsgemäßen Zellen mit Riboswitch-Elementen bindet der Metabolit ohne die Beteiligung jeglicher Proteinfaktoren direkt an eine strukturierte Region im 5'-UTR der mRNA und induziert eine Änderung der RNA-Sekundärstruktur. Diese Konformationsänderung im 5'-UTR führt zur Modulation der Expression des nachfolgenden, für das Autofluoreszenzprotein kodierenden Gens. Dabei kann die genregulierende Wirkung entweder durch die Beeinflussung der Transkription, der Translation oder gegebenenfalls auch der RNA-Prozessierung erreicht werden. Die Metabolit-bindende Region der Riboswitche (Aptamerdomäne) ist eine modulare, unabhängige RNA-Domäne. Der restliche Teil des Riboswitches (Expressionsplattform) liegt meist stromabwärts zur Aptamerdomäne. Die Expressionsplattform kann, abhängig davon, ob ein Metabolit an die Aptamerdomäne gebunden ist oder nicht, Basenpaarungen mit Bereichen der Aptamerdomäne eingehen. In den meist Fällen finden diese Basenpaarungen zwischen der Expressionsplattform und der Aptamerdomäne im Metabolit-ungebundenen Zustand statt und führen zur Aktivierung der Genexpression. Umgekehrt werden im Ligand-gebundenen Zustand diese Basenpaarungen verhindert, was meistens zur Hemmung der Genexpression führt. Ob sich der Regulationsmechanismus auf die Transkription oder die Translation auswirkt, ist abhängig von der Sekundärstruktur, die die Expressionsplattform im Metabolit-gebundenen bzw. - ungebundenen Zustand annimmt. Oft weist die Expressionsplattform Sequenzen auf, die einen Transkriptionsterminator und einen Transkriptionsantiterminator ausbilden können, wobei die beiden Sekundärstrukturen sich jedoch gegenseitig ausschließen. Ein anderes häufig auftretendes Motiv ist eine Sekundärstruktur, durch die je nach Metabolitbindungszustand die SD-Sequenz (Shine-Dalgarno-Sequenz) in eine einzelsträngige Form überführt oder maskiert wird. Wenn die SD-Sequenz durch Ausbildung einer Sekundärstruktur maskiert wird, kann die SD-Sequenz nicht vom Ribosom erkannt werden. Auf diese Weise kann durch Riboswitche der vorzeitige Transkriptionsabbruch oder die Translationsinitiation reguliert werden.

Als Beispiele geeigneter Riboswitch-Elemente, die auf der Ebene der Transkription oder der Ebene der Translation eine Kontrolle der Expression des Autofluoreszenzproteins ermöglichen, seien beispielsweise der Lysin-Riboswitch aus *Bacillus subtilis* (beschrieben durch Grundy *et al*., 2009), der Glycin-Riboswitch aus *Bacillus subtilis* (beschrieben durch Mandal et al., Science 306 (2004), Seiten 275-279), der Adenin-Riboswitch aus *Bacillus subtilis* (beschrieben durch Mandal und Breaker, Nat. Struct. Mol. Biol. 11 (2004), Seiten 29-35) oder der TPP-Tandemriboswitch aus *Bacillus anthracis* (beschrieben durch Welz und Breaker, RNA 13 (2007), Seiten 573-582). Neben diesen natürlich vorkommenden Riboswitch-Elementen können auch synthetische Riboswitch-Elemente verwendet werden, wie etwa der Theophyllin-Riboswitch (beschrieben durch Jenison et al., Science 263 (1994), Seiten 1425-1429 oder durch Desai und Gellivan, J. Am. Chem. Soc. 126 (2004), Seiten 1.3247-54), der Biotin-Riboswitch (beschrieben durch Wilson et al., Biochemistry 37 (1998), Seiten 14.410-14419) oder der Tet-Riboswitch (beschrieben durch Berens et al., Bioorg. Med. Chem. 9 (2001), Seiten 2.549- 2.556).

Einen Beitrag zur Lösung der eingangs genannten Aufgaben leistet weiterhin ein Verfahren zum Identifizieren eine Zelle mit erhöhter intrazellulärer Konzentration eines bestimmten Metaboliten in einer Zellsuspension wie in Anspruch 8 definiert.

Im Schritt i) des erfindungsgemäßen Verfahrens wird zunächst eine Zellsuspension umfassend ein Nährmedium sowie eine Vielzahl der eingangs beschriebenen, gentechnisch veränderten Zellen bereitgestellt.

Im Schritt ii) des erfindungsgemäßen Verfahrens wird bzw. werden sodann eine oder mehrere der Zellen in der Zellsuspension gentechnisch verändert, um eine Zellsuspension zu erhalten, in der sich die Zellen hinsichtlich der intrazellulären Konzentration eines bestimmten Metaboliten unterscheiden.

Das gentechnische Verändern der Zellsuspension kann durch zielgerichtete oder durch ungerichtete Mutagenese erfolgen, wobei die ungerichtete Mutagenese besonders bevorzugt ist.

Bei der zielgerichteten Mutagenese werden Mutationen gezielt in bestimmten Genen der Zelle erzeugt. Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen ("*missense mutations")* oder Nichtsinnmutationen ("*nonsense mutations")* gesprochen. Insertionen oder Deletionen von mindestens einem Basenpaar in einem Gen führen zu Rasterverschiebungsmutationen ("*frame shift mutations"),* in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie, wie z. B. dem Lehrbuch von Knippers ("Molekulare Genetik", 6. Auflage, Georg Thieme-Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer-Verlag, Stuttgart, 1986) entnommen werden.

Einzelheiten, insbesondere hilfreiche Literaturverweise zu diesen Methoden der gezielten Mutagenese können beispielsweise der DE-A-102 24 088 entnommen werden.

Erfindungsgemäß besonders bevorzugt ist jedoch, wenn die gentechnische Veränderung im Verfahrensschritt ii) durch ungerichtete Mutagenese erfolgt. Ein Beispiel einer solchen ungerichteten Mutagenese ist die Behandlung der Zellen mit Chemikalien wie z. B. N- Methyl-N-Nitro-N-Nitrosoguanidin oder die Bestrahlung der Zellen mit UV-Licht. Derartige Verfahren zur Mutationsauslösung sind allgemein bekannt und können unter anderem bei Miller ("A Short Course in Bacterial Genetics, A Laboratory Manual and Handbook for Escherichia coli and Related Bacteria" (Cold Spring Harbor Laboratory Press, 1992)) oder im Handbuch "Manual of Methods for General Bacteriology" der American Society for Bacteriology (Washington D. C., USA, 1981) nachgelesen werden.

Durch die gentechnische Veränderung der Zelle im Verfahrensschritt ii) kann es, je nach Art der erfolgten Mutation in der Zelle, dazu kommen, dass in einer bestimmten Zelle beispielsweise als Folge einer gesteigerten oder verminderten Enzymaktivität, einer gesteigerten oder verminderten Expression eines bestimmten Enzyms, einer gesteigerten oder verminderten Aktivität eines bestimmten Transporterproteins, einer gesteigerten oder verminderten Expression eines bestimmten Transporterproteins, einer Mutation in einem Regulatorprotein, einer Mutation in einem Strukturprotein oder einer Mutation in einem RNA-Kontrollelement zu einer Erhöhung der intrazellulären Konzentration desjenigen Metaboliten kommt, der durch Wechselwirkung mit einem entsprechenden Regulatorprotein über den Promotor oder durch Wechselwirkung mit einem Riboswitch-Element einen Einfluss auf die Expression des Autofluoreszenzproteins nimmt. Eine Zelle, in der als Folge der Mutation die Konzentration eines bestimmten Metaboliten erhöht ist, zeichnet sich daher dadurch aus, dass in dieser Zelle das Autofluoreszenzprotein gebildet wird. Das Gen für das Autofluoreszenzprotein wirkt somit als Reportergen für eine erhöhte intrazelluläre Metaboliten-Konzentration.

Im Verfahrensschritt iii) des erfindungsgemäßen Verfahrens werden daher einzelne Zellen in der Zellsuspension mit erhöhter intrazellulärer Konzentration dieses bestimmten Metaboliten durch Detektion der intrazellulären Fluoreszenzaktivität identifiziert. Dazu wird die Zellsuspension elektromagnetischer Strahlung in derjenigen Frequenz ausgesetzt, welche die Autofluoreszenzproteine zur Emission von Licht anregt.

Gemäß einer besonderen Ausgestaltung des erfindungsgemäßen Verfahrens erfolgt im Anschluss, vorzugsweise im unmittelbaren Anschluss an das Identifizieren der Zellen im Verfahrensschritt iii) ein weiterer Verfahrensschritt iv), in dem die identifizierten Zellen aus der Zellsuspension abgetrennt werden, wobei dieses Abtrennen vorzugsweise mittels Durchflusszytometrie (FACS = *fluorescens activated cell sorting),* ganz besonders bevorzugt mittels Hochdurchflusszytometrie (HAT-FACS = *high througput fluorescens activated cell sorting*) erfolgt. Einzelheiten zur Analyse von Zellsuspensionen mittels Durchflusszytometrie können beispielsweise Sack U, Tarnok A, Rothe G (Hrsg): Zelluläre Diagnostik. Grundlagen, Methoden und klinische Anwendungen der Durchflusszytometrie, Basel, Karger, 2007, Seiten 27 - 70 entnommen werden.

Mittels des erfindungsgemäßen Verfahrens ist es daher möglich, in einer Zellsuspension, in der gezielte oder ungezielte Mutationen in den Zellen erzeugt worden sind, ohne eine Beeinflussung der Vitalität der Zellen gezielt diejenigen Zellen zu isolieren, in denen die Mutation zu einer gesteigerten intrazellularen Konzentration eines bestimmten Metaboliten geführt hat.

Einen Beitrag zur Lösung der eingangs genannten Aufgaben leistet auch ein Verfahren zur Herstellung einer gegenüber ihrem Wildtyp gentechnisch veränderten

Zelle mit optimierter Produktion eines bestimmten wie in Anspruch 12 definiert.

Gemäß den Verfahrensschritten I) bis IV) werden zunächst Zellen mit erhöhter intrazellulärer Konzentration eines bestimmten Metaboliten durch Mutagenese erzeugt und aus einer Zellsuspension abgetrennt, wobei hier auf die vorstehend beschriebenen Verfahrensschritte i) bis iv) verwiesen werden kann.

Im Verfahrensschritt V) werden sodann in den identifizierten und abgetrennten Zellen mittels dem Fachmann bekannter gentechnischer Verfahren diejenigen gentechnisch veränderten Gene Gₗ bis Gₙ oder diejenigen Mutationen M₁ bis Mₘ identifiziert, welche für die erhöhte intrazelluläre Konzentration des bestimmten Metaboliten verantwortlich sind, wobei der Zahlenwert von n bzw. m von der Anzahl der beobachtenen veränderten Gene bzw. der beobachteten Mutationen in der identifizierten und abgetrennten Zelle abhängt. Vorzugsweise geht man dabei so vor, dass man zunächst die Sequenz derjenigen Gene oder Promotorsequenzen in den Zellen analysiert, von denen bekannt ist, dass sie die Bildung eines bestimmten Metaboliten stimulieren. Im Falle von L-Lysin als Metabolit sind dies beispielsweise die Gene *lysC*, *hom*, *zwf*, *mqo, leuC*, *gnd* oder *pyk.* Wird in keiner dieser Gene eine Mutation erkannt, so wird das gesamte Genom der identifizierten und abgetrennten Zelle analysiert, um gegebenenfalls weitere veränderten Gene Gᵢ bzw. weitere Mutationen Mᵢ zu identifizieren. Auf diese Weise können vorteilhafte veränderte Gensequenzen Gᵢ bzw. vorteilhafte Mutationen Mᵢ, welche in einer Zelle zu einer Erhöhung der intrazellulären Konzentration eines bestimmten Metaboliten führen, identifiziert werden.

In einem weiteren Verfahrensschritt VI) kann dann eine gegenüber ihrem Wildtyp gentechnisch veränderten Zelle mit optimierter Produktion des bestimmten Metaboliten hergestellt werden, deren Genom mindestens eines der Gene Gₗ bis Gₙ und/oder mindestens eine der Mutationen Mₗ bis Mₘ umfasst. Dazu werden eine oder mehrere der im Verfahrensschritt V) beobachteten, vorteilhaften veränderten Gene G und/oder veränderten Mutationen M gezielt in eine Zelle eingeführt. Dies gezielte Einführen bestimmter Mutationen kann beispielsweise mittels Genaustausch ("*gene replacement*") erfolgen. Bei diesem Verfahren wird eine Mutation wie z. B. eine Deletion, Insertion oder Basenaustausch in dem interessierenden Gen *in vitro* hergestellt. Das hergestellte Allel wird wiederum in einen für den Zielwirt nicht replikativen Vektor kloniert und dieser anschließend durch Transformation oder Konjugation in den Zielwirt überführt. Nach homologer Rekombination mittels eines ersten, Integration bewirkenden "*cross-over*"-Ereignisses und eines geeigneten zweiten, eine Exzision bewirkenden "*cross-over*"-Ereignisses im Zielgen bzw. in der Zielsequenz erreicht man den Einbau der Mutation bzw. des Allels.

Einen Beitrag zur Lösung der eingangs genannten Aufgaben leistet auch eine Zelle mit optimierter Produktion eines bestimmten Metaboliten, welche durch das vorstehend beschriebene Verfahren erhalten wurde.

Einen Beitrag zur Lösung der eingangs genannten Aufgaben leistet auch ein Verfahren zur Herstellung von Metaboliten, beinhaltend die Verfahrensschritte:
(a) Herstellung einer gegenüber ihrem Wildtyp gentechnisch veränderten Zelle mit optimierter Produktion eines bestimmten Metaboliten durch das vorstehend beschriebene Verfahren;
(b) Kultivieren der Zelle in einem Kulturmedium beinhaltend Nährstoffe unter Bedingungen, unter denen die Zelle aus den Nährstoffen den bestimmten Metaboliten produziert.

Die im Verfahrensschritt (a) hergestellten erfindungsgemäßen, gentechnisch veränderten Zellen mit optimierter Produktion eines bestimmten Metaboliten können im Verfahrensschritt (b) kontinuierlich oder diskontinuierlich im *batch*-Verfahren (Satzkultivierung) oder im *fed-batch*-Verfahren (Zulaufverfahren) oder *repeated-fed*-*batch*-Verfahren (repetitives Zulaufverfahren) zum Zwecke der Produktion des Metaboliten in dem Nährmedium kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Biore-aktoren und periphere Einrichtungen", Vieweg Verlag, Braunschweig/Wiesbaden, 1994) beschrieben.

Das zu verwendende Nährmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Als Kohlenstoffquelle kann das Nährmedium Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Methanol, Kohlenwasserstoffe wie Methan, Aminosäuren wie L-Glutamat oder L-Valin oder organische Säuren wie z. B. Essigsäure enthalten. Diese Stoffe können einzeln oder als Mischung verwendet werden.

Als Stickstoffquelle kann das Nährmedium organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat enthalten. Die Stickstoffquellen können einzeln oder als Mischung verwendet werden.

Als Phosphorquelle kann das Nährmedium Phosphorsäure, Kaliumdihydrogen-phosphat oder Dikaliumhydrogenphosphat oder die entsprechenden Natriumhaltigen Salze enthalten. Das Nährmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Nährmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen, wie z. B. Luft, in die Kultur eingetragen. Die Temperatur der Kultur liegt normalerweise bei 20°C bis 45°C und vorzugsweise bei 25°C bis 40°C.

Einen Beitrag zur Lösung der eingangs genannten Aufgaben leistet auch ein Verfahren zur Herstellung einer Mischung, beinhaltend die Verfahrensschritte:
(A) Herstellen von Metaboliten durch das vorstehend beschriebene Verfahren;
(B) Vermischen des Metaboliten mit einer von dem Metaboliten verschiedenen Mischungskomponente.

Sofern es sich bei dem Metaboliten um eine Aminosäure, insbesondere im L-Lysin handelt, ist die Mischung vorzugsweise ein Nahrungsmittel, ganz besonders bevorzugt ein Tierfutter, oder eine pharmazeutische Zusammensetzung.

Die Erfindung wird nun anhand von Figuren und nicht limitierenden Beispielen näher erläutert.

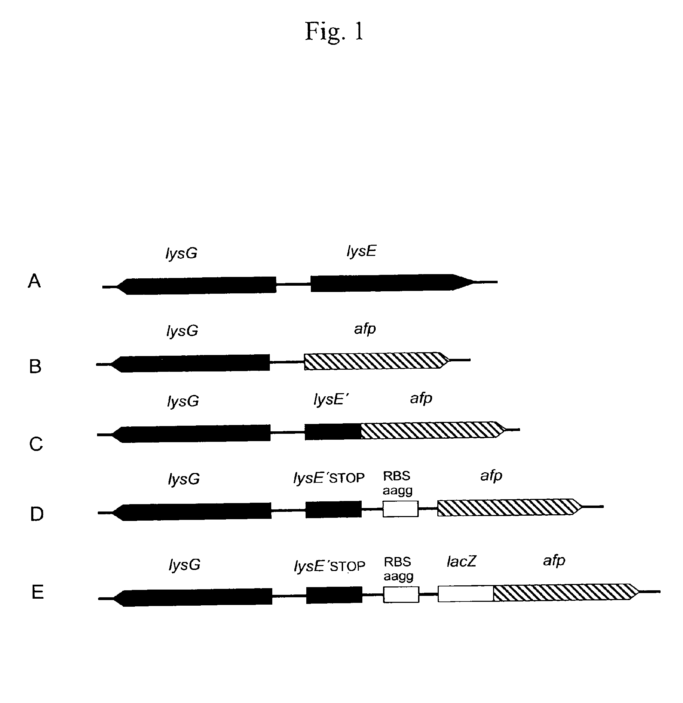

Es zeigt die Figur 1 mögliche Konstrukte, in denen die Gensequenz eines Autofluoreszenzproteins (afp) gemäß der ersten Ausführungsform der erfindungsgemäßen Zelle unter der Kontrolle eines Promotors (*lysE*-Promotor) steht.

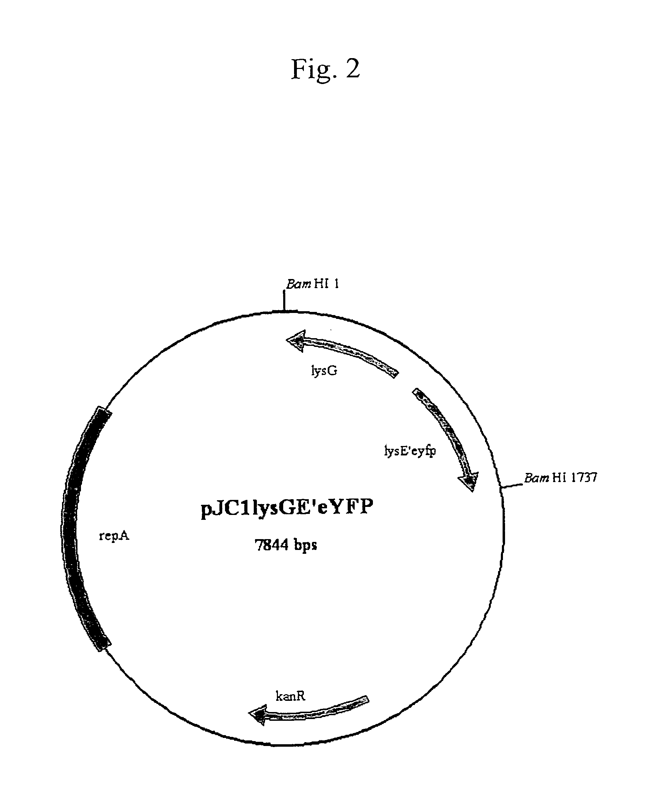

Es zeigt die Figur 2 den im Beispiel 1 hergestellten Vektor pJC1lysGE'eYFP (ly-sE'eYFP, kodierende Sequenz des LysE'eYFP-Fusionsproteins; lysG, kodierende Sequenz des Regulatorproteins LysG; kanR, kodierende Sequenz der Kanamycinvermittelten Restistenz; repA,: Replikationsursprung; BamHI: Erkennungssequenz und Schnittstelle des Restriktionsenzyms BamHI).

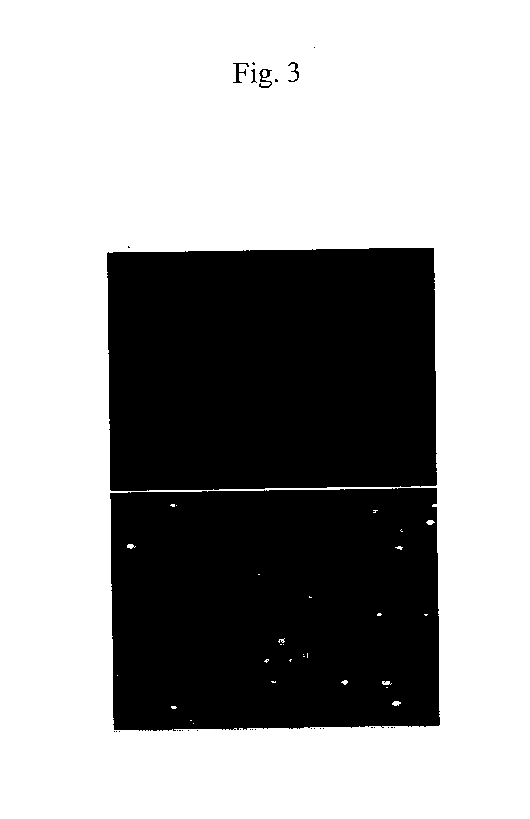

Es zeigt die Figur 3 eine konfokale Mikroskopie der im Beispiel 1 erhaltenen Stämme ATCC 13032 pJC1lysGE'eYFP (oben) und DM1800 p JC1lysGE'eYFP (unten). Der weiße Balken in der unteren Abbildung entspricht einer Länge von 10µm. Jeweils 3 µl Zellsuspensionen wurden auf einen Objektträger gegeben und durch eine dünne Schicht 1 % Agarose immobilisiert. Angeregt wurde die immobilisierte Suspension mit Licht der Wellenlänge 514 nm und einer Belichtungszeit von 700 ms. Die Fluoreszenzemmissionsmessung von eYFP erfolgte mit dem Zeiss Axiolmager M1 unter Verwendung eines Breitbandfilters im Bereich von 505 nm bis 550 nm.

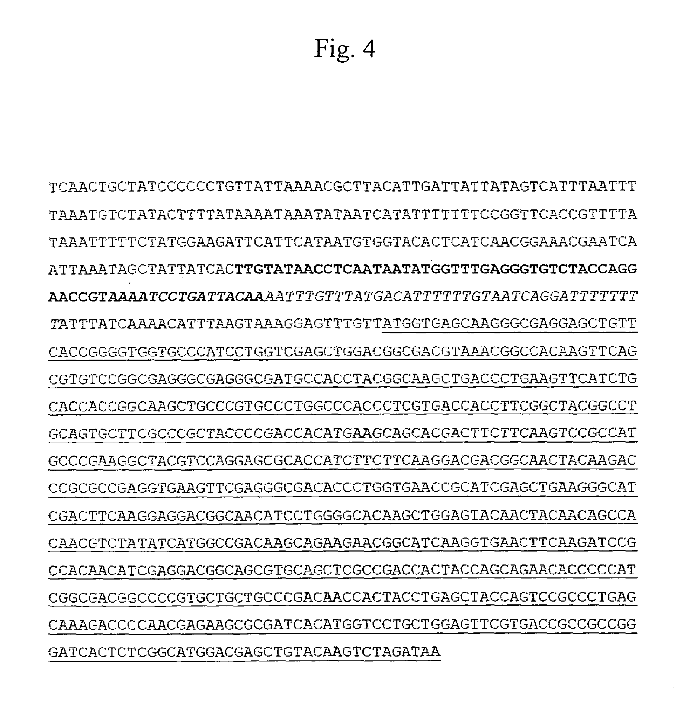

Es zeigt die Figur 4 die Sequenz des im Beispiel 2 herstellten, auf einem Riboswitch-Element basierende Gensequenz umfassend ein Riboswitch-Element und ein mit diesem Riboswitch-Element funktionell verknüpfte, für ein Autofluoreszenzprotein kodierende Gensequenz (fett: Aptamner; kursiv: Terminatorsequenz; unterstrichen: EYFP).

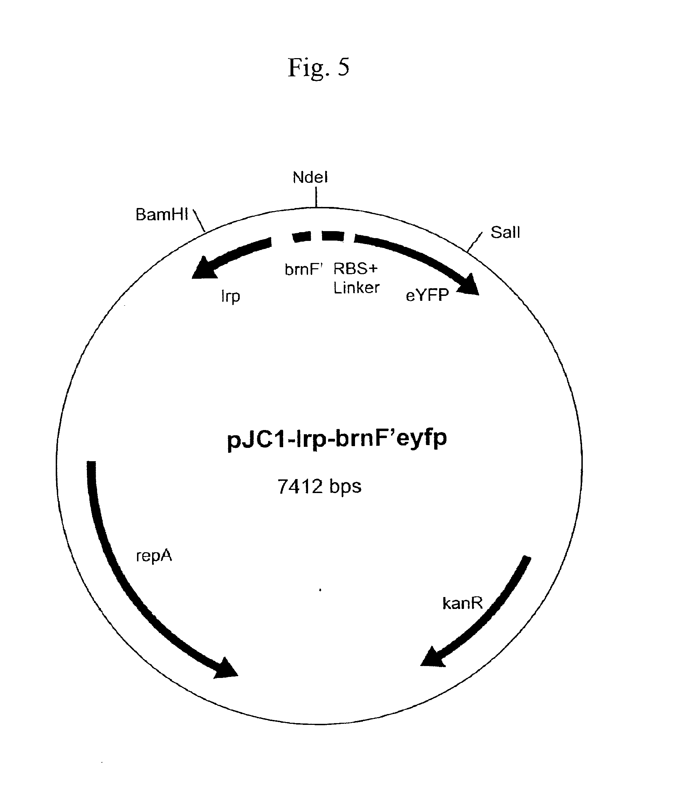

Es zeigt die Figur 5 den Vektor pJC1lrp-brnF'eYFP.

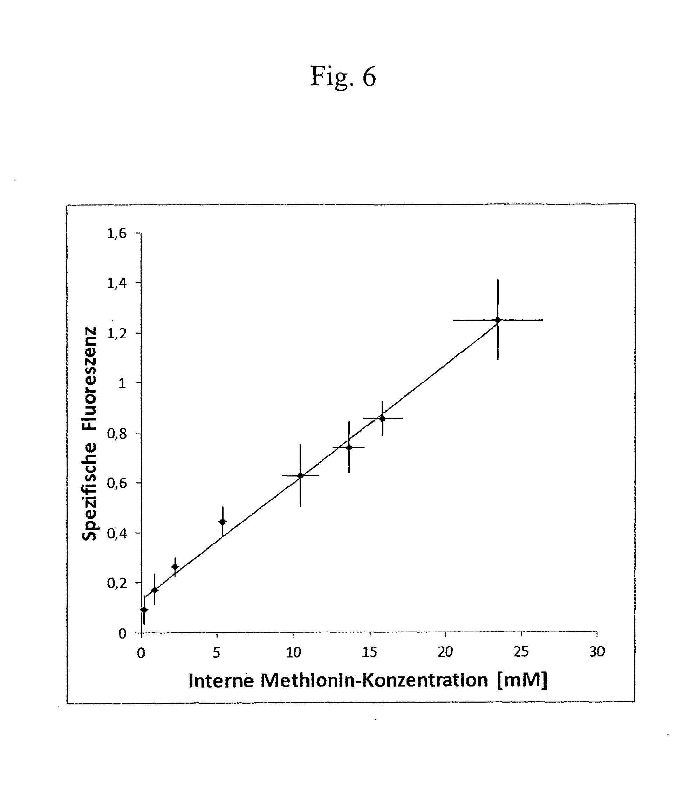

Es zeigt die Figur 6 die Korrelation der internen L-Methionkonzentration mit dem Fluoreszenz-Output Signal der im Beispiel 3 erhaltenen ATCC13032pJC1lrp-brnF'-eYFP-Kulturen.

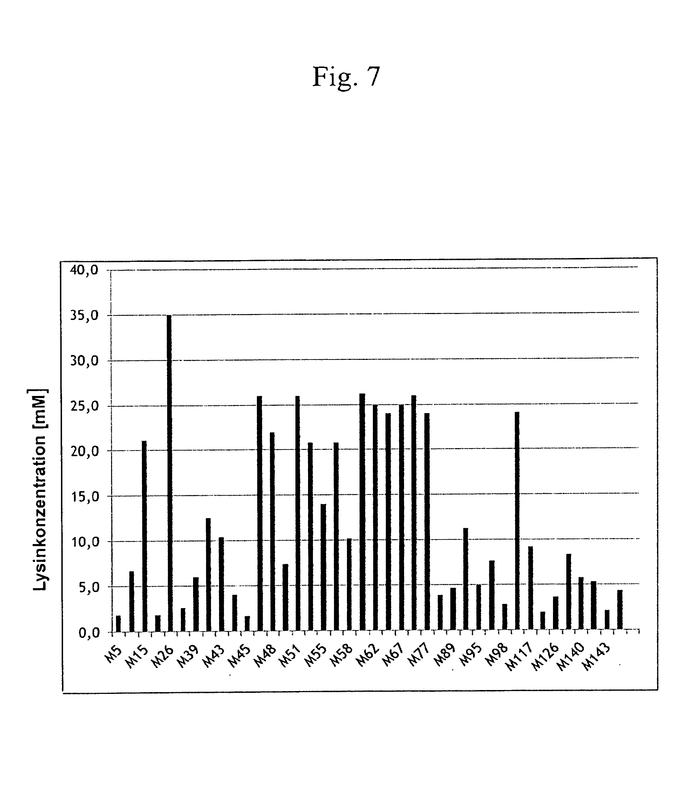

Es zeigt die Figur 7 die Bildung von Lysin durch die Mutanten des Ausgangsstammes ATCC13032pSenLysTK-C im Beispiel 4c).

Die Figur 1 zeigt mögliche Konstrukte, in denen die Gensequenz eines Autofluoreszenzproteins (afp) gemäß der ersten Ausführungsform der erfindungsgemäßen Zelle unter der Kontrolle eines Promotors (*lysE*-Promotor) steht. Variante A gibt eine Ausgangssituation an, in der der Metabolit-abhängige Regulator direkt benachbart zu seinem Zielgen *(lysE)* liegt, das er von der Metabolitkonzentration abhängig reguliert. Gemäß Variante B ist im einfachsten Falle das Zielgen durch ein fluoreszierendes Protein (afp) ersetzt. Gemäß der Variante C ist eine translationale Fusion der ersten Aminosäuren des Zielgens mit dem fluoreszierenden Protein erfolgt. Bei der Variante D ist eine transkriptionelle Fusion erfolgt, in der Weise, dass ein langes Transkript ausgehend von der Promoterregion gebildet wird, das die ersten Aminosäuren des Zielgens umfasst, endend durch ein Stoppcodon, gefolgt durch eine Ribosomenbindestelle (RBS) und dem Offenen Leseraster für das fluoreszierende Protein. Bei der Variante E ist eine transkriptionelle Fusion erfolgt, in der Weise, dass ein langes Transkript ausgehend von der Promoterregion gebildet wird, das die ersten Aminosäuren des Zielgens umfasst, endend durch ein Stoppcodon, gefolgt durch eine Ribosomenbindestelle und dem Beginn eines bekannten und gut exprimierten Proteins wie z.B. der beta-Galactosidase aus *E. coli*, LacZ, das wiederum fusioniert mit dem fluoreszierenden Protein ist.

### BEISPIELE

### Beispiel 1

Herstellung einer erfindungsgemäßen Zelle gemäß der ersten Ausführungsform am Beispiel einer Zelle, bei der eine für ein Autofluoreszenzprotein kodierende Gensequenz unter der Kontrolle des *lysE*-Promotors steht und bei der die Expression des Autofluoreszenzproteins von der intrazellularen L-Lysin-Konzentration abhängig ist.

### a) Konstruktion des Vektors pJC1lysGE'eYFP

Die Konstruktion der Fusion von *lysE* 'mit dem Reportergen *eyfp* (SEQ.-ID.-Nr. 49; Proteinsequenz des eYFP: SEQ.-ID.-Nr. 72) wurde durch eine *overlap-extension-PCR* erreicht. Als Template diente pUC18-2,3-kb-lysGE-BamHI, das die kodierende Sequenz von *lysE* zusammen mit dem Gen des divergent transkribierten Regulators LysG trägt (Bellmann et al., 2001; Microbiology 1471765-74), sowie pEKEx2-yfp-tetR (Frunzke et al., 2008; J Bacteriol. 190:5111-9), das die Amplifikation von *eyfp* ermöglicht. Zur Erstellung des *lysGE'eyfp*-Fragments wurden zunächst die kodierenden Sequenzen *lysGE'* und *lysGE'ns* (1010 bp) mit den Oligonukleotidkombinationen plysGE_for (SEQ.-ID.-Nr. 38) und plysGE_rev (SEQ.-ID.-Nr. 39) amplifiziert. Zur Amplifikation der kodierenden Sequenz von *eyfp* wurden die zwei Oligonukleotidkombinationen peYFP_rev (SEQ.-ID.-Nr. 40) und peYFP_fw2 (SEQ.-ID.-Nr. 41) verwendet.
plysGE_for 5'-CGCGGATCCCTAAGCCGCAATCCCTGATTG-3'
plysGE_rev 5'-TCCGATGGACAGTAAAAGACTGGCCCCCAAAGCAG-3'

Nach Aufreinigung der amplifizierten Fragmente aus einem 1%igen Agarosegel wurden diese in einer zweiten PCR-Reaktion mit den äußeren Primern plysGE-for und peYFP_rev als Matrizen eingesetzt. Durch Hybridisierung der Template-Fragmente in einem komplementären Bereich von 17 bp, der von den inneren Oligonukleotidprimem plysGE_rev und peYFP_fw2 geschaffen wurde, konnte das *overlap-extension-Fragment* erstellt werden. Das so entstandenen Produkt *lysGE'eyfp* wurde mit dem Restriktionsenzym *Bam*HI verdaut und nach Reinigung des Reaktionsansatzes in Ligationsreaktionen mit dem ebenfalls BamHI-geöffneten und dephosphorylierten Vektor pJC1 eingesetzt. Der Ligationsansatz wurde direkt zur Transformation von *E. coli* DH5αMCR benutzt, und die Selektion von Transformanten erfolgte auf LB-Platten mit 50 µg/ml Kanamycin. 20 Kolonien, die auf diesen Platten wuchsen und demnach Kanamycin-resistent waren, wurden für eine Kolonie-PCR eingesetzt. Die Kolonie-PCR erfolgte jeweils mit den oben beschriebenen Oligonukleotid-Kombinationen, um zu überprüfen, ob das Fragment *lysGE'eyfp* im Vektor pJC1 inseriert war. Die Analyse der Kolonie-PCR in einem Agarose-Gel wies das erwartete PCR-Produkt mit einer Größe von 1010 bp in den analysierten Proben auf, woraufhin eine Kolonie für eine Plasmid Präparationen im größerem Maßstab kultiviert wurde. Über die Testspaltung mit den Restriktionsenzymen *Bgl*II, *Xho*I und *Pvu*I konnte die Anwesenheit des inserierten Fragmentes pJC1lysGE'eYFP nachgewiesen werden.

Die Sequenzierung des Inserts zeigte eine 100 %ige Übereinstimmung mit der erwarteten Sequenz.

### b) Transformation von Corynebacterium glutamicum mit pJC1lysGE'eYFP

Es wurden kompetente Zellen der *C*. *glutamicum* Stämme ATCC 13032 und DM1800 wie bei Tauch et al., 2002 (Curr Microbiol. 45(5) (2002), Seiten 362-7) beschrieben hergestellt. Der Stamm ATCC 13032 ist ein Wildtyp der Lysin ausscheidet, wogegen der Stamm DM1800 durch gengerichtete Mutationen zu einem Lysinausscheider gemacht wurde (Georgi et al. Metab Eng. 7 (2005), Seiten 291-301) Diese Zellen wurden mit pJC1lysGE'eYFP wie bei Tauch et al. (Curr Microbiol. 45(5) (2002), Seiten 362-7) beschrieben durch Elektroporation transformiert. Die Selektion der Transformanten erfolgte auf BHIS-Platten mit 25 µg/ml Kanamycin. Kolonien, die auf diesen Platten wuchsen und demnach Kanamycin-resistent waren, wurden durch Plasmidpräparationen und Testspaltungen mit den Enzymen *Bgl*II, *Xho*I und *Pvu*I auf Anwesenheit der Vektoren überprüft. Je ein korrekter Klon wurde mit ATCC 13032 pJC1lysGE'eYFP und DM1800 pJC1lysGE'eYFP bezeichnet.

### c) Nachweis der Lysin-spezifischen Fluoreszenz

Die Prüfung der in vivo Fluoreszenzemmission erfolgte über konfokale Mikroskopie mit dem Zeiss Axiolmager MI. Zu diesem Zweck wurden 3 µl Zellsuspension der Stämme ATCC 13032 pJC1lysGE'eYFP und DM1800 pJC1lysGE'eYFP auf einen Objektträger gegeben, auf den zur Immobilisierung zuvor eine dünne Schicht 1 %iger Agarose aufgebracht worden war. Angeregt wurde die immobilisierte Suspension mit Licht der Wellenlänge 514 nm und einer Belichtungszeit von 700 ms. Die Fluoreszenzemmissionsmessung von eYFP erfolgte unter Verwendung eines Breitbandfilters im Bereich von 505 nm bis 550 nm. Fluoreszierende Zellen wurden digital mit Hilfe der AxioVision 4.6 software dokumentiert. In der Abbildung ist zu erkennen, dass Fluoreszenzemmission nur beim Lysin bildenden Stamm DM1800 pJC1lysGE'eYFP auftritt, wogegen der nicht Lysin bildende Stamm ATCC13032 pJC1lysGE'eYFP nicht fluoresziert.

### Beispiel 2

Herstellung einer erfindungsgemäßen Zelle gemäß der zweiten Ausführungsform am Beispiel einer Zelle, bei der die Expression eines Autofluoreszenzproteins unter vom Adeninriboswitch (ARS) reguliert wird und bei der die Expression des Autofluoreszenzproteins von der intrazellulären Adenin-Konzentration abhängig ist.

Der Adeninriboswitch (ARS) aus *Bacillus subtilis* (siehe Mandai und Breaker, Nat Struct Mol Biol, 11 (2004), Seiten 29-35) wurde zunächst ausgehend von genomischer DNA aus *Bacillus subtilis* mit den primem ARS_for (SEQ.-ID.-Nr. 42) und ARS_rev (SEQ.-ID.-Nr. 43) amplifiziert. In einer zweiten PCR wurde ausgehend des mittels Qiagen MinElute Gel Extraction Kit aufgereinigten ARS-Amplifikats unter Nutzung der primer ARS_for_BamHI und ARS_rev_NdeI ein ARS-Amplifikat mit 5' terminaler *BamH*I und 3' terminaler *Nde*I Schnittstelle amplifiziert und mit diesen Restriktionsenzymen geschnitten.

Das Reportergen *eyfp* wurde auf Basis von pEKEx2-EYFP mit den primern EYFP_for_NdeI (SEQ.-ID.-Nr. 44) und EYFP_rev_EcoRI (SEQ.-ID.-Nr. 45) amplifiziert, mit den Enzymen *Nde*I und *EcoR*I restringiert und ebenfalls mittels Qiagen MinElute Gel Extraction Kit aufgereinigt.
ARS_for: 5'-TCAACTGCTATCCCCCCTGTTA-3'
ARS_rev: 5'-AAACTCCTTTACTTAAATGTTTTGATAAATAAA-3'
EYFP_for_NdeI: 5'-TACATATGGTGAGCAAGGGCGA-3'
EYFP_rev_EcoRI: 5'-TAGAATTCTTATCTAGACTTGTACAGCTCG-3'

Die beiden restringierten PCR-Produkte wurden zusammen in den zuvor mit *BamH*I und *EcoR*I restringierten Vektor pEKEx2 ligiert und somit unter Kontrolle des durch IPTG-induzierbaren Promotors *ptac* gestellt. *E. coli* XL1 blue wurde anschließend mit dem Ligationsansatz transformiert.

Kanamyzin-resistente Transformanden wurden mittels *colony*-PCR auf Vorhandensein des Konstruktes pEKEx2-ARS-EYFP geprüft (primer pEKEx2_for (SEQ.-ID.-Nr. 46) und EYFP_rev (SEQ.-ID.-Nr. 47)) und das Plasmid zur weiteren Analyse aufgereinigt.

Zur Verifizierung des präparierten Konstruktes pEKEx2-ARS-EYFP wurde dieses mit dem Restriktionsenzym NadeI geschnitten und anhand des Bandenmusters geprüft.

Eine in Figur 4 gezeigte Sequenzierung (SEQ.-ID.-Nr. 48) des Adenin-Sensors bestätigte die intakte Fusion des Adenin-abhängigen Riboswitch (ydhL) mit dem Autofluoreszenzprotein EYFP.
pEKEx2_for: 5 '-CGGCGTTTCACTTCTGAGTTCGGC-3'
EYFP_rev: 5'-TAGAATTCTTATCTAGACTTGTACAGCTCG-3'

### Beispiel 3

Herstellung einer erfindungsgemäßen Zelle gemäß der ersten Ausführungsform am Beispiel einer Zelle, bei der eine für ein Autofluoreszenzprotein kodierende Gensequenz unter der Kontrolle des *brnFE*-Promotors steht und bei der die Expression des Autofluoreszenzproteins von der intrazellularen L-Methionin-Konzentration abhängt.

### a) Konstruktion des Vektors pJC1lrp-brnF'eYF

Bei der Konstruktion der Fusion von *brnF* mit dem Reportergen *eyfp* wurde wie folgt vorgegangen. In zwei getrennten Reaktionen wurde zunächst die kodierende lrp und die ersten 30 Nukleotide der *brnF*-Sequenz (*brnF*') samt intergener Region (560 bp) mit dem Oligonukleotidpaar lrp-fw-A-BamHI (SEQ.-ID.-Nr. 50) /lrp-brnF-rv-I-NdeI (SEQ.-ID.-Nr. 51) sowie *eyfp* (751 bp) mit dem Oligonukleotidpaar *eyfp*-fw-H-*Nde*I (SEQ.-ID.-Nr. 52) / *eyfp*-rv-D-*Sal*I (SEQ.-ID.-Nr. 53) amplifiziert. Als Template diente genomische DNA von *C*. *glutamicum*, sowie der Vektor pEKEx2-yfp-tetR (Frunzke et al., 2008, J. Bacteriol. 190: 5111-5119), der die Amplifikation von *eyfp* ermöglicht. Die Oligonukleotide *fw*-A-*BamH*I und lrp-*brnF*-rv-I-NdeI wurden mit 5'-terminalen *BamH*I- und *Nde*I- Restriktionsschnittstellen, die Oligonukleotide eyfp-fw-H-*Nde*I und *eyfp*-rv-D-*Sal*I mit 5'-terminalen *Nde*I- und *Sal*I- Restriktionsschnittstellen ergänzt. Nach Restriktion der lrp-brnF'-Amplifikate mit *BamH*I und *Nde*I bzw. des *eyfp*-Amplifikats mit *Nde*I und *Sal*I, wurden die lrp-*brnF*'-Amplifikate mit dem *eyfp*-Amplifikat über die freistehenden Enden der *Nde*I Schnittstelle in einem Ligationsansatz fusioniert und gleichzeitig in den ebenfalls *BamH*I- *Sal*I-geöffneten Vektor pJC1 kloniert werden (Fig. 5). Der Ligationsansatz wurde direkt zur Transformation von *E. coli* DH5α benutzt. Die Selektion von Transformanten erfolgte auf LB-Platten mit 50 µg/ml Kanamycin. Kolonien, die auf diesen Platten wuchsen und demnach Kanamycin-resistent waren, wurden für eine Kolonie-PCR eingesetzt. Um zu überprüfen, ob das Fragment lrp-brnF'eyfp im Vektor pJC1 inseriert war, erfolgte die Kolonie-PCR mit Oligonukleotiden, die den Bereich der "*multiple cloning site*" im Vektor pJC1 flankieren. Die Analyse der Kolonie-PCR in einem Agarose-Gel wies das erwartete PCR-Produkt mit einer Größe von 1530 bp in den analysierten Proben auf, woraufhin eine Kolonie für eine Plasmid Präparationen im größerem Maßstab kultiviert wurde. Über die Testspaltung mit den Restriktionsenzymen *BamH*I, *Nde*I und *Sal*I wurde die Anwesenheit des inserierten Fragmentes nachgewiesen. Die Sequenzierung des Inserts zeigte eine 100 %ige Übereinstimmung mit der erwarteten Sequenz. Die Transformation von kompetenten *C*. *glutamicum-*Zellen mit dem Vektor pJC1lrp-brnF'eYFP erfolgte nach der Methode von Tauch und Kirchner (Curr. Microbiol. (2002) 45:362- 367), und es wurde der Stamm *C. glutamicum* ATCC13032 pJC1lrp-brnF'eYFP erhalten.
lrp-fw-A-BamHI 5'-GCGCGGATCCTCACACCTGGGGGCGAGCTG-3' eyfp-fw-H-NdeI 5'-GCGCCATATGGTGAGCAAGGGCGAGGAG-3' Seq_pJC1_for1 (SEQ.-ID.-Nr. 54) 5'-CGATCCTGACGCAGATTTTT-3'
Seq_pJC1_rev1 (SEQ.-ID.-Nr. 55) 5'-CTCACCGGCTCCAGATTTAT-3'

### b) Korrelation der intrazellulären Methionin-Konzentration mit dem Fluoreszenz-Output

Zu näheren Charakterisierung wurden die Sensitivität und der dynamische Bereich des Sensors für L-Methionin bestimmt. Hierzu wurden in ATCC13032 pJC1lrp-brnF'eYFP verschiedene interne Konzentration von Methionin mit Peptiden eingestellt. Dieses Verfahren ist zum Beispiel bei Trötschel et al., beschrieben (J. Bacteriol. 2005, 187: 3786-3794). Es wurden folgende Dipeptide eingesetzt: L-Alanyl-L-Methionin (Ala-Met), L-Methionyl-L-Methionin (Met-Met), sowie L-Alanyl-L-Alanin (Ala-Ala). Um unterschiedliche L-Methionin-konzentrationen zu erreichen, wurden folgende Mischungsverhältnisse benutzt: 0,3 mM Ala-Met plus 2,7 mM Ala-Ala, 0,6 mM Ala-Met plus 2,4 mM Ala-Ala, 0,9 mM Ala-Met plus 2,1 mM Ala-Ala, 1,5 mM Ala-Met plus 1,5 mM Ala-Ala, 2,1 mM Ala-Met plus 0,9 mM Ala-Ala, 2,7 mM Ala-Met plus 0,3 mM Ala-Ala, 3 mM Ala-Met, 3 mM Met-Met, die zu dem Medium CGXII (Keilhauer et al., 1993, J Bacteriol. 175:5595-603) gegeben wurden. Die Kultivierung erfolgte mit 0,6 ml Medium im Mikrotitermaßstab (Flowerplate® MTP-48-B) im BioLector-System (m2p-labs GmbH, Forckenbeckstrasse 6, 52074 Aachen, Germany). Sieben Minuten nach Zugabe der Peptide wurden Zellen aus 200 µl der Zellsuspension durch Silikonölzentrifugation vom Medium abgetrennt und inaktiviert wie bei Klingenberg und Pfaff beschrieben (Methods in Enzymology 1967; 10: 680-684). Die cytoplasmatische Fraktion der Proben wurde aufgearbeitet wie bei Ebbinghausen et al. beschrieben (Arch. Microbiol. (1989), 151:238-244) und die Aminosäurekonzentration mittels reversed phase HPLC wie bei Lindroth und Mopper angegeben (Anal. Chem. (1979) 51, 1167-11174) quantifiziert. Die Fluoreszenz der Kulturen von ATCC13032 pJC1lrp-brnF'eYFP mit den unterschiedlichen Peptidkonzentrationen wurde online mit dem BioLector-System (m2p-labs GmbH, Forckenbeckstrasse 6, 52074 Aachen, Germany) erfasst. Die Korrelation der internen L-Methioninkonzentration mit dem Fluoreszenz-Output Signal ist in Fig. 6 gezeigt. Es ist ersichtlich, dass das Sensorplasmid pJC1lrp-brnF'eYFP die intrazelluläre Detektion von Methionin in einem linearen Bereich von ca. 0,2-25 mM ermöglicht. Es kann bereits eine Akkumulation von Methionin im unteren mM-Bereich detektiert werden (< 1 mM).

### Beispiel 4

Nutzung eines Metabolitsensors zur Isolation von Zellen mit erhöhter Lysinbildung und Identifikation neuer Mutationen die zu Lysinbildung führen.

### a) Konstruktion eines rekombinanten Wildtyps von Corynebacterium glutamicum mit dem Lysinsensor pSenLysTK-C

Der Vektor pJC1 ist bei Cremer et al. beschrieben (Molecular and General Genetics, 1990, 220:478-480). Dieser Vektor wurde mit *BamH*I und *Sal*I geschnitten, und mit dem durch GATC (GATC Biotech AG, Jakob-Stadler-Platz 7, 78467 Konstanz) synthetisierten 1.765 kb-Fragment *BamH*I-<-EYFP-lysE'-lysG->-*Sal*I (SEQ.-ID.-Nr. 56) ligiert.

Der resultierende Vektor pSenLysTK wurde mit dem Restriktionsenzym *BamH*I verdaut, und mit dem von GATC (GATC Biotech AG, Jakob-Stadler-Platz 7, 78467 Konstanz) synthetisierten 2,506 kb Fragment BamHI-T7terminator-<-Crimson----lacIQ->-*BamH*I (SEQ.-ID:-Nr. 57) ligiert.

Der resultierende Vektor wurde pSenLysTK-C genannt. Er enthält EYFP als transkriptionelle Fusion und als Lebendmarker das Protein Crimson. Das Sensorplasmid pSenLysTK-C wurde in kompetente Zellen des Wildtyps wie bei Tauch et al. beschrieben eingebracht (Curr. Microbiol. 45 (2002), Seiten 362-7), und der Stamm *Corynebacterium glutamicum* ATCC13032 pSenLysTK-C erhalten.

### b) Mutagenese von Corynebacterium glutamicum ATCC13032 pSenLysTK-C

Der hergestellte Stamm ATCC13032 pSenLysTK-C wurde über Nacht in dem Medium "*Difco Brain Heart Infusion"* (Difco, Becton Dikinson BD, 1 Becton Drive, Franklin Lakes, NJ USA) bei 30 °C angezogen, und 5 ml dieser Kultur mit 0,1 ml einer Lösung von 0,5 mg N-methyl-N-nitroso-N'-nitroguanidin, gelöst in 1 ml Dimethylsulfoxid, versetzt. Diese Kultur wurde bei 30 °C 15 Minuten geschüttelt. Anschließend wurden die Zellen bei 4 °C und 2.500 g abzentrifugiert, und in 5 ml 0,9% NaCl resuspendiert. Der Zentrifugationsschritt und die Resuspension wurden wiederholt. Zu der so erhaltenen Zellsuspension wurden 7,5 ml 80 %iges Glyzerin zugefügt, und Aliquots dieser mutierten Zellsuspension bei -20 °C gelagert.

### c) Hochdurchflusszytometrie (HT-FACS = "high throughput fluorescence activated cell sorting") und Zellsortierung

Von der unter b) erhaltenen Zellsuspension wurden 200 µl in 20 ml Flüssigmedium CGXII-Kan25 (Keilhauer et al., J. Bacteriol. 1993; 175(17):5595-603) gegeben und die Kultur bei 30 °C und 180 UpM inkubiert. Nach 45 Minuten wurde Isopropyl-β-D-thiogalactopyranosid in einer Endkonzentration von 0,1 mM zugegeben. Nach weiterer Inkubation für 2 Stunden erfolgte die Analyse der optischen Eigenschaften und die Sortierung von Zellpartikeln am FACS Aria II-Zellsortierer von Becton Dickinson (Becton Dikinson BD, 1 Becton Drive, Franklin Lakes, NJ USA). Die FACS Einstellungen betrugen als threshhold-Grenzen für den "*forward scatter*" und "*side scatter*" 500 bei einer elektronischen Verstärkung von 50 mV für den "*forward scatter*" (ND Filter 1.0) und 550 mV für den "*side scatter".* Anregung von EYFP erfolgte bei einer Wellenlänge von 488 nm und Detektion mittels "*parameter gain"* (PMT) von 530 zu 30 bei 625 mV. Anregung von Crimson erfolgte bei einer Wellenlänge von 633 nm und Detektion mittels PMT von 660 zu 20 bei 700 mV. Es wurden 2 Millionen Crimson-positive Zellen in 20 ml CGXII-Kan25 sortiert und die Kultur 22 Stunden bei 180 UpM und 30 °C kultiviert. Anschließend wurde wieder Isopropyl-β-D-thiogalactopyranosid in einer Endkonzentration von 0,1 mM zugegeben. Nach 2 weiteren Stunden wurden 18.000.000 Zellen mit einer Analysegeschwindigkeit von 10.000 Partikel pro Sekunde auf EYFP und Crimson Fluoreszenz analysiert, und 580 Zellen aussortiert die automatisch mit Hilfe des FACS Aria II-Zellsortierers auf BHIS-Kan25 Platten abgelegt wurden. Die Platten wurden 16 h bei 30 °C inkubiert. Von den 580 abgelegten Zellen wuchsen 270 an. Diese wurden alle in 0,8 ml CGXII-Kan25 in Mikrotiterplatten überführt, und für 48 h bei 400 UpM und 30 °C kultiviert. Die Platten wurden 30 min bei 4 °C im Mikrotiterplattenrotor bei 4000 x g zentrifugiert, die Überstände 1 : 100 mit Wasser verdünnt und mittels HPLC analysiert. Es wurden 185 Klone als Lysinbildner identifiziert. Zur detaillierteren Charakterisierung wurde von 40 dieser Klone erneut eine Analyse auf Produktbildung in 50 ml CGXII-Kan25 in Schüttelkolben durchgeführt. Während der Ausgangsstamm ATCC13032 pSenLysTK-C kein Lysin ausscheidet, bilden die 40 Mutanten unterschiedliche Lysinmengen, im Bereich von 2- 35 mM (Fig. 7).

### d) Identifikation von Mutationen in lysC, hom, thrB und thrC

Zur weiteren Charakterisierung der 40 Mutanten wurde deren chromosomale DNA mittels des DNeasy kits von Quiagen (Quiagen, Hilden, Germany) isoliert. Das Gen *lysC* wurde mit den primern lysC-32F (SEQ.-ID.-Nr. 58) und *lysC-*1938R (SEQ.-ID.-Nr. 59) amplifiziert, und die Amplifikate bei Eurofins MWG Operon (Anzingerstr. 7a, 85560 Ebersberg, Deutschland) sequenziert.
lysC-32F 5'-GAACATCAGCGACAGGACAA-3'
lysC-1938R 5'-GGGAAGCAAAGAAACGAACA-3'

Es wurden die bereits bekannten Mutationen T311I, T308I, A279T, A279V, und A279T erhalten. Zusätzlich wurden die neuen Mutationen H357Y (cac->tac), T313I (acc->atc), G277D (ggc->gac), und G277S (ggc->agc) erhalten. In Klammem ist jeweils das kodierende Triplett des Wildtyps, und nachfolgend das entsprechend mutierte der Mutante angegeben.

Das Gen *hom* wurde mit den Primem hom-289F (SEQ.-ID.-Nr. 60) und thrB-2069R (SEQ.-ID.-Nr. 61) amplifiziert, und die Amplifikate bei Eurofins MWG Operon (Anzingerstr. 7a, 85560 Ebersberg, Deutschland) sequenziert.
hom-289F 5'-CCTCCCCGGGTTGATATTAG-3'
thrB-2069R 5'-GGCCAGCACGAATAGCTTTA-3'

Es wurden die neuen Mutationen A346V (gct->gtt), V211F (gtc->ttc), G241S (ggt->agt), A328V (gct->gtt), T233I (acc->atc), sowie die Doppelmutation R158C (cgc->tgc) T351I (acc->atc) erhalten.

Die weitere Sequenzierung von *thrB* in den Mutanten mit dem Primerpaar hom-1684F (SEQ.-ID.-Nr. 62) und thrB-2951R (SEQ.-ID.-Nr. 63) ergab die neue Mutation S102F (tcc->ttc).
hom-1684F 5'-AGGAATCTCCCTGCGTACAA-3'
thrB-2951R 5'-CCGGATTCATCCAAGAAAGC-3'

Die weitere Sequenzierung von *thrC* in den Mutanten mit dem Primerpaar thrC-22F (SEQ.-ID.-Nr. 64) und thrC-2046R (SEQ.-ID.-Nr. 65) ergab die neue Mutation A372V (gcc->gtc).
thrC-22F 5'-GCCTTAAAACGCCACTCAAT-3'
thrC-2046R 5'-GGCCGTTGATCATTGTTCTT-3'

### e) Identifikation einer Mutation in murE

Zur weiteren Identifikation von Mutationen in Mutanten die weder in *lysC,* noch *hom, thrB,* oder *thrC* Mutationen aufwiesen, wurde zusätzlich *murE* sequenziert. Das Gen *murE* wurde mit den Primern murE-34F (SEQ.-ID.-Nr. 66) und murE-1944R (SEQ.-ID.-Nr. 67) amplifiziert, und die Amplifikate bei GATC (GATC Biotech AG, Jakob-Stadler-Platz 7, 78467 Konstanz) sequenziert.
murE-34F 5'-AACTCCACGCTGGAGCTCAC-3'
murE-1944R 5'-AGAACGCGGAGTCCACG-3'

Es wurde die *murE*-Gensequenz (SEQ.-ID.-Nr. 69) bestimmt, die eine C zu T Transition in Nukleotid 361 (ctc->ttc) aufweist, was im MurE-Protein (SEQ.-ID.-Nr. 68) zum Aminosäureaustausch L121F in Position 121 des Proteins führt.

### f) Auswirkung der murE Mutation auf die Lysinbildung im Wildtyp

Mittels der Primer 7-39-L-F (SEQ.-ID.-Nr. 70) und 7-39-R-R (SEQ.-ID.-Nr. 71) wurden 1 kb des Gens *murE* mit chromosomaler DNA der C. *glutamicum* Mutante M39 aus Beispiel e) amplifiziert und somit ein *murE*-Fragment erhalten, welches die neu identifizierte Mutation trägt. Das erhaltene Amplifikat wurde über *BamH*I und *Sal*I in den in *C. glutamicum* nicht replikativen Vektor pK19mobsacB (Schäfer et al., Gene 1994; 145:69-73). kloniert und mittels homologer Rekombination in das Wildtypgenom eingebracht (Tauch et al., Curr. Microbiol. 45 (2002), Seiten 362-7; Schäfer et al., Gene 1994; 145:69-73). Der resultierende Stamm *C. glutamicum* Lys39 wurde anschließend in 50 ml BHIS-Kan25 bei 30 °C und 130 UpM für 12 h kultiviert. Aus dieser Kultur wurden 500 µl in 50 ml CGXII-Kan25 übertragen, und erneut bei 30 °C und 130 UpM 24 h kultiviert. Davon ausgehend wurde die 50 ml CGXII-Hauptkultur mit einer Anfangs OD von 0,5 beimpft und diese für 48 h bei 130 UpM und 30 °C kultiviert. Der Kulturüberstand wurde 1 : 100 mit Wasser verdünnt, und mittels HPLC die in Tabelle 1 erhaltene L-Lysinkonzentration bestimmt.
7-39-L-F 5'-TAGGATCCCGACAACATCCCACTGTCTG-3'
7-39-R-R 5'-AAGTCGACGTCTGCTTCTTGCCCAAGG-3'

**Tabelle 1**

| Stamm | L-Lysin (mM) |
|---|---|
| *C. glutamicum* ATCC13032 | 0,5 |
| *C. glutamicum* Lys39 | 3,4 |

| | |
|---|---|
| L-Lysin im Überstand von *C. glutamicum* | |

### SEQUENZEN

SEQ.-ID.-NR. 01
SEQ.-ID.-NR.02
SEQ.-ID.-NR.03
SEQ.-ID.-NR.04
SEQ.-ID.-NR. 05
SEQ.-ID.-NR. 06
SEQ.-ID.-NR. 07
SEQ.-ID.-Nr. 08
SEQ.-ID.-NR. 09
SEQ.-ID.-NR. 10
SEQ.-ID.-NR. 11
SEQ.-ID.-NR. 12
SEQ.-ID.-NR. 13
   tagaccaaga tgttca 16
SEQ.-ID.-NR. 14
SEQ.-ID.-NR. 15
SEQ.-ID.-NR. 16
SEQ.-ID.-NR. 17
SEQ.-ID.-NR. 18
SEQ.-ID.-NR. 19
SEQ.-ID.-NR. 20
SEQ.-ID.-NR. 21
SEQ.-ID.-NR. 22
SEQ.-ID.-NR. 23
SEQ.-ID.-NR. 24
SEQ.-ID.-NR. 25
SEQ.-ID.-NR. 26
SEQ.-ID.-NR. 27
SEQ.-ID.-NR. 28
SEQ.-ID.-NR. 29
SEQ.-ID.-NR. 30
SEQ.-ID.-NR. 31
SEQ.-ID.-NR. 32
SEQ.-ID.-NR. 33
SEQ.-ID.-NR. 34
SEQ.-ID.-NR. 35
SEQ.-ID.-NR. 36
SEQ.-ID.-NR. 37
SEQ.-ID.-NR. 38 cgcggatccc taagccgcaa tccctgattg 30
SEQ.-ID.-NR. 39 tccgatggac agtaaaagac tggcccccaa agcag 35
SEQ.-ID.-NR. 40 tgaggatcct tattacttgt cagctcgtcc atgccgagag tgatcc 46
SEQ.-ID.-NR. 41 cttttactgt ccatcggaac tagctatggt gagcaagggc gaggagctgt tcacc 55
SEQ.-ID.-NR. 42
   tcaactgcta tcccccctgt ta 22
SEQ.-ID.-NR. 43
   aaactccttt acttaaatgt tttgataaat aaa 33
SEQ.-ID.-NR. 44
   tacatatggt gagcaagggc ga 22
SEQ.-ID.-NR. 45
   tagaattctt atctagactt gtacagctcg 30
SEQ.-ID.-NR. 46
   cggcgtttca cttctgagtt cggc 24
SEQ.-ID.-NR. 47
   tagaattctt atctagactt gtacagctcg 30
SEQ.-ID.-NR. 48
SEQ.-ID.-NR. 49
SEQ.-ID.-NR. 50
   gcgcggatcc tcacacctgg gggcgagctg 30
SEQ.-ID.-NR. 51
   gcgccatatg atatctcctt cttaaagttc agcttgaatg aatctcttgc g 51
SEQ.-ID.-NR. 52
   gcgccatatg gtgagcaagg gcgaggag 28
SEQ.-ID.-NR. 53
   gcgcgtcgac ttatctagac ttgtacagct cgtc 34
SEQ.-ID.-NR. 54
   cgatcctgac gcagattttt 20
SEQ.-ID.-NR. 55
   ctcaccggct ccagatttat 20
SEQ.-ID.-NR. 56
SEQ.-ID.-NR. 57
SEQ.-ID.-NR. 58
   gaacatcagc gacaggacaa 20
SEQ.-ID.-NR. 59
   gggaagcaaa gaaacgaaca 20
SEQ.-ID.-NR. 60
   cctccccggg ttgatattag 20
SEQ.-ID.-NR. 61
   ggccagcacg aatagcttta 20
SEQ.-ID.-NR. 62
   aggaatctcc ctgcgtacaa 20
SEQ.-ID.-NR. 63
   ccggattcat ccaagaaagc 20
SEQ.-ID.-NR. 64
   gccttaaaac gccactcaat 20
SEQ.-ID.-NR. 65
   ggccgttgat cattgttctt 20
SEQ.-ID.-NR. 66
   aactccacgc tggagctcac 20
SEQ.-ID.-NR. 67
   agaacgcgga gtccacg 17
SEQ.-ID.-NR. 68
SEQ.-ID.-NR. 69
SEQ.-ID.-NR. 70
   taggatcccg acaacatccc actgtctg 28
SEQ.-ID.-NR. 71
   aagtcgacgt ctgcttcttg cccaagg 27
SEQ.-ID.-NR. 72

## Patentansprüche

1. Eine gegenüber ihrem Wildtyp gentechnisch veränderte Zelle, die eine für ein Autofluoreszenzprotein kodierende Gensequenz umfasst, wobei die Expression des Autofluoreszenzproteins von der intrazellulären Konzentration eines bestimmten Metaboliten abhängig ist und wobei die Zelle nach einer Mutation diesen Metaboliten überproduziert,
- wobei sich die für das Autofluoreszenzprotein kodierende Gensequenz unter der Kontrolle eines heterologen Promotors befindet, der im Wildtyp der Zelle die Expression eines Gens kontrolliert, dessen Expression in der Wildtyp-Zelle von der intrazellulären Konzentration des Metaboliten abhängig ist, oder
- wobei die für das Autofluoreszenzprotein kodierende Gensequenz funktionell mit einer DNA-Sequenz verbunden ist, die auf der Ebene der mRNA in der Lage ist, den Metaboliten zu binden, wobei in Abhängigkeit von der Bindung des Metaboliten an die mRNA die Transkription entlang der DNA oder die Translation an den Ribosomen beeinflusst wird.

2. Die Zelle nach Anspruch 1, wobei die Zelle eine Zelle der Gattung *Corynebacterium* oder *Escherichia* ist.

3. Die Zelle nach einem der vorhergehenden Ansprüche, wobei der Metabolit ausgewählt ist aus der Gruppe bestehend aus Aminosäuren, Nukleotiden, Fettsäuren und Kohlenhydraten.

4. Die Zelle nach Anspruch 3, wobei der Metabolit eine Aminosäure ist.

5. Die Zelle nach Anspruch 4, wobei die Aminosäure L-Lysin ist.

6. Die Zelle nach einem der vorhergehenden Ansprüche, wobei der Promotor der lysE-Promotor und das Gen das lysE-Gen ist.

7. Die Zelle nach einem der vorhergehenden Ansprüche, wobei das Autofluoreszenzprotein das grün fluoreszierende Protein (GFP) oder eine Variante dieses Proteins ist.

8. Ein Verfahren zum Identifizieren eine Zelle mit erhöhter intrazellulärer Konzentration eines bestimmten Metaboliten in einer Zellsuspension, beinhaltend die Verfahrensschritte:
i) Bereitstellen einer Zellsuspension beinhaltend eine gegenüber ihrem Wildtyp gentechnisch veränderte Zelle, die eine für ein Autofluoreszenzprotein kodierende Gensequenz umfasst, wobei die Expression des Autofluoreszenzproteins von der intrazellulären Konzentration eines bestimmten Metaboliten abhängig ist,
- wobei sich die für das Autofluoreszenzprotein kodierende Gensequenz unter der Kontrolle eines heterologen Promotors be-findet, der im Wildtyp der Zelle die Expression eines Gens kontrolliert, dessen Expression in der Wildtyp-Zelle von der intrazellulären Konzentration des Metaboliten abhängig ist, oder
- wobei die für das Autofluoreszenzprotein kodierende Gensequenz funktionell mit einer DNA-Sequenz verbunden ist, die auf der Ebene der mRNA in der Lage ist, den Metaboliten zu binden, wobei in Abhängigkeit von der Bindung des Metaboliten an die mRNA die Transkription entlang der DNA oder die Translation an den Ribosomen beeinflusst wird;
ii) gentechnisches Verändern der Zellen unter Erhalt einer Zellsuspension, in der sich die Zellen hinsichtlich der intrazellulären Konzentration eines bestimmten Metaboliten unterscheiden;
iii) Identifizieren einzelner Zellen in der Zellsuspension mit erhöhter intrazellulärer Konzentration dieses bestimmten Metaboliten durch Detektion der intrazellulären Fluoreszenzaktivität.

9. Das Verfahren nach Anspruch 8, wobei das gentechnische Verändern im Verfahrensschritt ii) durch ungerichtete Mutagenese erfolgt.

10. Das Verfahren nach Anspruch 8 oder 9, weiterhin beinhaltend den Verfahrensschritt:
iv) das Abtrennen der identifizierten Zellen aus der Zellsuspension.

11. Das Verfahren nach Anspruch 10, wobei das Abtrennen mittels Durchflusszytometrie erfolgt.

12. Ein Verfahren zur Herstellung einer gegenüber ihrem Wildtyp gentechnisch veränderten Zelle mit optimierter Produktion eines bestimmten Metaboliten, beinhaltend die Verfahrensschritte:
I) Bereitstellen einer Zellsuspensionen beinhaltend gegenüber ihrem Wildtyp gentechnisch veränderte Zellen, die eine für ein Autofluoreszenzprotein kodierende Gensequenz umfassen, wobei die Expression des Autofluoreszenzproteins von der intrazellulären Konzentration eines bestimmten Metaboliten abhängig ist,
- wobei sich die für das Autofluoreszenzprotein kodierende Gensequenz unter der Kontrolle eines heterologen Promotors be-findet, der im Wildtyp der Zelle die Expression eines Gens kontrolliert, dessen Expression in der Wildtyp-Zelle von der intrazellulären Konzentration des Metaboliten abhängig ist, oder
- wobei die für das Autofluoreszenzprotein kodierende Gensequenz funktionell mit einer DNA-Sequenz verbunden ist, die auf der Ebene der mRNA in der Lage ist, den Metaboliten zu binden, wobei in Abhängigkeit von der Bindung des Metaboliten an die mRNA die Transkription entlang der DNA oder die Translation an den Ribosomen beeinflusst wird;
II) gentechnisches Verändern der Zellen unter Erhalt einer Zellsuspension, in der sich die Zellen hinsichtlich ihrer intrazellulären Konzentration eines bestimmten Metaboliten unterscheiden;
III) Identifizieren einzelner Zellen in der Zellsuspension mit erhöhter intrazellulärer Konzentration des bestimmten Metaboliten durch Detektion der intrazellulären Fluoreszenzaktivität;
IV) Abtrennen der identifizierten Zellen aus der Zellsuspension;
V) Identifizieren derjenigen gentechnisch veränderten Gene G₁ bis Gₙ oder derjenigen Mutationen M₁ bis Mₘ in den identifizierten und abgetrennten Zellen, welche für die erhöhte intrazelluläre Konzentration des bestimmten Metaboliten verantwortlich sind;
VI) Herstellung einer gegenüber ihrem Wildtyp gentechnisch veränderten Zelle mit optimierter Produktion des bestimmten Metaboliten, deren Genom mindestens eines der Gene G₁ bis Gₙ und/oder mindestens eine der Mutationen M₁ bis Mₘ umfasst.

13. Das Verfahren nach Anspruch 12, wobei das gentechnische Verändern im Verfahrensschritt II) durch ungerichtete Mutagenese erfolgt.

14. Zelle, erhalten durch ein Verfahren nach Anspruch 12 oder 13.

15. Ein Verfahren zur Herstellung von Metaboliten, beinhaltend die Verfahrensschritte:
(a) Herstellung einer gegenüber ihrem Wildtyp gentechnisch veränderten Zelle mit optimierter Produktion eines bestimmten Metaboliten durch ein Verfahren nach Anspruch 12 oder 13;
(b) Kultivieren der Zelle in einem Kulturmedium beinhaltend Nährstoffe unter Bedingungen, unter denen die Zelle aus den Nährstoffen den bestimmten Metaboliten produziert.

16. Verfahren nach Anspruch 15, wobei der Metabolit ausgewählt ist aus der Gruppe bestehend aus Aminosäuren, Nukleotiden, Fettsäuren und Kohlenhydraten.

17. Verfahren nach Anspruch 16, wobei der Metabolit eine Aminosäure ist.

18. Das Verfahren nach Anspruch 17, wobei die Aminosäure L-Lysin ist.

19. Verfahren zur Herstellung einer Mischung, beinhaltend die Verfahrensschritte:
(A) Herstellen von Metaboliten durch das Verfahren nach einem der Ansprüche 15 bis 18;
(B) Vermischen des Metaboliten mit einer von dem Metaboliten verschiedenen Mischungskomponente.

20. Verfahren nach Anspruch 19, wobei der Metabolit L-Lysin die Mischung ein Nahrungsmittel oder eine pharmazeutische Zusammensetzung ist.

## Claims

1. A cell genetically modified with respect to its wild-type, comprising a gene sequence coding for an autofluorescent protein, wherein the expression of the autofluorescent protein depends on the intracellular concentration of a particular metabolite and wherein the cell overproduces this metabolite after a mutation,
- wherein the gene sequence coding for the autofluorescent protein is under the control of a heterologous promoter which in the wild-type of the cell controls the expression of a gene, expression of which in the wild-type cell depends on the intracellular concentration of the metabolite, or
- wherein the gene sequence coding for the autofluorescent protein is associated functionally with a DNA sequence which at the level of the mRNA is capable of binding the metabolite, the transcription along the DNA or the translation at the ribosomes being influenced as a function of the binding of the metabolite to the mRNA.

2. The cell according to claim 1, wherein the cell is a cell of the genus *Corynebacterium* or *Escherichia.*

3. The cell according to one of the preceding claims, wherein the metabolite is selected from the group consisting of amino acids, nucleotides, fatty acids and carbohydrates.

4. The cell according to claim 3, wherein the metabolite is an amino acid.

5. The cell according to claim 4, wherein the amino acid is L-lysine.

6. The cell according to one of the preceding claims, wherein the promoter is the lysE promoter and the gene is the lysE gene.

7. The cell according to one of the preceding claims, wherein the autofluorescent protein is the green fluorescent protein (GFP) or a variant of this protein.

8. A method for identifying a cell having an increased intracellular concentration of a particular metabolite in a cell suspension, comprising the method steps:
i) providing a cell suspension comprising a cell genetically modified with respect to its wild-type, which comprises a gene sequence coding for an autofluorescent protein, wherein the expression of the autofluorescente protein depends on the intracellular concentration of a particular metabolite,
- wherein the gene sequence coding for the autofluorescent protein is under the control of a heterologous promoter which in the wild-type of the cell controls the expression of a gene, expression of which in the wild-type cell depends on the intracellular concentration of the metabolite, or
- wherein the gene sequence coding for the autofluorescent protein is associated functionally with a DNA sequence which at the level of the mRNA is capable of binding the metabolite, the transcription along the DNA or the translation at the ribosomes being influenced as a function of the binding of the metabolite to the mRNA;
ii) genetically modifying the cells to obtain a cell suspension in which the cells differ with respect to the intracellular concentration of a particular metabolite;
iii) indentifying individual cells in the cell suspension having an increased intracellular concentration of this particular metabolite by detection of the intracellular fluorescence activity.

9. The method according to claim 8, wherein the genetic modification in method step ii) is carried out by non-directed mutagenesis.

10. The method according to claim 8 or 9, furthermore comprising the method step:
iv) separating off the identified cells from the cell suspension.

11. The method according to claim 10, wherein the separating off is carried out by means of flow cytometry.

12. A method of producing a cell genetically modified with respect to its wild-type having optimised production of a particular metabolite, comprising the method steps:
I) providing a cell suspension comprising cells genetically modified with respect to its wild-type, which comprise a gene sequence coding for an autofluorescent protein, wherein the expression of the autofluorescente protein depends on the intracellular concentration of a particular metabolite,
- wherein the gene sequence coding for the autofluorescent protein is under the control of a heterologous promoter which in the wild-type of the cell controls the expression of a gene, expression of which in the wild-type cell depends on the intracellular concentration of the metabolite, or
- wherein the gene sequence coding for the autofluorescent protein is associated functionally with a DNA sequence which at the level of the mRNA is capable of binding the metabolite, the transcription along the DNA or the translation at the ribosomes being influenced as a function of the binding of the metabolite to the mRNA;
II) genetically modifying the cells to obtain a cell suspension in which the cells differ with respect to the intracellular concentration of a particular metabolite;
III) indentifying individual cells in the cell suspension having an increased intracellular concentration of the particular metabolite by detection of the intracellular fluorescence activity;
IV) separating off the identified cells from the cell suspension;
V) identifying in the cells identified and separated off those genetically modified genes G₁ to Gₙ or those mutations M₁ to Mₘ which are responsible for the increased intracellular concentration of the particular metabolite;
VI) producing a cell genetically modified with respect to its wild-type having optimised production of the particular metabolite, the genome of which comprises at least one of the genes G₁ to Gₙ and/or at least one of the mutations M₁ to Mₘ.

13. The method according to claim 12, wherein the genetic modification in method step II) is carried out by non-directed mutagenesis.

14. Cell obtained by a method according to claim 12 or 13.

15. A method for producing metabolites, comprising the method steps:
(a) producing a cell genetically modified with respect to its wild-type having optimised production of a particular metabolite by a method according to claim 12 or 13;
(b) cultivating the cell in a culture medium comprising nutrients under conditions under which the cell produces the particular metabolite from the nutrients.

16. Method according to claim 15, wherein the metabolite is selected from the group consisting of amino acids, nucleotides, fatty acids and carbohydrates.

17. Method according to claim 16, wherein the metabolite is an amino acid.

18. The method according to claim 17, wherein the amino acid is L-lysine.

19. Method for producing a mixture, comprising the method steps:
(A) producing metabolites by the method according to one of claims 15 to 18;
(B) mixing the metabolite with a mixing component which differs from the metabolite.

20. Method according to claim 19, wherein the metabolite is L-lysine and the mixture is a foodstuff or a pharmaceutical composition.

## Revendications

1. Cellule manipulée génétiquement par rapport à son type sauvage, qui comprend une séquence génique codant pour une protéine autofluorescente, dans laquelle l'expression de la protéine autofluorescente est dépendante de la concentration intracellulaire d'un métabolite déterminé, ladite cellule surproduisant ledit métabolite après une mutation,
- dans laquelle la séquence génique codant pour la protéine autofluorescente se trouve sous le contrôle d'un promoteur hétérologue, qui contrôle l'expression d'un gène dans le type sauvage de la cellule, dont l'expression dans la cellule de type sauvage est dépendante de la concentration intracellulaire du métabolite, ou
- dans laquelle la séquence génique codant pour la protéine autofluorescente est reliée de manière fonctionnelle à une séquence d'ADN qui est en mesure de lier le métabolite au niveau de l'ARNm, la transcription le long de l'ADN ou la traduction sur le ribosome étant influencée en fonction de la liaison du métabolite à l'ARNm.

2. Cellule selon la revendication 1, ladite cellule étant une cellule du genre *Corynebacterium* ou *Escherichia.*

3. Cellule selon l'une des revendications précédentes, dans laquelle le métabolite est choisi dans le groupe constitué par les acides aminés, les nucléotides, les acides gras et les hydrates de carbone.

4. Cellule selon la revendication 3, dans laquelle le métabolite est un acide aminé.

5. Cellule selon la revendication 4, dans laquelle l'acide aminé est la L-lysine.

6. Cellule selon l'une des revendications précédentes, dans laquelle le promoteur est le promoteur de la lysE et le gène est le gène de la lysE.

7. Cellule selon l'une des revendications précédentes, dans laquelle la protéine autofluorescente est la protéine fluorescente verte (GFP) ou une variante de cette protéine.

8. Procédé d'identification d'une cellule avec une concentration intracellulaire accrue d'un métabolite déterminé dans une suspension cellulaire, comprenant les étapes de procédé suivantes :
i) la fourniture d'une suspension cellulaire comprenant une cellule manipulée génétiquement par rapport à son type sauvage, qui comprend une séquence génique codant pour une protéine autofluorescente, dans laquelle l'expression de la protéine autofluorescente est dépendante de la concentration intracellulaire d'un métabolite déterminé,
- dans laquelle la séquence génique codant pour la protéine autofluorescente se trouve sous le contrôle d'un promoteur hétérologue, qui contrôle l'expression d'un gène dans le type sauvage de la cellule, dont l'expression dans la cellule de type sauvage est dépendante de la concentration intracellulaire du métabolite, ou
- dans laquelle la séquence génique codant pour la protéine autofluorescente est reliée de manière fonctionnelle à une séquence d'ADN qui est en mesure de lier le métabolite au niveau de l'ARNm, la transcription le long de l'ADN ou la traduction sur le ribosome étant influencée en fonction de la liaison du métabolite à l'ARNm ;
ii) la manipulation génétique des cellules en obtenant une suspension cellulaire dans laquelle les cellules se distinguent en fonction de la concentration intracellulaire d'un métabolite déterminé ;
iii) l'identification des différentes cellules dans la suspension cellulaire avec une concentration intracellulaire accrue de ce métabolite déterminé par détection de l'activité fluorescente intracellulaire.

9. Procédé selon la revendication 8, dans lequel la manipulation génétique s'effectue au cours de l'étape de procédé ii) par mutagenèse non dirigée.

10. Procédé selon la revendication 8 ou 9, comprenant en outre l'étape de procédé suivante :
iv) la séparation des cellules identifiées et de la suspension cellulaire.

11. Procédé selon la revendication 10, dans lequel la séparation s'effectue par cytométrie en flux.

12. Procédé de préparation d'une cellule manipulée génétiquement par rapport à son type sauvage avec une production optimisée d'un métabolite déterminé, comprenant les étapes de procédé suivantes :
I) la fourniture d'une suspension cellulaire comprenant une cellule manipulée génétiquement par rapport à son type sauvage, qui comprend une séquence génique codant pour une protéine autofluorescente, dans laquelle l'expression de la protéine autofluorescente est dépendante de la concentration intracellulaire d'un métabolite déterminé,
- dans laquelle la séquence génique codant pour la protéine autofluorescente se trouve sous le contrôle d'un promoteur hétérologue, qui contrôle l'expression d'un gène dans le type sauvage de la cellule, dont l'expression dans la cellule de type sauvage est dépendante de la concentration intracellulaire du métabolite, ou
- dans laquelle la séquence génique codant pour la protéine autofluorescente est reliée de manière fonctionnelle à une séquence d'ADN qui est en mesure de lier le métabolite au niveau de l'ARNm, la transcription le long de l'ADN ou la traduction sur le ribosome étant influencée en fonction de la liaison du métabolite à l'ARNm ;
II) la manipulation génétique des cellules en obtenant une suspension cellulaire dans laquelle les cellules se distinguent en fonction de leur concentration intracellulaire d'un métabolite déterminé ;
III) l'identification des différentes cellules dans la suspension cellulaire avec une concentration intracellulaire accrue de ce métabolite déterminé par détection de l'activité fluorescente intracellulaire;
IV) la séparation des cellules identifiées et de la suspension cellulaire ;
V) l'identification des gènes manipulés génétiquement G₁ à Gₙ ou des mutations M₁ à Mₘ dans les cellules identifiées et séparées, qui sont responsables de la concentration intracellulaire accrue du métabolite déterminé ;
VI) la préparation d'une cellule manipulée génétiquement par rapport à son type sauvage avec une production optimisée du métabolite déterminé, dont le génome comprend au moins un des gènes G₁ à Gₙ et/ou au moins une des mutations M₁ à Mₘ.

13. Procédé selon la revendication 12, dans lequel la manipulation génétique dans l'étape de procédé II) s'effectue par mutagenèse non dirigée,

14. Cellule obtenue par un procédé selon la revendication 12 ou 13.

15. Procédé de préparation de métabolites, comprenant les étapes de procédé suivantes :
(a) la préparation d'une cellule manipulée génétiquement par rapport à son type sauvage avec une production optimisée d'un métabolite déterminé par un procédé selon la revendication 12 ou 13 ;
(b) la culture de la cellule dans un milieu de culture comprenant des nutriments dans des conditions où la cellule produit le métabolite déterminé à partir des nutriments.

16. Procédé selon la revendication 15, dans lequel le métabolite est choisi dans le groupe constitué par les acides aminés, les nucléotides, les acides gras et les hydrates de carbone.

17. Procédé selon la revendication 16, dans lequel le métabolite est un acide aminé.

18. Procédé selon la revendication 17, dans lequel l'acide aminé est la L-lysine.

19. Procédé de préparation d'un mélange comprenant les étapes de procédé suivantes :
(A) la préparation des métabolites par le procédé selon l'une des revendications 15 à 18 ;
(B) le mélange du métabolite avec un compostant de mélange différent du métabolite.

20. Procédé selon la revendication 19, dans lequel le métabolite est la L-lysine et le mélange est un aliment ou une composition pharmaceutique.
